(19) Europäisches Patentamt
European Patent Office
Office européen des brevets

(11) EP 1 246 793 B1

(12) # EUROPÄISCHE PATENTSCHRIFT

(45) Veröffentlichungstag und Bekanntmachung des Hinweises auf die Patenterteilung:
**10.11.2004 Patentblatt 2004/46**

(21) Anmeldenummer: **00993710.3**

(22) Anmeldetag: **27.12.2000**

(51) Int Cl.7: **C07C 229/38**, A61K 31/135, A61P 23/00, C07C 237/30, C07C 217/74, C07C 215/64

(86) Internationale Anmeldenummer:
**PCT/EP2000/013281**

(87) Internationale Veröffentlichungsnummer:
**WO 2001/049654 (12.07.2001 Gazette 2001/28)**

(54) **SUBSTITUIERTE AMINOMETHYL-PHENYL-CYCLOHEXANDERIVATE**
SUBSTITUTED AMINOMETHYL-PHENYL-CYCLOHEXANE DERIVATIVES
DERIVES D'AMINOMETHYLE-PHENYLE-CYCLOHEXANE SUBSTITUES

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **05.01.2000 DE 10000312**

(43) Veröffentlichungstag der Anmeldung:
**09.10.2002 Patentblatt 2002/41**

(73) Patentinhaber: **Grünenthal GmbH**
**52078 Aachen (DE)**

(72) Erfinder:
- **PÜTZ, Claudia**
  **52349 Düren (DE)**
- **STRASSBURGER, Wolfgang**
  **52146 Würselen (DE)**
- **KÖGEL, Babette-Yvonne**
  **52379 Langerwehe-Hamich (DE)**

(56) Entgegenhaltungen:
**EP-A- 0 780 369**
**WO-A-95/21813**
**DE-A- 19 525 137**

- **FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN STUDIES ON CHEMICAL STRUCTURE AND ANALGETIC ACTIVITY OF PHENYL SUBSTITUTED AMINOMETHYLCYCLOHEXANOLES" ARZNEIMITTEL FORSCHUNG. DRUG RESEARCH, EDITIO CANTOR. AULENDORF, DE, Bd. 28, Nr. 1A, 1978, Seiten 107-113, XP000608150 ISSN: 0004-4172**

EP 1 246 793 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft substituierte Aminomethyl-Phenyl-Cyclohexanderivate, sowie Verfahren zu deren Herstellung, deren Verwendung zur Herstellung von Arzneimitteln und Arzneimittel enthaltend diese Verbindungen.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem zeigen sie bei einigen Schmerzzuständen, insbesondere bei neuropathischen Schmerzen, eine geringere Wirksamkeit.

Ein weiteres bekanntes Therapeutikum zur Behandlung starker Schmerzen ist Tramadolhydrochlorid - (1RS, 2RS)-2-[(Dimethylamino)methyl]-1-(3-methoxyphenyl)cyclohexanol, Hydrochlorid. Es nimmt unter den zentralwirksamen Analgetika insofern eine Sonderstellung ein, daß dieser Wirkstoff eine starke Schmerzhemmung ohne die für Opioide bekannten Nebenwirkungen hervorruft (J. Pharmacol. Exptl. Ther. 267, 331 (1993)), wobei sowohl die Enantiomeren von Tramadol als auch die Enantiomeren der Tramadolmetabolite an der analgetischen Wirkung beteiligt sind (J. Pharmacol. Exp. Ther. 260, 275 (1992)). Auch Tramadol ist allerdings nicht nebenwirkungsfrei.

Eine der Erfindung zugrundeliegende Aufgabe bestand darin, analgetisch wirksame Substanzen zur Verfügung zu stellen, die sich zur Schmerztherapie eignen. Darüber hinaus sollten diese Substanzen möglichst wenig Nebenwirkungen wie z.B. Übelkeit, Erbrechen, Abhängigkeit, Atemdepression oder Obstipation aufweisen.

Diese Aufgabe wird durch die erfindungsgemäßen substituierten Aminomethyl-Phenyl-Cyclohexanderivate gelöst. Gegenstand der Erfindung sind daher substituierte Aminomethyl-Phenyl-Cyclohexanderivate der allgemeinen Formel I bzw. Ia, auch in Form ihrer Diastereomere oder Enantiomere sowie ihrer freien Basen oder eines mit einer physiologisch verträglichen Säure gebildeten Salzes, insbesondere des Hydrochloridsalzes,

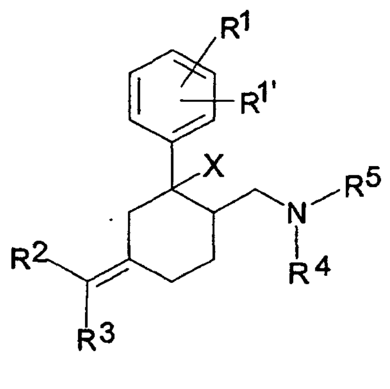

I

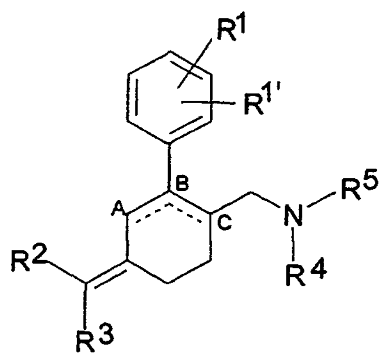

Ia

, worin

$R^1$ und $R^{1'}$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; F, Cl, Br, I, $NR^6R^{6'}$, $NO_2$, CN, $OR^6$, $SR^6$, $OC(O)R^6$, $C(O)OR^6$, $C(O)R^6$ oder C (O)$NR^6R^6$, wobei $R^6$ und $R^{6'}$ ausgewählt sind aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl

oder Heteroaryl, jeweils einfach oder mehrfach substituiert oder unsubstituiert;

oder

$R^1$ und $R^{1'}$ zusammen -CH=CH-CH=CH- bilden, wobei das entstehende Naphthylsystem ein- oder mehrfach substituiert sein kann,

X ausgewählt ist aus

H, F, Cl, Br I, $CF_3$, $OS(O_2)C_6H_4$-$pCH_3$, $OR^7$ oder $OC(O)R^7$, wobei $R^7$ ausgewählt ist aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist,

$R^4$, $R^5$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^4$ und $R^5$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

und

$R^2$, $R^3$ unabhängig voneinander augewählt sind aus

$R^9$ oder $YR^9$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^9$ ausgewählt ist aus

H, F, Cl, Br, I, CN, $NO_2$, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{10}$ ersetzt ist, mit $R^{10}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

$OR^{11}$, $OC(O)R^{11}$, $OC(O)OR^{11}$, $OC(S)R^{11}$, $C(O)R^{11}$, $C(O)OR^{11}$, $C(S)R^{11}$, $C(S)OR^{11}$, $SR^{11}$, $S(O)R^{11}$ bzw. $S(O_2)R^{11}$, wobei $R^{11}$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

$NR^{13}R^{14}$, $NR^{13}C(O)R^{14}$, $C(O)NR^{13}R^{14}$ oder $S(O_2)NR^{13}R^{14}$, wobei $R^{13}$ und $R^{14}$ unabhängig voneinander ausgewählt sind aus

H, O; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{15}$ ersetzt ist, mit $R^{15}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^{13}$ und $R^{14}$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{16}$ ersetzt ist, mit $R^{16}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

Alkylaryl, Aryl oder Heteroaryl, jeweils einfach oder mehrfach substituiert oder unsubstituiert; insbesondere

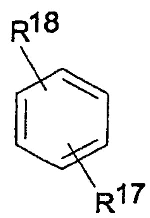

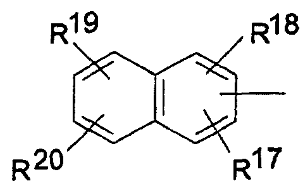

oder

, wobei $R^{17}$, $R^{18}$, $R^{19}$ und $R^{20}$ unabhängig voneinander ausgewählt sind aus

$R^{21}$ oder $ZR^{21}$ mit Z = $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert, wobei $R^{21}$ ausgewählt ist aus

H, F, Cl, Br, I, CN, $NO_2$; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{22}$ ersetzt ist, mit $R^{22}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, Aryl oder Heteroaryl, jeweils einfach oder mehrfach substituiert oder unsubstituiert;

$OR^{23}$, $OC(O)R^{23}$, $OC(O)OR^{23}$, $OC(S)R^{23}$, $C(O)R^{23}$, $C(O)OR^{23}$, $C(S)R^{23}$, $C(S)OR^{23}$, $SR^{23}$, $S(O)R^{23}$ bzw. $S(O_2)R^{23}$, wobei $R^{23}$ ausgewählt ist aus

H, $C_1$-$C_{18}$-Alkyl, $C_2$-$C_{18}$-Alkenyl bzw. $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{24}$ ersetzt ist, mit $R^{24}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

$NR^{25}R^{26}$, $NR^{25}C(O)R^{26}$, $C(O)NR^{25}R^{26}$ oder $S(O_2)NR^{25}R^{26}$, wobei $R^{25}$ und $R^{26}$ unabhängig voneinander ausgewählt sind aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{27}$ ersetzt ist, mit $R^{27}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^{25}$ und $R^{26}$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{27}$ ersetzt ist, mit $R^{27}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert.

Dabei versteht man im Zusammenhang mit Alkyl, Alkenyl Alkinyl und Cycloalkyl bzw. dem "entsprechenden Heterocyclus" unter dem Begriff substituiert im Sinne dieser Erfindung die Substitution eines Wasserstoffrestes durch F, Cl, Br, I, $NH_2$, SH oder OH, wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von $CF_3$ oder an verschiedenen Stellen wie im Falle von -CH(OH)-CH=CH-$CHCl_2$.

Weiter bedeutet -C(O)-

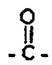

, was auch für -C(S)- oder -S(O)- bzw. -$S(O_2)$- gilt.

Der Ausdruck "$C_1$-$C_8$-Alkyl" bzw. "$C_1$-$C_{10}$-Alkyl" bedeutet im Sinne dieser Erfindung Kohlenwasserstoffe mit 1 bis 8 bzw. 10 Kohlenstoffatomen. Bespielhaft seien Methyl, Ethyl, Propyl, Isopropyl, n-Butan, sek.-Butyl, tert-Butyl, n-Pentan, Neopentyl, n-Hexan, n-Heptan, n-Octan, n-Nonan oder n-Decan genannt

Der Ausdruck "$C_1$-$C_{18}$-Alkyl" bedeutet im Sinne dieser Erfindung Kohlenwasserstoffe mit 1 bis 18 Kohlenstoffatomen. Beispielhaft seien Methyl, Ethyl, Propyl, Isopropyl, n-Butan, sek. Butyl, tert. Butyl, n-Pentan, Neopentyl, n-Butan, sek. Butyl, tert. Butyl, n-Hexan, n-Heptan, n-Octan, n-Nonan, n-Decan, n-Undecan, n-Dodecan, n-Dodecan, n-Tridecan, n-Tetradecan, n-Pentadecan, n-Hexadecan, n-Heptadecan oder n-Octadecan unsubstituiert oder ein oder mehrfach substituiert genannt.

Der Ausdruck "$C_2$-$C_{10}$-Alkenyl" bzw. "$C_2$-$C_{10}$-Alkinyl" oder "$C_2$-$C_{18}$-Alkenyl" bzw. "$C_2$-$C_{18}$-Alkinyl" bedeutet im Sinne dieser Erfindung Kohlenwasserstoffe mit 2 bis 8 bzw. 2 bis 18 Kohlenstoffatomen. Beispielhaft genannt seien Propenyl, Butenyl, Pentenyl, Hexenyl, Heptenyl, Octenyl unsubstituiert oder ein oder mehrfach substituiert bzw. Propinyl, Butinyl, Pentinyl, Hexinyl, Heptinyl, Octinyl unsubstituiert oder ein oder mehrfach substituiert.

Der Ausdruck $C_3$-$C_7$-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclohexenyl oder Cycloheptenyl gesättigt oder ungesättigt, unsubstituiert oder ein oder mehrfach substituiert genannt. Dabei versteht man im Sinne der Erfindung unter einem einem "entsprechenden Heterocyclus" ein $C_3$-$C_7$-Cycloalkyl, bei dem ein C-Atom im Ring durch S, O oder N ersetzt ist. Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle oder Naphthyle.

Der Ausdruck "Alkylaryl" bedeutet im Sinne dieser Erfindung mindestens mit $C_1$-$C_{10}$-Alkylen substituierte Aryle, wobei die Ausdrücke Aryl und Alkyl die gleiche Bedeutung haben wie oben. In dieser Gruppe sei insbesondere Benzaryl erwähnt.

Der Ausdruck "Heteroaryl" bedeutet im Sinne dieser Erfindung 5- oder 6-gliedrige, gegebenenfalls mit einem ankondensierten Ringsystem versehene, aromatische Verbindungen, die ein oder zwei Heteroatome aus der Gruppe Stickstoff, Sauerstoff und/oder Schwefel enthalten. In dieser Gruppe seien beispielhaft Furan, Thiophen, Pyrrol, Pyridin, Pyrimidin, Chinolin Isochinolin, Phttalazin oder Chinazolin aufgeführt.

In Bezug auf Aryl, Alkylaryl oder Heteroaryl versteht man im Sinne dieser Erfindung unter ein- oder mehrfach substituiert, die Substitution des Ringsystems mit F, Cl, Br, I, $NH_2$, SH, OH, $CF_3$; ein- oder mehrfach substituiertem oder unsubstituiertem $C_1$-$C_6$-Alkyl, $C_1$-$C_6$-Alkoxy, $C_2$-$C_8$-Alkenyl, $C_2$-$C_8$-Alkinyl; oder Aryl, insbesondere Phenyl; an einem oder verschiedenen Atomen.

Unter dem Bgriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid.

In bevorzugten erfindungsgemäßen substituierten Aminomethyl-Phenyl-Cyclohexanderivaten gemäß der allgemeinen Formel I bzw. Ia haben $R^2$ und $R^3$ unterschiedliche Bedeutungen und/oder $R^3$ ist H oder $CH_3$, vorzugsweise H,, während $R^1$, $R^{1'}$, $R^2$, $R^4$, $R^5$ und X eine der bereits für sie genannten Bedeutungen haben.

In weiteren bevorzugten erfindungsgemäßen substituierten Aminomethyl-Phenyl-Cyclohexanderivaten gemäß der allgemeinen Formel I bzw. Ia ist $R^2$ ausgewählt aus

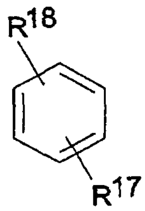
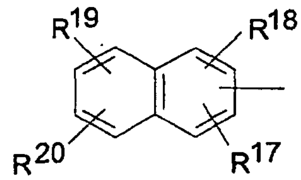

oder

, wobei $R^{17}$, $R^{18}$, $R^{19}$ und $R^{20}$ sowie $R^1$, $R^{1'}$, $R^3$ , $R^4$, $R^5$ und X eine der bereits für sie genannten Bedeutungen haben

oder $R^2$ ist ausgewählt aus $C_{1-3}$-Alkyl.

In weiteren bevorzugten erfindungsgemäßen substituierten Aminomethyl-Phenyl-Cyclohexanderivaten gemäß der allgemeinen Formel I bzw. Ia ist $R^2$

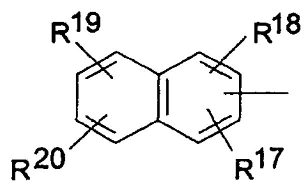

und $R^{19}$ und $R^{20}$ sind H, während $R^1$, $R^{1'}$, $R^3$, $R^4$, $R^5$, X sowie $R^{17}$ und $R^{18}$ eine der bereits für sie genannten Bedeutungen haben.

In weiteren bevorzugten erfindungsgemäßen substituierten Aminomethyl-Phenyl-Cyclohexanderivaten gemäß der allgemeinen Formel I bzw. la ist $R^2$ ausgewählt aus

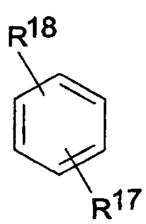
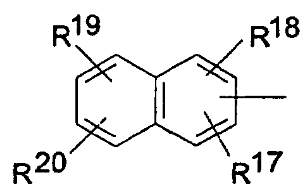

oder

, haben $R^{17}$ und $R^{18}$ unterschiedliche Bedeutung und/oder $R^{18}$ ist $R^{21}$ mit $R^{21}$ ausgewählt aus

H, F, Cl, Br, I, $CF_3$ oder $OR^{23}$ mit $R^{23}$ = H, Methyl- Ethyl-, Propyl-, Isopropyl-, Butyl- oder Isobutyl-,

während $R^1$, $R^{1'}$, $R^3$, $R^4$, $R^5$ und X sowie $R^{17}$, $R^{19}$ und $R^{20}$ eine der bereits für sie genannten Bedeutungen haben.

In weiteren besonders bevorzugten erfindungsgemäßen substituierten. Aminomethyl-Phenyl-Cyclohexanderivaten gemäß der allgemeinen Formel I bzw. la ist $R^2$ ausgewählt aus

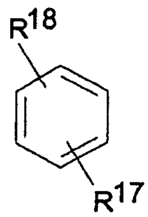
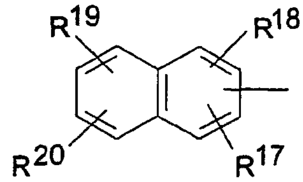

oder

, wobei $R^{17}$ ausgewählt ist aus

$R^{21}$, wobei $R^{21}$ ausgewählt ist aus

H, F, Cl, Br, I, $CF_3$, $OR^{23}$, $OC(O)R^{23}$, $C(O)R^{23}$ oder $C(O)OR^{23}$,vorzugsweise H, F, Cl, $C(O)OR^{23}$, wobei $R^{23}$ ausgewählt ist aus

H; $C_1$-$C_6$-Alkyl, $C_2$-$C_8$-Alkenyl oder $C_2$-$C_8$-Alkinyl, insbesondere $C_1$-$C_4$-Alkyl, verzweigt oder unverzweigt, einfach oder mehrfach substituiert oder unsubstituiert; vorzugsweise H, Methyl- Ethyl-, Propyl-, Isopropyl-, Butyloder Isobutyl-, insbesondere H, $CH_3$, $C_2H_5$, oder Isobutyl-;

$C(O)NR^{25}R^{26}$, wobei $R^{25}$ und $R^{26}$ unabhängig voneinander ausgewählt sind aus

H, O; $C_1$-$C_{18}$-Alkyl, insbesondere $C_1$-$C_4$-Alkyl, verzweigt oder unverzweigt, gesättigt oder ungesättigt, einfach oder mehrfach substituiert oder unsubstituiert, vorzugsweise H, Methyl-Ethyl-, Propyl-, Isopropyl-, Butyl- oder Isobutyl-, insbesondere $C_2H_5$;

oder

$ZR^{21}$ mit Z = $CH_2$ oder $C_2H_4$, vorzugsweise $CH_2$, wobei $R^{21}$ ausgewählt ist aus

$OR^{23}$, $OC(O)R^{23}$, $C(O)R^{23}$ oder $C(O)OR^{23}$, vorzugsweise $OR^{23}$, wobei $R^{23}$, ausgewählt ist aus

H; $C_1$-$C_6$-Alkyl, $C_2$-$C_8$-Alkenyl oder $C_2$-$C_8$-Alkinyl, insbesondere $C_1$-$C_4$-Alkyl, verzweigt oder unverzweigt, einfach oder mehrfach substituiert oder unsubstituiert, vorzugsweise H, Methyl- Ethyl-, Propyl-, Isopropyl-, Butyloder Isobutyl-, insbesondere H;

$C(O)NR^{25}R^{26}$, wobei $R^{25}$ und $R^{26}$ unabhängig voneinander ausgewählt sind aus

H, O, $C_1$-$C_{18}$-Alkyl, insbesondere $C_1$-$C_4$-Alkyl, verzweigt oder unverzweigt, gesättigt oder ungesättigt, einfach oder mehrfach substituiert oder unsubstituiert, vorzugsweise H, Methyl-Ethyl-, Propyl-, Isopropyl-, Butyl- oder Isobutylwährend $R^1$, $R^{1'}$, $R^3$, $R^4$, $R^5$ und X sowie $R^{18}$, $R^{19}$ und $R^{20}$ eine der bereits für sie genannten Bedeutungen haben.

In weiteren besonders bevorzugten erfindungsgemäßen substituierten Aminomethyl-Phenyl-Cyclohexanderivaten gemäß der allgemeinen Formel I bzw. Ia

ist $R^1$ ausgewählt aus

H, F, Cl, Br, I, $CF_3$, $SCH_3$ oder $OR^6$, vorzugsweise $OR^6$, wobei $R^6$ ausgewählt ist aus

H; $C_1$-$C_4$-Alkyl, verzweigt oder unverzweigt, einfach oder mehrfach substituiert oder unsubstituiert; vorzugsweise H oder $CH_3$;

und/oder

ist $R^{1'}$ ausgewählt aus

H, F, Cl, $SCH_3$ oder $OCH_3$, vorzugsweise H

und/oder

ist X ausgewählt aus

H, F, Br, I, Cl, $OR^7$, vorzugsweise H, F, Cl, $OR^7$, mit $R^7$ = H oder $CH_3$, vorzugsweise H;

oder,

wenn die Verbindung kein X aufweist, gemäß Formel Ia zwischen C-Atom A und C-Atom B oder C-Atom B und C-Atom C eine Doppelbindung ausgebildet ist;

und/oder

sind $R^4$ und $R^5$ unabhängig voneinander ausgewählt aus $C_1$-$C_4$-Alkyl, verzweigt oder unverzweigt, einfach oder mehrfach substituiert oder unsubstituiert, vorzugsweise $CH_3$,

während $R^2$ und $R^3$ eine der bereits für sie genannten Bedeutungen haben.

Besonders bervorzugt sind die folgenden erfindungsgemäßen substituierten Aminomethyl-Phenyl-Cyclohexanderivate:

*rac-cis-E*-[-4-Benzyliden-2-(3-methoxy-phenyl)-cyclohexylmethyl]-dimethylamin,
*rac-trans-E*-[4-Benzyliden-2-(3-methoxy-phenyl)-cyclohexylmethyl]-dimethylamin,

*rac-trans-Z*-[4-Benzyliden-2-(3-methoxy-phenyl)-cyclohexylmethyl]-dimethylamin,
*rac-cis-Z*-[4-Benzyliden-2-(3-methoxy-phenyl)-cyclohexylmethyl]-dimethylamin,
*rac-cis-E*-3-[4-Dimethylaminomethyl-3-(3-methoxy-phenyl)-cyclohexylidenmethyl]-benzoesäuremethylester,
*rac-cis*-Z-3-[4-Dimethylaminomethyl-3-(3-methoxy-phenyl)-cyclohexylidenmethyl]-benzoesäuremethylester,
*rac-trans-Z*-3-[4-Dimethylaminomethyl-3-(3-methoxy-phenyl)-cyclohexylidenmethyl]-benzoesäuremethylester,
*rac-trans-E*-3-[4-Dimethylaminomethyl-3-(3-methoxy-phenyl)-cyclohexylidenmethyl]-benzoesäuremethylester,
*Z*-3-[4-Dimethylaminomethyl-3-(3-methoxy-phenyl)-2-methyl-cyclohex-2-enylidenmethyl]-benzoe-säuremethylester,
*E*-3-[4-Dimethylaminomethyl-3-(3-methoxy-phenyl)-2-methyl-cyclohex-2-enylidenmethyl]-benzoesäuremethylester,
*Z*-3-[4-Dimethylaminomethyl-3-(3-methoxy-phenyl)-2-methyl-cyclohex-2-enylidenmethyl]-naphthalen-1-carbonsäureethylester,
*E*-3-[4-Dimethylaminomethyl-3-(3-methoxy-phenyl)-2-methyl-cyclohex-2-enylidenmethyl]-naphthalen-1-carbonsäureethylester,
*Z*-3-[4-Dimethylaminomethyl-3-(3-methoxy-phenyl)-cyclohexylidenmethyl]-2-fluor-benzoesäureethylester
*Z*-3-[4-Dimethylaminomethyl-3-(3-methoxy-phenyl)-cyclohex-2-enylidenmethyl]-benzoesäure,
*E*-3-[4-Dimethylaminomethyl-3-(3-methoxy-phenyl)-cyclohex-2-enylidenmethyl]-benzoesäure,
*E*-{3-[4-Dimethylaminomethyl-3-(3-methoxy-phenyl)-cyclohex-2-enylidenmethyl]-phenyl}-methanol,
*rac-trans-Z*-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäure,
*rac-trans-E*-(3-(2-Dimethylaminomethyl-5-(3-hydroxymethyl-benzyliden)-cyclohexyl)-phenol,
*rac-trans-E*-3-(5-Benzyliden-2-dimethylaminomethyl-cyclohexyl)-phenol,
*E*-3-[4-Dimethylaminomethyl-3-(3-methoxy-phenyl)-cyclohex-2-enylidenmethyl]-benzoesäure-tert.-butylester,
*E*-3-[6-Dimethylaminomethyl-3-(3-hydroxymethyl-benzyliden)-cyclohex-1-enyl]-phenol,
*rac-trans*-Z-3-(5-Benzyliden-2-dimethylaminomethyl-cyclohexyl)-phenol,Z-3-[2-Dimethylaminomethyl-5-(3-hydroxymethyl-benzyliden)-cyclohex-1-enyl]-phenol,
*Z*-{3-[4-Dimethylaminomethyl-3-(3-methoxy-phenyl)-cyclohex-2-enylidenmethyl]-phenyl}-methanol,
*rac-cis-E*-4-Dimethylaminomethyl-3-(3-methoxy-phenyl)-cyclohexylidenmethyl]-benzoesäure,
*rac-cis-Z*-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäuretert-butylester,
*rac-trans-E*-3-[4-Dimethylaminomethyl-3-(3-methoxy-phenyl)-cyclohexylidenmethyl)-phenyl)-methanol],
*rac-cis-Z*-3-[4-Dimethylaminomethyl-3-(3-methoxy-phenyl)-cyclohexylidenmethyl]-benzoesäureethylester,
*E*-3-[4-Dimethylaminomethyl-3-(3-methoxy-phenyl)-cyclohex-2-enylidenmethyl]-N,N-diethyl-benzamid
*rac-trans-E*-3-[4-Dimethylaminomethyl-3-(3-methoxy-phenyl)-cyclohex-2-enylidenmethyl]-N,N-diethyl-benzamid,
*E*-3-[4-Dimethylaminomethyl-3-(3-methoxy-phenyl)-cyclohex-2-enylidenmethyl]-benzoesäureethylester
*Z*-3-[4-Dimethylaminomethyl-3-(3-methoxy-phenyl)-cyclohex-2-enylidenmethyl]-N,N-diethyl-benzamid,
*rac-cis-E*-3-[4-Dimethylaminomethyl-3-(3-methoxy-phenyl)-cyclohexylidenmethyl]-N,N-diethyl-benzamid,
*rac-trans-Z*-3-[4-Dimethylaminomethyl-3-(3-methoxy-phenyl)-cyclohexylidenmethyl]-N,N-diethyl-benzamid,
*E*-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-methoxy-phenyl)-cyclohex-2-enylidenmethyl]-benzoesäureisobutylester,
Z-3-[6-Dimethylaminomethyl-3-(3-hydroxymethyl-benzyliden)-cyclohex-1-enyl]-phenol
*Z*-[4-(4-Chloro-benzyliden)-2-(3-methoxy-phenyl)-cyclohex-2-enylmethyl]-dimethyl-amin,
*E-[*4-(4-Fluoro-benzyliden)-2-(3-methoxy-phenyl)-cyclohex-2-enylmethyl]-dimethyl-amin,
*Z*-[4-(4-Fluoro-benzyliden)-2-(3-methoxy-phenyl)-cyclohex-2-enylmethyl]-dimethyl-amin,
*E*-3-[6-Dimethylaminomethyl-3-(4-fluor-benzyliden)-cyclohex-1-enyl]-phenol,
*Z*-3-[6-Dimethylaminomethyl-3-(4-fluor-benzyliden)-cyclohex-1 -enyl]-phenol,
*E*-[4-(4-Chlor-benzyliden)-2-(3-methoxy-phenyl)-cyclohex-2-enylmethyl]-dimethyl-amin
*E*-3-[3-(4-Chlor-benzyliden)-6-dimethylaminomethyl-cyclohex-1-enyl]-phenol,
*Z*-3-[4-Dimethylaminomethyl-3-(3-hydroxy-phenyl)-cyclohex-2-enylidenmethyl]-benzoesäuremethylester,
*E*-3-[4-Dimethylaminomethyl-3-(3-hydroxy-phenyl)-cyclohex-2-enylidenmethyl]-benzoesäure,
*rac-cis-E*-3-[4-Dimethylaminomethyl-3-(3-hydroxy-phenyl)-cyclohexylidenmethyl]-benzoesäuremethylester,
*rac-cis-Z*-3-[4-Dimethylaminomethyl-3-(3-hydroxy-phenyl)-cyclohexylidenmethyl]-benzoesäuremethylester,
*rac-trans-Z*-3-[4-Dimethylaminomethyl-3-(3-hydroxy-phenyl)-cyclohexylidenmethyl]-benzoesäure,
*rac-trans-E*-[4-Dimethylaminomethyl-3-(3-hydroxy-phenyl)-cyclohexylidenmethyl]-benzoesäure,
rac-cis-Z-3-[4-Dimethylaminomethyl-3-(3-hydroxy-phenyl)-cyclohexylidenmethyl]-benzoesäure,
rac-cis-Z-3-[4-Dimethylaminomethyl-3-hydroxy-3-(3-methoxy-phenyl)-cyclohexylidenemethyl]-benzoesäuremethylester,

rac-cis-E-3-[3-Chloro-4-dimethylaminomethyl-3-(3-methoxy-phenyl)-cyclohexylidenmethyl]-benzoesäuremethylester,
rac-cis-E-3-[4-Dimethylaminomethyl-3-hydroxy-3-(3-methoxy-phenyl)-cyclohexylidenmethyl]-benzoesäuremethylester,
rac-cis-Z-3-[4-Dimethyfaminomethyl-3-hydroxy-3-(3-methoxy-phenyl)-cyclohexylidenemethyl]-benzoesäuremethylester,
(+}-trans-E-3-[4-Dimethylaminomethyl-3-(3-methoxy-phenyl)-cyclohexylidenmethyl]-benzoesäure,
(-)-trans-E-3-[4-Dimethylaminomethyl-3-(3-methoxy-phenyl)-cyclohexylidenmethyl]-benzoesäure,
rac-trans-E-3-(4-Dimethylaminomethyl-3-phenylcyclohexylidenemethyl)-benzoesäuremethylester,
rac-trans-Z-3-(4-Dimethylaminomethyl-3-phenyl-cyclohexylidenmethyl)-benzoesäuremethylester,
rac-cis-E-3-(4-Dimethylaminomethyl-3-phenyl-cyclohexylidenmethyl)-benzoesäure,
rac-cis-E-3-(4-Dimethylaminomethyl-3-phenyl-cyclohexylidenmethyl)-benzoesäure,
rac-trans-Z-3-(4-Dimethylaminomethyl-3-phenylcyclohexylidenmethyl)benzoesäure,
rac-trans-E-3-(4-Dimethylaminomethyl-3-phenylcyclohexylidenemethyl)-benzoesäure,
rac-trans-E-3-[4-Dimethylaminomethyl-3-(3-trifluoromethyl-phenyl)-cyclohexylidenmethyl]-benzoesäure,
rac-trans-Z-3-[4-Dimethylaminomethyl-3-(3-trifiuoromethyl-phenyl)-cyclohexylidenmethyl]-benzoesäure,
rac-trans-E-3-[4-Dimethylaminomethyl-3-(3-fluoro-phenyl)-cyclohexylidenmethyl]-benzoesäuremethylester,
rac-trans-Z-3-[4-Dimethylaminomethyl-3-(3-fluoro-phenyl)-cyclohexylidenmethyl]benzoesäuremethylester,
rac-cis-E-3-[4-Dimethylaminomethyl-3-(3-fluoro-phenyl)-cyclohexylidenemethyl]-benzoesäuremethylester,
rac-trans-E-3-[4-Dimethylaminomethyl-3-(3-fluoro-phenyl)-cyclohexylidenemethyl]-benzoesäure,
rac-trans-Z-3-[4-Dimethylaminomethyl-3-(3-fluoro-phenyl)-cyclohexylidenemethyl]-benzoesäure,
rac-cis-E-3-[4-Dimethylaminomethyl-3-(3-fluoro-phenyl)-cyclohexyfidenemethyl]-benzoesäure
E-[4-Ethyliden-2-(3-methoxy-phenyl)-cyclohex-2-enylmethyl]-dimethylamin
[4-Isopropyliden-2-(3-methoxy-phenyl)-cyclohex-2-enylmethyl]-dimethylamin,
E-[2-(3-Methoxy-phenyl)-4-propyliden-cyclohex-2-enylmethyl]-dimethylamin,
E-[4-Butyliden-2-(3-methoxy-phenyl)-cyclohex-2-enylmethyl]-dimethylamin

sowie deren Salze, insbesondere Hydrochloridsalze.

Ein weiterer Gegenstand der Erfindung ist ein Verfahren zur Herstellung substituierter Aminomethyl-Phenyl-Cyclohexanderivate der allgemeinen Formel I bzw. Ia

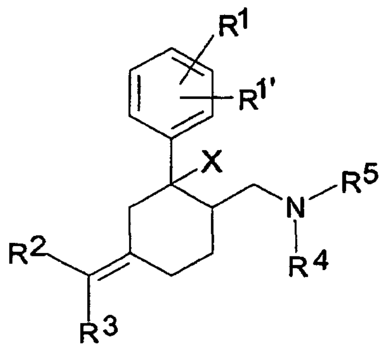

I

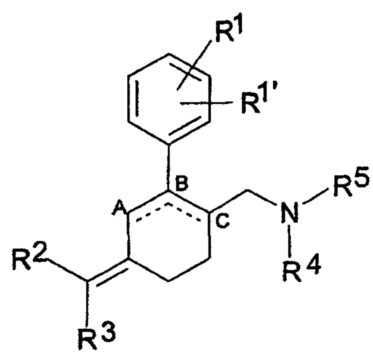

Ia

, in denen $R^1$, $R^{1'}$, $R^2$, $R^3$, $R^4$, $R^5$ und X eine der bereits genannten Bedeutungen haben, bei dem Cyclohexanone der Formel II bzw. IIa

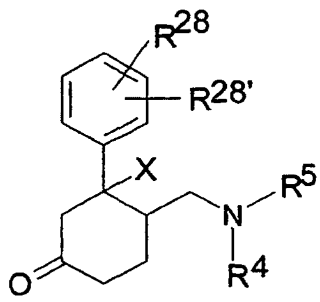

**II**

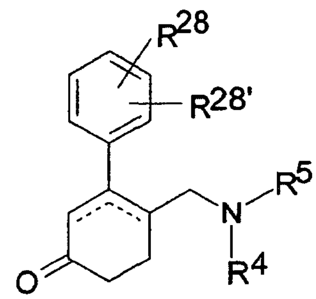

**IIa**

in denen $R^4$, $R^5$ und X eine der bereits genannten Bedeutungen haben und $R^{28}$ eine der bereits für $R^1$ genannten Bedeutungen und $R^{28'}$ eine der bereits für $R^{1'}$ genannten Bedeutungen hat,
in einer Wittig-Reaktion in einem organischen Lösungsmittel in Gegenwart einer Base mit Alkyltriphenylphosphoniumsalzen der Formel III umgesetzt werden,

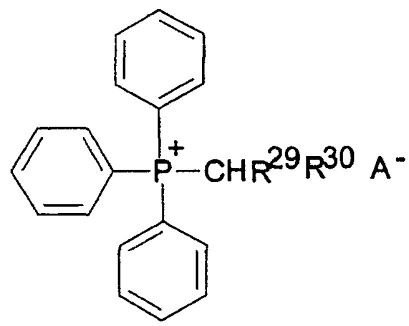

**III**

, wobei A Chlorid oder Bromid bedeutet und unabhängig voneinander $R^{29}$ eine der für $R^2$ und $R^{30}$ eine der für $R^3$ bereits genannten Bedeutungen haben.

Bevorzugte organische Lösungsmittel sind dabei Benzol, Toluol oder ein chlorierter Kohlenwasserstoff und als Base werden vorzugsweise Kaliumtert.butylat oder Natriumhydrid verwendet. Weiter wird die Reaktionstemperatur, insbesondere während der Wittig-Reaktion, vorzugsweise zwischen 50°C und 90°C gehalten.

Unter den genannten Reaktionsbedingungen können OH- SH und $NH_2$-Gruppen unerwünschte Nebenreaktionen eingehen. Es ist daher bevorzugt, diese mit Schutzgruppen zu versehen oder im Falle von $NH_2$ durch $NO_2$ zu ersetzen und nach der Wittig-Reaktion die Schutzgruppe abzuspalten, bzw. die $NO_2$-Gruppe zu reduzieren. Ein weiterer Gegenstand der Anmeldung ist daher eine Abwandlung des oben beschriebenen Verfahrens, bei dem in $R^{28}$ und/oder $R^{28'}$ gemäß Formel II bzw. IIa und/oder $R^{29}$ und/oder $R^{30}$ gemäß Formel III, mindestens eine OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wurde und nach Abschluß der Wittig-Reaktion mindestens eine $OSi(Ph)_2$tert.but.-Gruppe mit Tetrabutylammoniumflluorid in Tetrahydrofuran und/oder mindestens eine p-Methoxybenzylgruppe mit einem Metallamin bevorzugt Natriumamin abgespalten und/oder mindestens eine $NO_2$-Gruppe zu $NH_2$ reduziert wird.

Weiter sind Carbonsäure- oder Thiocarbonsäure-Gruppen unter den Bedingungen der Wittig-Reaktion unter Umständen nicht stabil, so daß es bevorzugt ist, deren Methylester in der Wittigreaktion umzusetzen und das Verfahrensprodukt aus der Wittigreaktion anschließend mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem nach der Wittig-Reaktion ein Verfahrensprodukt mit mindestens einer $C(O)OCH_3$- $OC(O)OCH_3$- und/oder $C(S)OCH_3$-Gruppe mit KOH-Lösung bzw. NaOH-Lösung in Methanol bei 40°C - 60°C verseift wird.

Die Reaktionen verlaufen spezifisch und mit hohen Ausbeuten. Trotzdem ist meist eine Aufreinigung der in den einzelnen Reaktionssequenzen erhaltenen Verbindungen, insbesondere des Endprodukts erforderlich. Die Reinigung erfolgt bevorzugt über Kristallisation oder chromatographische Methoden, insbesondere Säulenchromatographie.

Die Verbindungen der Formel I bzw. Ia lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Die Cyclohexanone der Formeln II bzw. IIa werden dargestellt, indem man zunächst 3,3-Dimethyl-1,5-dioxaspiro[5.5]undecan-8-on mit Immoniumsalzen der Formel IV bzw. mit Formaldehyd und einem Amin der Formel V umsetzt. Anschließend setzt man die so erhaltenen Mannich-Basen mit einer metallorganischen Verbindung der Formel **VI**, in der Z MgCl, MgBr, MgI oder Lithium bedeuten, um. Die Umsetzung der Mannich-Basen mit einer Grignard-Verbindung der Formel **VI** in der Z MgCl, MgBr oder MgI bedeutet oder mit einer lithiumorganischen Verbindung der Formel **VI** in der Z Li ist, kann in einem aliphatischen Ether beispielsweise Diethylether und/oder Tetrahydrofuran bei Temperaturen zwischen -70°C und 60°C durchgeführt werden. Die Umsetzung mit einer Grignard-Verbindung der Formel **VI** kann mit oder ohne Zusatz eines Mitführreagenzes erfolgen. Bei Einsatz eines Mitführreagenzes ist 1,2-Dibromethan bevorzugt.

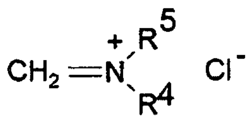

$R^4 R^5 NH$

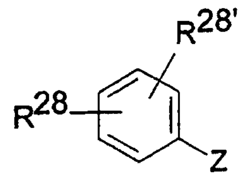

**IV** **V** **VI**

Man erhält so zunächst Produkte der allgemeinen Formel **VII.**

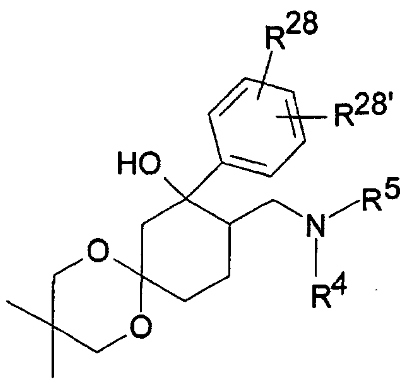

**VII**

Verbindungen der Formel **IIa** erhält man, indem man Produkte der allgemeinen Formel **VII** mit Säuren beispielsweise Salzsäure, Ameisensäure, Essigsäure, bei Raumtemperatur umsetzt. Anschließende Hydrierung der so erhaltenen Produkte mit katalytisch aktiviertem Wasserstoff, wobei Platin oder Palladium, absorbiert auf einem Trägermaterial wie Aktivkohle, als Katalysator dienen, führt zu Verbindungen der Formel **II** mit X gleich H. Die Hydrierung wird in einem Lösungsmittel wie Essigsäureethylester oder einem $C_1$ - $C_4$-Alkylalkohol bei Drücken von 0,1 bis 10 bar und Temperaturen von 20°C bis 80°C durchgeführt.

Verbindungen der allgemeinen Formel **II** mit X gleich OH, erhält man, indem man Produkte der allgemeinen Formel **VII** mit Säuren beispielsweise Salzsäure bei Temperaturen zwischen 5°C und 10°C umsetzt.

Verbindungen der allgemeinen Formel **II** mit X gleich F, Cl, Br, I bzw. $CF_3$, erhält man durch Austausch von OH gegen F bzw. Cl bzw. Br bzw. I bzw. $CF_3$ nach bekannten Verfahren.

Verbindungen der allgemeinen Formel **II** mit X gleich $OR^7$, erhält man durch Veretherung der OH-Gruppe mit einem Halogenid der Formel **VIII.**

$$R^7Cl$$

**VIII**

Verbindungen der allgemeinen Formel **II** mit X gleich $OC(O)R^7$ erhält man durch Veresterung der OH-Gruppe mit einem Säurechlorid der Formel **IX.**

$$R^7COCl$$

**IX**

Die meisten der im Herstellungsverfahren verwendeten substituierten Alkyltriphenylphosphoniumsalze sind käuflich zu erwerben. In einigen Ausnahmefällen müssen diese allerdings synthetisiert werden. Beispielhaft für die Synthese der substituierten Alkyltriphenylphosphoniumsalzen sei 3-(Benzoesäuremethylester)-methyltriphenylphosphoniumbromid genannt:

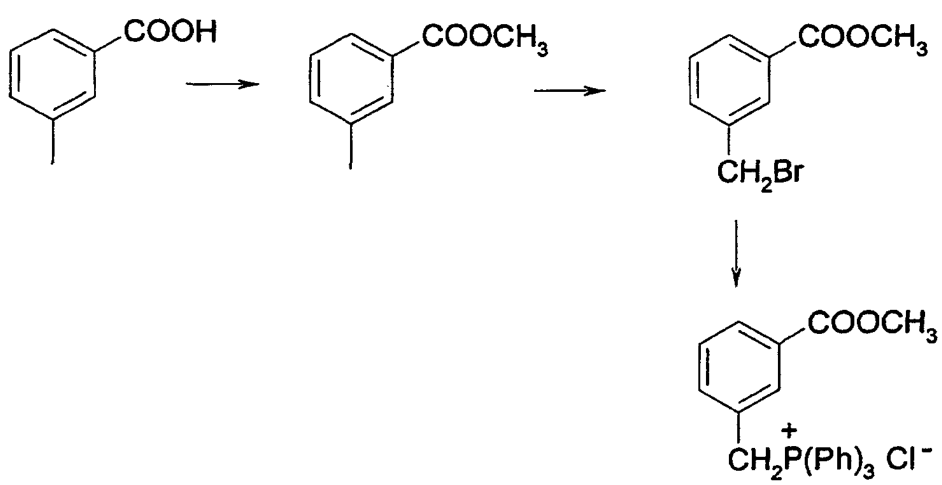

Dieses wird folgendermaßen hergestellt: 3-Toluylsäure wird mit Methanol in Gegenwart von Schwefelsäure in 3-Toluylsäuremethylester überführt, der mit N-Bromsuccinimid zu 3-Brommethyl-benzoesäuremethylester reagiert. Umsetzung von Brommethyl-benzoesäuremethylester mit Triphenylphosphan führt schließlich zu 3-(Benzoesäuremethylester)-methyltriphenylphosphoniumbromid.

Die erfindungsgemäßen substituierten Aminomethyl-Phenyl-Cyclohexanderivate sind toxikologisch unbedenklich, so daß sie sich als pharmazeutischer Wirkstoff in Arzneimitteln eignen.

Ein weiterer Gegenstand der Erfindung sind daher auch Arzneimittel, die als Wirkstoff mindestens ein substituiertes Aminomethyl-Phenyl-Cyclohexanderivat der allgemeinen Formel I bzw. Ia

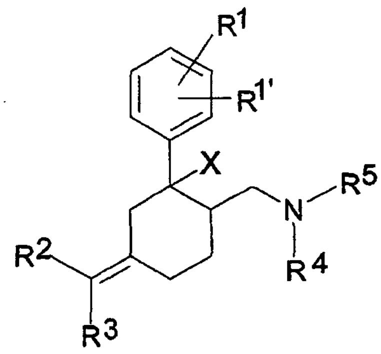

I

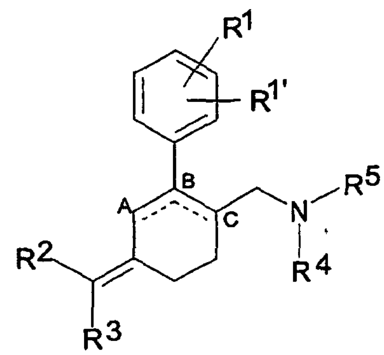

Ia

, in der $R^1$, $R^{1'}$, $R^2$, $R^3$, $R^4$, $R^5$ und X eine der in Anspruch 1 genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, enthalten.

Besonders bevorzugt sind dabei Arzneimittel, die als Wirkstoff mindestens ein aus folgender Gruppe ausgewähltes substituiertes Aminomethyl-Phenyl-Cyclohexanderivat enthalten:

*rac-cis-E*-[-4-Benzyliden-2-(3-methoxy-phenyl)-cyclohexylmethyl]-dimethylamin,
*rac-trans-E*-[4-Benzyliden-2-(3-methoxy-phenyl)-cyclohexylmethyl]-dimethylamin,
*rac-trans-Z* [4-Benzyliden-2-(3-methoxy-phenyl)-cyclohexylmethyl]-dimethylamin,
rac-cis-Z-[4-Benzyliden-2-(3-methoxy-phenyl)-cyclohexylmethyl]-dimethylamin,
*rac-cis-E* 3-[4-Dimethylaminomethyl-3-(3-methoxy-phenyl)-cyclohexylidenmethyl]-benzoesäuremethylester,
rac-cis-Z-3-[4-Dimethylaminomethyl-3-(3-methoxy-phenyl)-cyclohexylidenmethyl]-benzoesäuremethylester,
*rac-trans*-Z-3-[4-Dimethylaminomethyl-3-(3-methoxy-phenyl)-cyclohexylidenmethyl]-benzoesäuremethylester,
*rac-trans*-E-3-[4-Dimethylaminomethyl-3-(3-methoxy-phenyl)-cyclohexylidenmethyl]-benzoesäuremethylester,
*Z*-3-[4-Dimethylaminomethyl-3-(3-methoxy-phenyl)-2-methyl-cyclohex-2-enylidenmethyl]-benzoe-säuremethylester,
*E*-3-[4-Dimethylaminomethyl-3-(3-methoxy-phenyl)-2-methyl-cyclohex-2-enylidenmethyl]-benzoesäuremethylester,
*Z*-3-[4-Dimethylaminomethyl-3-(3-methoxy-phenyl)-2-methyl-cyclohex-2-enylidenmethyl]-naphthalen-1-carbonsäureethylester,
*E*-3-[4-Dimethylaminomethyl-3-(3-methoxy-phenyl)-2-methyl-cyclohex-2-enylidenmethyl]-naphthalen-1-carbonsäureethylester,
*Z*-3-[4-Dimethylaminomethyl-3-(3-methoxy-phenyl)-cyclohexylidenmethyl]-2-fluor-benzoesäureethylester
*Z*-3-[4-Dimethylaminomethyl-3-(3-methoxy-phenyl)-cyclohex-2-enylidenmethyl]-benzoesäure,
*E*-3-[4-Dimethylaminomethyl-3-(3-methoxy-phenyl)-cyclohex-2-enylidenmethyl]-benzoesäure,
*E*-{3-[4-Dimethylaminomethyl-3-(3-methoxy-phenyl)-cyclohex-2-enylidenmethyl]-phenyl}-methanol,
*rac-trans*-Z-3-[4-Dimethylaminomethyl-3-(3-methoxy-phenyl)-cyclohexylidenmethyl]-benzoesäure,
*rac-trans*-E-3-[4-Dimethylaminomethyl-3-(3-methoxy-phenyl)-cyclohexylidenmethyl]-benzoesäure,
*rac-trans-E*-[3-(2-Dimethylaminomethyl-5-(3-hydroxymethyl-benzyliden)-cyclohexyl)-phenol,
*rac-trans-E*-3-(5-Benzyliden-2-dimethytaminomethyl-cyclohexyl)-phenol,
*E*-3-[4-Dimethylaminomethyl-3-(3-methoxy-phenyl)-cyclohex-2-enylidenmethyl]-benzoesäure-tert.-butylester,
*E*-3-[6-Dimethylaminomethyl-3-(3-hydroxymethyl-benzyliden)-cyclohex-1-enyl]-phenol,
*rac-trans-Z*-3-(5-Benzyliden-2-dimethylaminomethyl-cyclohexyl)-phenol,
*Z*-3-[2-Dimethylaminomethyl-5-(3-hydroxymethyl-benzyliden)-cyclohex-1-enyl]-phenol,
*Z*-{3-[4-Dimethylaminomethyl-3-(3-methoxy-phenyl)-cyclohex-2-enylidenmethyl]-phenyl}-methanol,
*rac-cis*-E-4-Dimethylaminomethyl-3-(3-methoxy-phenyl)-cyclohexylidenmethyl]-benzoesäure,
*rac-cis*-Z-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäuretert-butylester,
*rac-trans-E-[*3-[4-Dimethylaminomethyl-3-(3-methoxy-phenyl)-cyclohexylidenmethyl)-phenyl)-methanol],
*rac-cis-Z*-3-[4-Dimethylaminomethyl-3-(3-methoxy-phenyl)-cyclohexylidenmethyl]-benzoesäureethylester,
*E*-3-[4-Dimethylaminomethyl-3-(3-methoxy-phenyl)-cyclohex-2-enylidenmethyl]-N,N-diethyl-benzamid
*rac-trans-E*-3-[4-Dimethylaminomethyl-3-(3-methoxy-phenyl)-cyclohex-2-enylidenmethyl]-N,N-diethyl-benzamid,

*E*-3-[4-Dimethylaminomethyl-3-(3-methoxy-phenyl)-cyclohex-2-enylidenmethyl]-benzoesäureethylester
*Z*-3-[4-Dimethylaminomethyl-3-(3-methoxy-phenyl)-cyclohex-2-enylidenmethyl]-N,N-diethyl-benzamid,
*rac-cis-E*-3-[4-Dimethylaminomethyl-3-(3-methoxy-phenyl)-cyclohexylidenmethyl]-N,N-diethyl-benzamid,
*rac-trans*-Z-3-[4-Dimethylaminomethyl-3-(3-methoxy-phenyl)-cyclohexylidenmethyl]-N,N-diethyl-benzamid,
*E*-3-[4-Dimethylaminomethyl-3-(3-hydroxy-phenyl)-cyclohex-2-enylidenmethyl]-benzoesäuremethylester,
Z-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-methoxy-phenyl)-cyclohex-2-enylidenmethyl]-benzoesäureisobutylester,
*Z*-3-[6-Dimethylaminomethyl-3-(3-hydroxymethyl-benzyliden)-cyclohex-1-enyl]-phenol
*Z*-[4-(4-Chloro-benzyliden)-2-(3-methoxy-phenyl)-cyclohex-2-enylmethyl]-dimethyl-amin,
*E*-[4-(4-Fluoro-benzyliden}-2-(3-methoxy-phenyl)-cyclohex-2-enylmethyl]-dimethyl-amin,
*Z*-[4-(4-Fluoro-benzyliden)-2-(3-methoxy-phenyl)-cyclohex-2-enylmethyl]-dimethyl-amin,
*E*-3-[6-Dimethylaminomethyl-3-(4-fluor-benzyliden)-cyclohex-1-enyl]-phenol,
*Z*-3-[6-Dimethylaminomethyl-3-(4-fluor-benzyliden)-cyclohex-1-enyl]-phenol,
*E*-[4-(4-Chlor-benzyliden)-2-(3-methoxy-phenyl)-cyclohex-2-enylmethyl]-dimethyl-amin
*E*-3-[3-(4-Chlor-benzyliden)-6-dimethylaminomethyl-cyclohex-1-enyl]-phenol,
*Z*-3-[4-Dimethylaminomethyl-3-(3-hydroxy-phenyl)-cyclohex-2-enylidenmethyl]-benzoesäuremethylester,
*E*-3-[4-Dimethylaminomethyl-3-(3-hydroxy-phenyl)-cyclohex-2-enylidenmethyl]-benzoesäure,
*rac-cis-E*-3-[4-Dimethylaminomethyl-3-(3-hydroxy-phenyl)-cyclohexylidenmethyl]-benzoesäuremethylester,
*rac-cis-Z*-3-[4-Dimethylaminomethyl-3-(3-hydroxy-phenyl)-cyclohexylidenmethyl]-benzoesäuremethylester,
*rac-trans*-Z-3-[4-Dimethylaminomethyl-3-(3-hydroxy-phenyl)-cyclohexylidenmethyl]-benzoesäure,
*rac-trans-E*-[4-Dimethylaminomethyl-3-(3-hydroxy-phenyl)-cyclohexylidenmethyl]-benzoesäure,
rac-cis-Z-3-[4-Dimethylaminomethyl-3-(3-hydroxy-phenyl)-cyclohexylidenmethyl]-benzoesäure,
rac-cis-Z-3-[4-Dimethylaminomethyl-3-hydroxy-3-(3-methoxy-phenyl)-cyclohexylidenemethyl]-benzoesäuremethylester,
rac-cis-E-3-[3-Chloro-4-dimethylaminomethyl-3-(3-methoxy-phenyl)-cyclohexylidenmethyl]-benzoesäuremethylester,
rac-cis-E-3-[4-Dimethylaminomethyl-3-hydroxy-3-(3-methoxy-phenyl)-cyclohexylidenmethyl]-benzoesäuremethylester,
rac-cis-Z-3-[4-Dimethylaminomethyl-3-hydroxy-3-(3-methoxy-phenyl)-cyclohexylidenemethyl]-benzoesäuremethylester,
(+)-trans-E-3-[4-Dimethylaminomethyl-3-(3-methoxy-phenyl)-cyclohexylidenmethyl]-benzoesäure,
(-)-trans-E-3-[4-Dimethylaminomethyl-3-(3-methoxy-phenyl)-cyclohexylidenmethyl]-benzoesäure,
rac-trans-E-3-(4-Dimethylaminomethyl-3-phenylcyclohexylidenemethyl)-benzoesäuremethylester,
rac-trans-Z-3-(4-Dimethylaminomethyl-3-phenyl-cyclohexylidenmethyl)-benzoesäuremethylester,
rac-cis-E-3-(4-Dimethylaminomethyl-3-phenyl-cyclohexylidenemethyl)-benzoesäure,
rac-cis-E-3-(4-Dimethylaminomethyl-3-phenyl-cyclohexylidenmethyl)-benzoesäure,
rac-trans-Z-3-(4-Dimethylaminomethyl-3-phenylcyclohexylidenmethyl)benzoesäure,
rac-trans-E-3-(4-Dimethylaminomethyl-3-phenylcyclohexylidenemethyl)-benzoesäure,
rac-trans-E-3-[4-Dimethylaminomethyl-3-(3-trifluoromethyl-phenyl)-cyclohexylidenmethyl]-benzoesäure,
rac-trans-Z-3-[4-Dimethylaminomethyl-3-(3-trifluoromethyl-phenyl)-cyclohexylidenmethyl]-benzoesäure,
rac-trans-E-3-[4-Dimethylaminomethyl-3-(3-fluoro-phenyl)-cyclohexylidenmethyl]-benzoesäuremethylester,
rac-trans-Z-3-[4-Dimethylaminomethyl-3-(3-fluoro-phenyl)-cyclohexylidenmethyl]benzoesäuremethylester,
rac-cis-E-3-[4-Dimethylaminomethyl-3-(3-fluoro-phenyl)-cyclohexylidenemethyl]-benzoesäuremethylester,
rac-trans-E-3-[4-Dimethylaminomethyl-3-(3-fluoro-phenyl)-cyclohexylidenemethyl]-benzoesäure,
rac-trans-Z-3-[4-Dimethylaminomethyl-3-(3-fluoro-phenyl)-cyclohexylidenemethyl]-benzoesäure,
rac-cis-E-3-[4-Dimethylaminomethyl-3-(3-fluoro-phenyl)-cyclohexylidenemethyl]-benzoesäure
E-[4-Ethyliden-2-(3-methoxy-phenyl)-cyclohex-2-enylmethyl]-dimethylamin
[4-Isopropyliden-2-(3-methoxy-phenylrcyclohex-2-enylmethyl)-dimethylamin,
E-[2-(3-Methoxy-phenyl)-4-propyliden-cyclohex-2-enylmethyl]-dimethylamin,
E-[4-Butyliden-2-(3-methoxy-phenyl)-cyclohex-2-enylmethyl]-dimethylamin

als freie Base oder in Form eines mit einer physiologisch verträglichen Säure gebildeten Salzes, insbesondere des Hydrochloridsalzes.

Die erfindungsgemäßen Arzneimittel enthalten neben mindestens einem erfindungsgemäßen substituierten Aminomethyl-Phenyl-Cyclohexanderivat Trägermaterialien Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Aminomethyl-Phenyl-Cyclohexanderivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Aminomethyl-Phenyl-Cyclohexanderivate verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblichweise werden 50 bis 500 mg/kg wenigstens eines erfindungsgemäßen substituierten Aminomethyl-Phenyl-Cyclohexanderivats appliziert.

Vorzugsweise werden die erfindungsgemäßen substituierten Aminomethyl-Phenyl-Cyclohexanderivate zur Schmerzbehandlung eingesetzt, so daß ein weiterer Erfindungsgegenstand die Verwendung mindestens eines substituierten Aminomethyl-Phenyl-Cyclohexanderivats der allgemeinen Formel I bzw. Ia

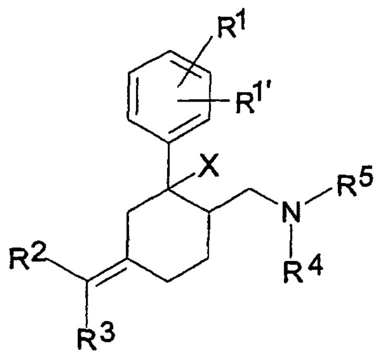

I

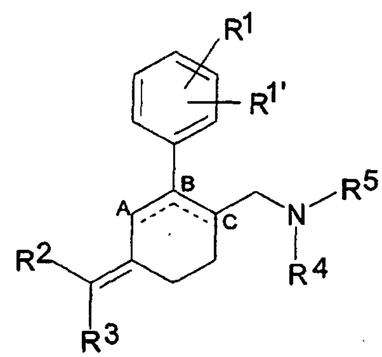

Ia

, in der $R^1$, $R^{1'}$, $R^2$, $R^3$, $R^4$, $R^5$ und X eine der bereits genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von Schmerz ist.

Überraschenderweise hat es sich herausgestellt, daß die erfindungsgemäßen substituierten Aminomethyt-Phenyl-Cyclohexanderivate für weitere Indikationen, insbesondere zur Behandlung von Harninkontinenz, Juckreiz und/oder Diarrhoe aber auch in andereren Indikationen sehr geeignet sind. Ein weiterer Gegenstand der Anmeldung ist daher die Verwendung mindestens eines substituierten Aminomethyl-Phenyl-Cyclohexanderivats der allgemeinen Formel I bzw. Ia

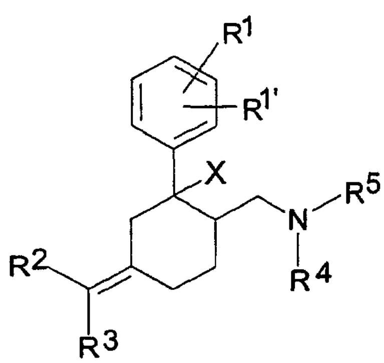

I

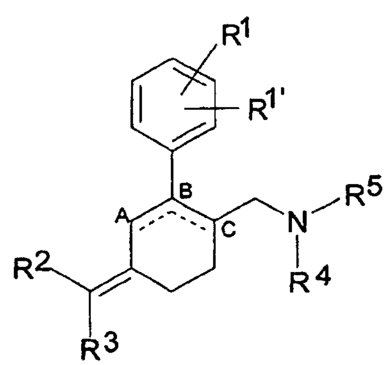

Ia

, in der $R^1$, $R^{1'}$, $R^2$, $R^3$, $R^4$, $R^5$ und X eine der bereits genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von inflammatorischen und allergischen Reaktionen, Depressionen, Drogen- und/oder Alkoholmißbrauch, Gastritis, cardiovaskulären Erkrankungen, Atemwegserkrankungen, Husten, seelischen Erkrankungen und/oder Epilepsie sowie insbesondere von Harninkontinenz, Juckreiz und/oder Diarrhoe.

Im folgenden wird die Erfindung weiter durch Beispiele erläutert, ohne sie darauf zu beschränken.

**Beispiele**

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Die Angabe Ether bedeutet Diethylether.

Soweit nicht anders angegeben, wurde Petrolether mit dem Siedebereich von 50°C-70°C benutzt.

Die Verbindungen sind numeriert, wobei die Angabe in Klammern grundsätzlich der Nummer der zugeordneten Verbindung entspricht.

**Beispiel 1**

Z-3-[4-Dimethylaminomethyl-3-(3-methoxy-phenyl)-2-methyl-cyclohex-2-enylidenmethyl]-benzoesäuremethylester Hydrochlorid (9) und *E*-3-[4-Dimethylaminomethyl-3-(3-methoxy-phenyl)-2-methyl-cyclohex-2-enylidenmethyl]-benzoesäuremethylester Hydrochlorid (10)

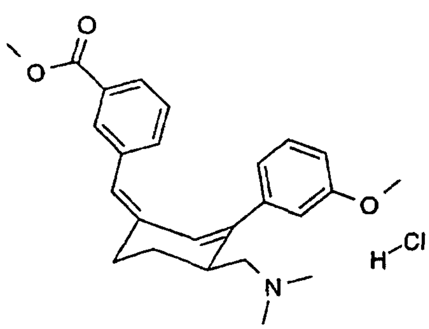

(9)

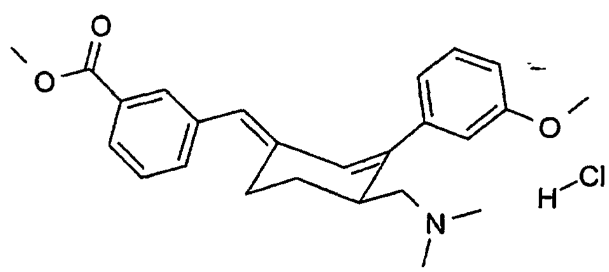

(10)

42.6 g Kalium-tert.butylat und 169.8 g 3-(Benzoesäuremethylester)-methyltriphenylphosphoniumchlorid wurden unter Stickstoffatmosphäre bei Raumtemperatur in 1.5 L Toluol p.a. suspendiert und anschließend eine Stunde bei 70 °C gerührt. Bei dieser Temperatur wurden 10 g 4-Dimethylaminomethyl-3-(3-methoxy-phenyl)-cyclo-hex-2-enon in 100 ml Toluol p.a. zugegeben und 3 Tage bei 70 °C gerührt. Das Gemisch wurde mit 500 ml Wasser gequenscht. Die Phasen wurden getrennt und die wässrige Phase wurde 3 Mal mit je 200 ml Essigsäureethylester gewaschen. Die vereinten organischen Phasen wurden über Magnesiumsulfat getrocknet und anschließend im Vakuum von Lösungsmittel befreit. Der so erhaltene Rückstand wurde in einem Gemisch aus 150 ml Essigsäureethylester und 150 ml

Diisoether aufgenommen. Der ausgefallene Feststoff wurde abfiltriert und mit Diisoether gewaschen. Die vereinten organischen Phasen wurden im Vakuum vom Lösemittel befreit. Der Rückstand wurde säulenchromatographisch an Kieselgel mit Essigsäureethylester/Methanol = 9/1 gereinigt. Als erste Produktfraktion erhielt man 5.3 g E-3-[4-Dimethylaminomethyl-3-(3-methoxy-phenyl)-2-methyl-cyclohex-2-enylidenmethyl]-benzoesäuremethylester in Form eines orange-gelben Öles. Zur Darstellung des Hydrochlorids wurde das Öl in 100 ml Aceton gelöst und mit einer äquimolaren Menge Trimethylchlorsilan und Wasser versetzt. Man erhielt so 5 g (30.7 % d. Th.) der Titelverbindung 10 in Form weißer Kristalle. Als zweite Produktfraktion erhielt man 4 g Z-3-[4-Dimethylaminomethyl-3-(3-methoxy-phenyl)-2-methyl-cyclohex-2-enylidenemethyl]-benzoesäuremethylester gleichfalls in Form eines orange-gelben Öles. Zur Freisetzung des Hydrochlorids wurde das Öl in 100 ml Aceton gelöst und mit einer äquimolaren Menge Trimethylchlorsilan und Wasser versetzt. Man erhielt so 3.5 g (21.5 % d. Th.) der Titelverbindung in Form weißer Kristalle.

**Beispiel 2:**

*rac-trans*-Z-3-[4-Dimethylaminomethyl-3-(3-methoxy-phenyl)-cyclohexylidenmethyl]-benzoesäuremethylester Hydrochlorid (7) und *rac-trans-E*-3-[4-Dimethylaminomethyl-3-(3-methoxy-phenyl)-cyclohexylidenmethyl]-benzoesäuremethylester Hydrochlorid (8)

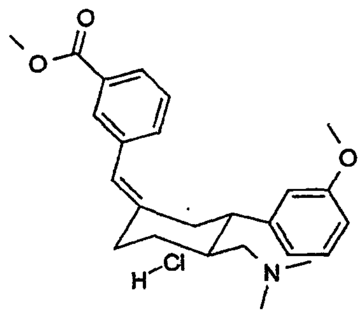

(7)

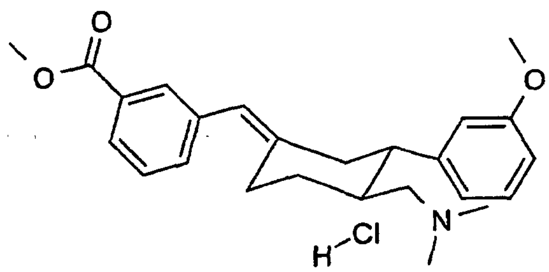

(8)

Unter Einsatz von:

*rac-trans*-4-Dimethylaminomethyl-3-(3-methoxy-phenyl)-cyclohexanon

anstelle von Dimethylaminomethyl-3-(3-methoxy-phenyl)-cyclo-hex-2-enon in Beispiel 1 erhielt man nach der in Beispiel 1 beschriebenen Verfahrensweise:

*rac-trans-E*-3-[4-Dimethylaminomethyl-3-(3-methoxy-phenyl)-cyclohexylidenemethyl]-benzoesäuremethylester Hydrochlorid (8)

*rac-trans-Z*-3-[4-Dimethylaminomethyl-3-(3-methoxy-phenyl)-cyclohexylidenmethyl]-benzoesäuremethylester Hydrochlorid (7)

**Beispiel 3:**

*rac-cis-Z*-3-[4-Dimethylaminomethyl-3-(3-methoxy-phenyl)-cyclohexylidenmethyl]-benzoesäuremethylester Hydrochlorid (6) und *rac-cis-E*-3-[4-Dimethylaminomethyl-3-(3-methoxy-phenyl)-cyclohexylidenmethyl]-benzoesäuremethylester; Hydrochlorid (5)

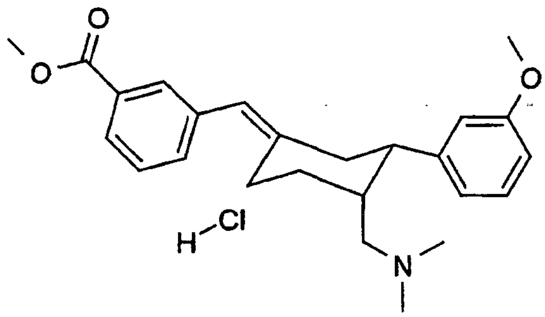

(5)

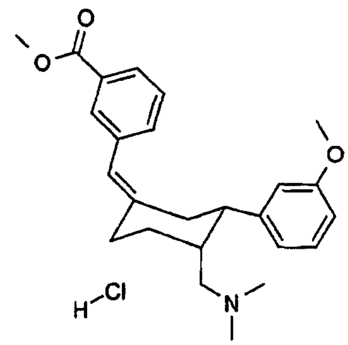

(6)

Unter Einsatz von *rac-cis*-4-Dimethylaminomethyl-3-(3-methoxyphenyl)-cyclohexanon anstelle von Dimethylaminomethyl-3-(3-methoxyphenyl)-cyclo-hex-2-enon in Beispiel 1 erhielt man nach der in Beispiel 1 beschriebenen Verfahrensweise:

*rac-cis-E* 3-[4-Dimethylaminomethyl-3-(3-methoxy-phenyl)-cyclohexylidenemethyl]-benzoesäuremethylester Hydrochlorid (5)
*rac-cis*-Z-*3*-[4-Dimethylaminomethyl-3-(3-methoxy-phenyl)-cyclohexylidenmethyl]-benzoesäuremethylester Hydrochlorid (6)

**Beispiel 4:**

*Z*-3-[4-Dimethylaminomethyl-3-(3-methoxy-phenyl)-cyclohex-2-enylidenmethyl]-benzoesäure Hydrochlorid (14)

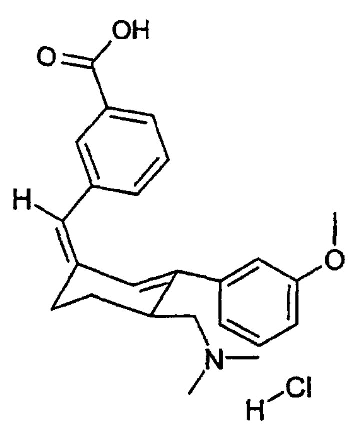

(14)

3 g des nach Beispiel 1 hergestellten Z-3-[4-Dimethylaminomethyl-3-(3-methoxy-phenyl)-2-methyl-cyclohex-2-enylidenemethyl]-benzoesäuremethylesters als Base wurden in 30 ml Methanol gelöst und mit 30 ml 1 n Kaliumhydroxid-Lösung versetzt. Es wurde 2 Stunden bei 60 °C gerührt. Nach Abkühlen der Reaktionsmischung auf Raumtemperatur wurde das Gemisch mit 1 n Salzsäure solange versetzt bis ein pH-Wert von 4 eingestellt wurde. Die Phasen wurden getrennt und die wäßrige Phase wurde 3 Mal mit je 20 ml Essigsäureethylester gewaschen. Die vereinten organischen Phasen wurden über Magnesiumsulfat getrocknet und im Vakuum vom Lösemittel befreit. Man erhielt so 2.7 g Z-3-[4-Dimethylaminomethyl-3-(3-methoxy-phenyl)-cyclohex-2-enylidenmethyl]-benzoesäure Base in Form eines orange-gelben Öles. Zur Darstellung des Hydrochlorids wurde die Base in 10 ml Aceton gelöst und mit einer äquimolaren Menge Trimethylchlorsilan und Wasser versetzt. So erhielt man 2.4 g ( 77.7 % d. Th.) Z-3-[4-Dimethylaminomethyl-3-(3-methoxy-phenyl)-cyclohex-2-enylidenmethyl]-benzoesäure Hydrochlorid (14) in Form weißer Kristalle.

**Beispiel 5:**

*E*-3-[4-Dimethylaminomethyl-3-(3-methoxy-phenyl)-cyclohex-2-enylidenmethyl]-benzoesäure Hydrochlorid (15)

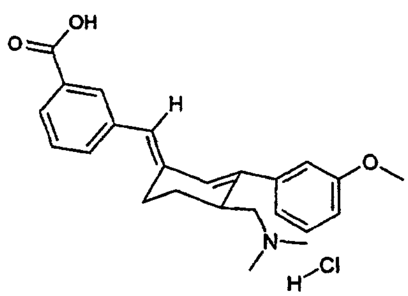

(15)

Unter Einsatz von:

*E*-3-[4-Dimethylaminomethyl-3-(3-methoxy-phenyl)-2-methyl-cyclohex-2-enylidenmethyl]-benzoesäuremethylester Base, das gemäß Beispiel 1 hergestellt wurde,

anstelle von *Z*-3-[4-Dimethylaminomethyl-3-(3-methoxy-phenyl)-2-methyl-cyclohex-2-enylidenemethyl]-benzoesäuremethylester Base in Beispiel 4 erhielt man nach der in Beispiel 4 beschriebenen Vorgehensweise *E*-3-[4-Dimethylaminomethyl-3-(3-methoxy-phenyl)-cyclohex-2-enylidenmethyl]-benzoesäure Hydrochlorid (15)

**Beispiel 6:**

*rac-trans-Z*-3-[4-Dimethylaminomethyl-3-(3-methoxy-phenyl)-cyclohexylidenmethyl]-benzoesäure; Hydrochlorid (17)

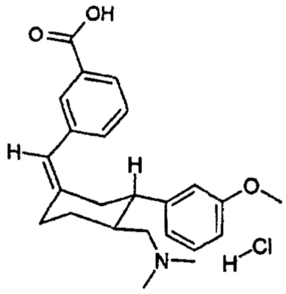

(17)

Unter Einsatz von:

*rac-trans-Z*-3-[4-Dimethylaminomethyl-3-(3-methoxy-phenyl)-cyclohexylidenmethyl]-benzoesäuremethylester Base, die nach Beispiel 2 hergestellt wurde,

anstelle von Z-3-[4-Dimethylaminomethyl-3-(3-methoxy-phenyl)-2-methyl-cyclohex-2-enylidenemethyl]-benzoesäuremethylester Base in Beispiel 4 erhielt man nach der in Beispiel 4 beschriebenen Vorgehensweise *rac-trans-Z*-3-[4-Dimethylaminomethyl-3-(3-methoxyphenyl)-cyclohexylidenmethyl]-benzoesäure; Hydrochlorid (17

**Beispiel 7:**

*rac-trans-E*-3-[4-Dimethylaminomethyl-3-(3-methoxy-phenyl)-cyclohexylidenmethyl]-benzoesäure Hydrochlorid (18)

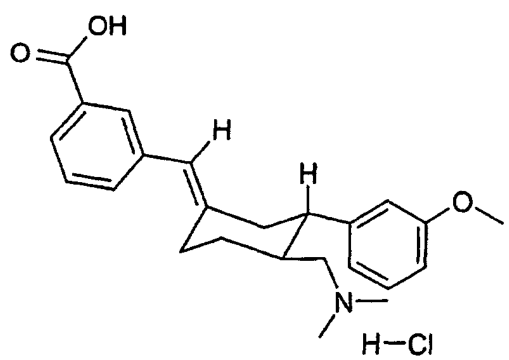

(18)

Unter Einsatz von

*rac-trans*-E-3-[4-Dimethylaminomethyl-3-(3-methoxy-phenyl)-cyclohexylidenmethyl]-benzoesäuremethylester

Base, die nach Beispiel 2 hergestellt wurde,

anstelle von *Z*-3-[4-Dimethylaminomethyl-3-(3-methoxy-phenyl)-2-methyl-cyclohex-2-enylidenemethyl]-benzoesäuremethylester Base in Beispiel 4 erhielt man nach der in Beispiel 4 beschriebenen Vorgehensweise *rac-trans-E*-3-[4-Dimethylaminomethyl-3-(3-methoxyphenyl)-cyclohexylidenmethyl]-benzoesäure Hydrochlorid (18)

**Beispiel 8:**

*rac-cis-Z*-3-[4-Dimethylaminomethyl-3-(3-methoxy-phenyl)-cyclohexylidenmethyl]-benzoesäure Hydrochlorid (27)

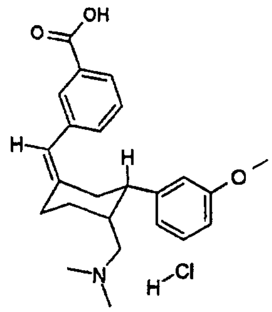

(27)

Unter Einsatz von

*rac-cis-Z*-3-[4-Dimethylaminomethyl-3-(3-methoxy-phenyl)-cyclohexylidenmethyl]-benzoesäuremethylester Base, die nach Beispiel 3 hergestellt wurde,

anstelle von *Z*-3-[4-Dimethylaminomethyl-3-(3-methoxy-phenyl)-2-methyl-cyclohex-2-enylidenemethyl]-benzoesäuremethylester Base in Beispiel 4 erhielt man nach der in Beispiel 4 beschriebenen Vorgehensweise *rac-cis-Z*-3-[4-Dimethylaminomethyl-3-(3-methoxyphenyl)-cyclohexylidenmethyl]-benzoesäure Hydrochlorid (27)

**Beispiel 9:**

*rac-cis-E*-4-Dimethylaminomethyl-3-(3-methoxy-phenyl)-cyclohexylidenmethyl]-benzoesäure Hydrochlorid (26)

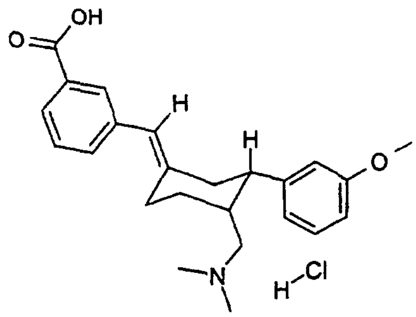

(26)

Unter Einsatz von

*rac-cis*-E-3-[4-Dimethylaminomethyl-3-(3-methoxy-phenyl)-cyclohexylidenmethyl]-benzoesäuremethylester Base, die nach Beispiel 3 hergestellt wurde,

anstelle von Z-3-[4-Dimethylaminomethyl-3-(3-methoxy-phenyl)-2-methyl-cyclohex-2-enylidenemethyl]-benzoesäuremethylester Base in Beispiel 4 erhielt man nach der in Beispiel 4 beschriebenen Vorgehensweise *rac-cis-E*-3-[4-Dimethylaminomethyl-3-(3-methoxyphenyl)-cyclohexylidenmethyl]-benzoesäure Hydrochlorid (26)

**Beispiel 10:**

Z-3-[4-Dimethylaminomethyl-3-(3-methoxy-phenyl)-cyclohex-2-enylidenmethyl]-N,N-diethyl-benzamid Hydrochlorid (34)

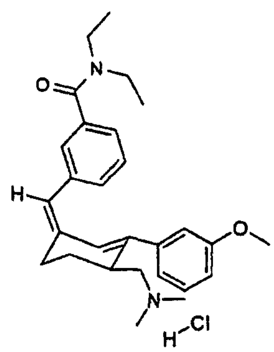

(34)

1.8 g der nach Beispiel 4 hergestellten Z-3-[4-Dimethylaminomethyl-3-(3-methoxy-phenyl)-cyclohex-2-enylidenmethyl]-benzoesäure Base wurden in 50 ml Dimethylformamid p.a. gelöst und bei 0°C bis 15°C mit 2 g DCC und 1.1 g Hydroxysuccinimid versetzt. Es wurde eine Stunde bei 0°C gerührt und dann bei dieser Temperatur 2 ml Diethylamin zugetropft. Es wurde noch eine Stunde bei 0°C und dann vier Tage bei Raumtemperatur gerührt. Das Reaktionsgemisch wurde auf 300 ml gesättigte Natriumchlorid-Lösung gegossen und anschließend 3 Mal mit je 250 ml Essigsäureethylester extrahiert. Die organischen Phasen wurden mit 50 ml gesättigter Natriumchlorid-Lösung gewaschen und anschließend über Magnesiumsulfat getrocknet. Das Lösemittel wurde im Vakuum entfernt. Der Rückstand wurde an Kieselgel mit Essigsäureethylester / Methanol = 6/5 chromatographiert. Man erhielt so 0.87 g Z-3-[4-Dimethylaminomethyl-3-(3-methoxy-phenyl)-cyclohex-2-enylidenmethyl]-N,N-diethyl-benzamid Base in Form eines orange-gelben Öles. Zur Freisetzung des Hydrochlorids wurde das Öl in 5 ml Aceton gelöst und mit einer äquimolaren Menge Trimethylchlorsilan und Wasser versetzt. Man erhielt so 0.52 g (22.1 % d. Theorie) der Titelverbindung 34 in Form weißer Kristalle.

**Beispiel 11:**

*E*-3-[4-Dimethylaminomethyl-3-(3-methoxy-phenyl)-cyclohex-2-enylidenmethyl]-N,N-diethyl-benzamid Hydrochlorid (31)

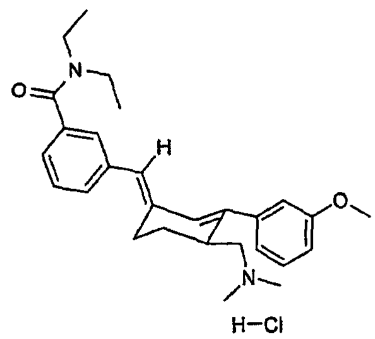

(31)

Unter Einsatz von:

*E*-3-[4-Dimethylaminomethyl-3-(3-methoxy-phenyl)-cyclohex-2-enylidenmethyl]-benzoesäure als Base, die gemäß Beispiel 5 hergestellt wurde,

anstelle von Z-3-[4-Dimethylaminomethyl-3-(3-methoxy-phenyl)-cyclohex-2-enylidenmethyl]-benzoesäure in Beispiel 10 erhielt man nach der in Beispiel 10 beschriebenen Vorgehensweise *E*-3-[4-Dimethylaminomethyl-3-(3-methoxy-phenyl)-cyclohex-2-enylidenmethyl]-N,N-diethyl-benzamid Hydrochlorid (31)

**Beispiel 12:**

*rac-trans-Z*-3-[4-Dimethylaminomethyl-3-(3-methoxy-phenyl)-cyclohexylidenmethyl]-N,N-diethyl-benzamid Hydrochlorid (36)

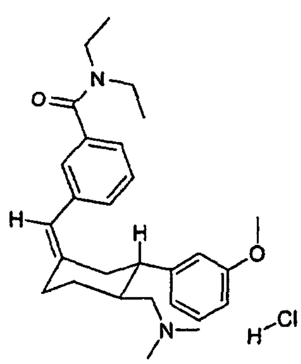

(36)

Unter Einsatz von:

*rac-trans*-Z-3-[4-Dimethylaminomethyl-3-(3-methoxy-phenyl)-cyclohexylidenmethyl]-benzoesäure als Base, die gemäß Beispiel 6 hergestellt wurde,

anstelle von Z-3-[4-Dimethylaminomethyl-3-(3-methoxy-phenyl)-cyclohex-2-enylidenmethyl]-benzoesäure in Beispiel 10 erhielt man nach der in Beispiel 10 beschriebenen Vorgehensweise *rac-trans-Z*-3-[4-Dimethylaminomethyl-3-(3-methoxy-phenyl)-cyclohexylidenmethyl]-N,N-diethyl-benzamid Hydrochlorid (36).

**Beispiel 13:**

*rac-trans-E*-3-[4-Dimethylaminomethyl-3-(3-methoxy-phenyl)-cyclohex-2-enylidenmethyl]-N,N-diethyl-benzamid Hydrochlorid (32)

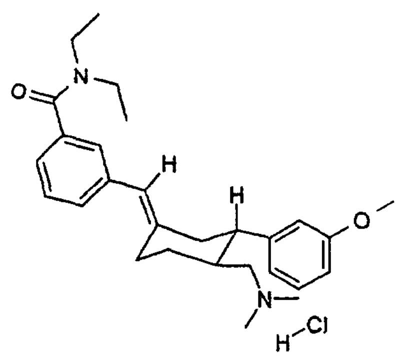

(32)

Unter Einsatz von:

*rac-trans-E*-3-[4-Dimethylaminomethyl-3-(3-methoxy-phenyl)-cyclohexylidenmethyl]-benzoesäure als Base, die gemäß Beispiel 7 hergestellt wurde,

anstelle von Z-3-[4-Dimethylaminomethyl-3-(3-methoxy-phenyl)-cyclohex-2-enylidenmethyl]-benzoesäure in Beispiel 10 erhielt man nach der in Beispiel 10 beschriebenen Vorgehensweise *rac-trans-E-3*-[4-Dimethylaminomethyl-3-(3-methoxy-phenyl)-cyclohexylidenmethyl]-N,N-diethyl-benzamid Hydrochlorid (32).

**Beispiel 14:**

*rac-cis-E*-3-[4-Dimethylaminomethyl-3-(3-methoxy-phenyl)-cyclohexylidenmethyl]-N,N-diethyl-benzamid; Hydrochlorid (35)

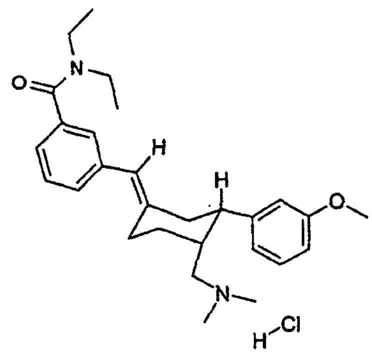

(35)

Unter Einsatz von:

*rac-cis-E*-3-[4-Dimethylaminomethyl-3-(3-methoxy-phenyl)-cyclohexylidenmethyl]-benzoesäure als Base, die gemäß Beispiel 9 hergestellt wurde,
anstelle von Z-3-[4-Dimethylaminomethyl-3-(3-methoxy-phenyl)-cyclohex-2-enylidenmethyl]-benzoesäure in Beispiel 10 erhielt man nach der in Beispiel 10 beschriebenen Vorgehensweise *rac-cis-E*-3-[4-Dimethylaminomethyl-3-(3-methoxy-phenyl)-cyclohexylidenmethyl]-N,N-diethyl-benzamid Hydrochlorid (35).

**Beispiel 15**

*Z*-{3-[4-Dimethylaminomethyl-3-(3-methoxy-phenyl)-cyclohex-2-enylidenmethyl]-phenyl}-methanol; Hydrochlorid (25)

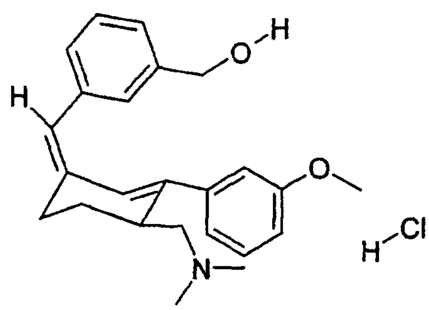

(25)

Unter Stickstoffatmosphäre wurden 50 ml Diisobutylaluminiumhydrid-Lösung (25 Gew.% Lsg in Toluol) vorgelegt. Anschließend wurden bei Raumtemperatur 1 g Z-3-[4-Dimethylaminomethyl-3-(3-methoxyphenyl)-2-methyl-cyclohex-2-enylidenmethyl]-benzoesäuremethylester Base, die gemäß Beispiel 1 hergestellt wurde, gelöst in 100 ml

Toluol p.a. zugetropft. Es wurde zwei Stunden unter Rückfluß erhitzt. Nach Beendigung der Reaktion wurde das Gemisch auf 0°C abgekühlt und mit einem Gemisch aus 25 ml Ethanol und 20 ml Wasser gequenscht. Der ausgefallene Niederschlag wurde abgesaugt und mit Essigester gewaschen. Die organische Phase wurde über Magnesiumsulfat getrocknet und anschließend im Vakuum vom Lösemittel befreit. Der Rückstand wurde säulenchromatographisch an Kieselgel mit Essigsäureethylester/Methanol = 9/1 gereinigt. Man erhielt so 580 mg der Titelverbindung als Base. Zur Freisetzung des Hydrochlorids wurde die Base in 20 ml Aceton gelöst und mit einer äquimolaren Menge Trimethylchlorsilan und Wasser versetzt. So erhielt man 550 mg (52.9% d. Th.) Z-(3-[4-Dimethylaminomethyl-3-(3-methoxyphenyl)-cyclohex-2-enylidenmethyl]-phenyl)-methanol; Hydrochlorid (25) in Form weißer Kristalle.

**Beispiel 16**

*E*-{3-[4-Dimethylaminomethyl-3-(3-methoxy-phenyl)-cyclohex-2-enylidenmethyl]-phenyl}-methanol; Hydrochlorid (16)

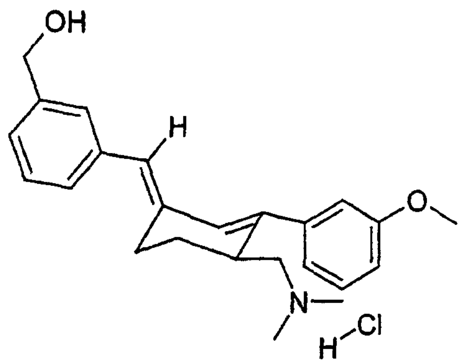

(16)

Unter Einsatz von:

*E*-3-[4-Dimethylaminomethyl-3-(3-methoxy-phenyl)-2-methyl-cyclohex-2-enylidenmethyl]-benzoesäuremethylester Base, die gemäß Beispiel 1 hergestellt wurde,

anstelle von Z-3-[4-Dimethylaminomethyl-3-(3-methoxy-phenyl)-2-methyl-cyclohex-2-enylidenemethyl]-benzoesäuremethylester Base in Beispiel 15 erhielt man nach der in Beispiel 15 beschriebenen Vorgehensweise *E*-{3-[4-Dimethylaminomethyl-3-(3-methoxy-phenyl)-cyclohex-2-enylidenmethyl]-phenyl}-methanol; Hydrochlorid (16)

**Beispiel 17**

Z-3-[6-Dimethylaminomethyl-3-(3-hydroxymethyl-benzyliden)-cyclohex-1-enyl]-phenol; Hydrochlorid (40) und *Z*-3-[2-Dimethylaminomethyl-5-(3-hydroxymethyl-benzyliden)-cyclohex-1 -enyl]-phenol; Hydrochlorid (24)

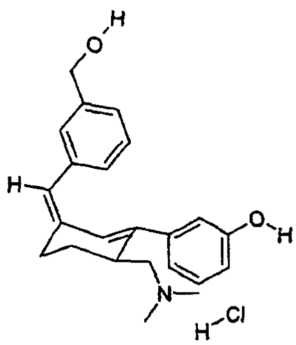

(40)

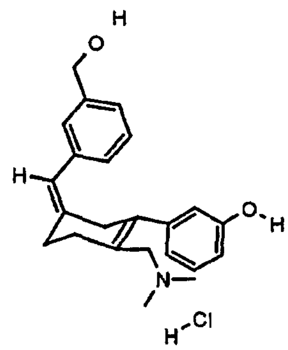

(24)

Unter Stickstoffatmosphäre wurden 100 ml Diisobutylaluminiumhydrid-Lösung (25 Gew.% Lsg in Toluol) vorgelegt. Anschließend wurden bei Raumtemperatur 1 g Z-3-[4-Dimethylaminomethyl-3-(3-methoxyphenyl)-2-methyl-cyclohex-2-enylidenemethyl]-benzoesäuremethylester Base, die gemäß Beispiel 1 hergestellt wurde, gelöst in 100 ml Toluol p.a. zugetropft. Es wurde sechs Stunden unter Rückfluß erhitzt. Nach Beendigung der Reaktion wurde das Gemisch auf 0°C abgekühlt und mit einem Gemisch aus 25 ml Ethanol und 20 ml Wasser gequenscht. Der ausgefallene Niederschlag wurde abgesaugt und mit Essigester gewaschen. Die organische Phase wurde über Magnesiumsulfat getrocknet und anschließend im Vakuum vom Lösemittel befreit. Der Rückstand wurde säulenchromatographisch an Kieselgel mit Essigsäureethylester/Methanol = 9/1 gereinigt. Man erhielt als erste Produktfraktion 200 mg Z-3-[2-Dimethylaminomethyl-5-(3-hydroxymethyl-benzyliden)-cyclohex-1-enyl]-phenol Base. Zur Freisetzung des Hydrochlorids wurde die Base in 20 ml Aceton gelöst und mit einer äquimolaren Menge Trimethylchlorsilan und Wasser versetzt. So erhielt man 180 mg (17.9% d. Th.) Z-3-[2-Dimethylaminomethyl-5-(3-hydroxymethyl-benzyliden)-cyclohex-1-enyl]-phenol; Hydrochlorid (24) in Form weißer Kristalle. Als zweite Produktfraktion erhielt man 440 mg Z-3-[6-Dimethylaminomethyl-3-(3-hydroxymethyl-benzyliden)-cyclohex-1 -enyl]-phenol Base. Zur Freisetzung des Hydrochlorids wurde die Base in 20 ml Aceton gelöst und mit einer äquimolaren Menge Trimethylchlorsilan und Wasser versetzt und erhielt so 410 mg (40.9 % d. Th.) Z-3-[6-Dimethylaminomethyl-3-(3-hydroxymethyl-benzyliden)-cyclohex-1-enyl]-phenol; Hydrochlorid (40) in Form weißer Kristalle.

**Beispiel 18**

*E*-3-[6-Dimethylaminomethyl-3-(3-hydroxymethyl-benzyliden)-cyclohex-1-enyl]-phenol; Hydrochlorid (22)

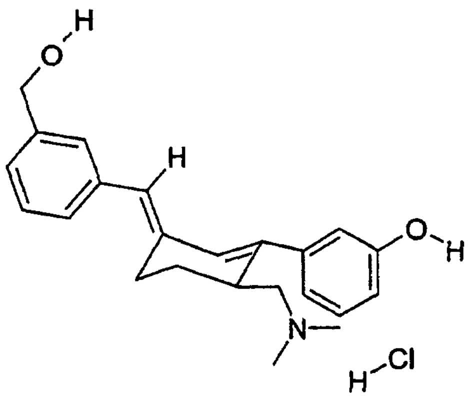

(22)

Unter Einsatz von:

*E*-3-[4-Dimethylaminomethyl-3-(3-methoxy-phenyl)-2-methyl-cyclohex-2-enylidenmethyl]-benzoesäuremethylester Base, die gemäß Beispiel 1 hergestellt wurde,

anstelle von Z-3-[4-Dimethylaminomethyl-3-(3-methoxy-phenyl)-2-methyl-cyclohex-2-enylidenemethyl]-benzoesäuremethylester Base in Beispiel 17 erhielt man nach der in Beispiel 17 beschriebenen Vorgehensweise ausschließlich *E*-3-[6-Dimethylaminomethyl-3-(3-hydroxymethyl-benzyliden)-cyclohex-1-enyl]-phenol; Hydrochlorid (22)

**Beispiel 19**

*rac-trans-E*-[3-(2-Dimethylaminomethyl-5-(3-hydroxymethyl-benzyliden)-cyclohexyl)-phenol, Hydrochlorid (19)

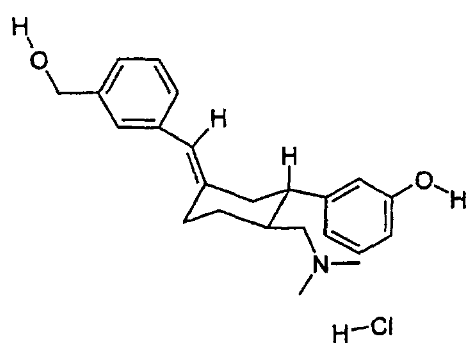

(19)

Unter Einsatz von:

*rac-trans-E*-3-[4-Dimethylaminomethyl-3-(3-methoxy-phenyl)-cyclohexylidenemethyl] Base, die gemäß Beispiel 2 hergestellt wurde,

anstelle von Z-3-[4-Dimethylaminomethyl-3-(3-methoxy-phenyl)-2-methyl-cyclohex-2-enylidenemethyl]-benzoesäuremethylester Base in Beispiel 17 erhielt man nach der in Beispiel 17 beschriebenen Vorgehensweise ausschließlich *rac-trans-E*-[3-(2-Dimethylaminomethyl-5-(3-hydroxymethyl-benzyliden)-cyclohexyl)-phenol Hydrochlorid (19)

**<u>Beispiel 20</u>**

*Z*-3-[4-Dimethylaminomethyl-3-(3-methoxy-phenyl)-2-methyl-cyclohex-2-enylidenmethyl]-naphthalen-1-carbonsäureethylester; Hydrochlorid (11) und *E*-3-[4-Dimethylaminomethyl-3-(3-methoxy-phenyl)-2-methylcyclohex-2-enylidenmethyl]-naphthalen-1-carbonsäureethylester Hydrochlorid (12)

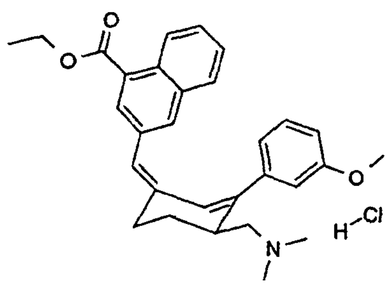

(11)

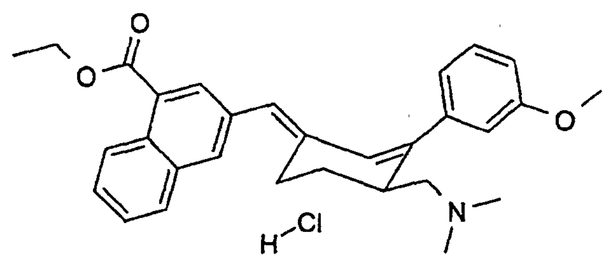

(12)

Unter Einsatz von:

3-(Naphtyl-1-carbonsäureethylester)-methyltriphenylphosphoniumchlorid

anstelle von 3-(Benzoesäuremethylester)-methyltriphenylphosphoniumchlorid in Beispiel 1 erhielt man nach der in Beispiel 1 beschriebenen Vorgehensweise *Z*-3-[4-Dimethylaminomethyl-3-(3-methoxy-phenyl)-2-methyl-cyclohex-2-enylidenmethyl]-naphthaten-1-carbonsäureethylester; Hydrochlorid (11) und *E*-3-[4-Dimethylaminomethyl-3-(3-methoxy-phenyl)-2-methylcyclohex-2-enylidenmethyl]-naphthalen-1-carbonsäureethylester Hydrochlorid (12)

**Beispiel 21**

*Z*-3-[4-Dimethylaminomethyl-3-(3-methoxy-phenyl)-cyclohexylidenmethyl]-2-fluor-benzoesäureethylester Hydrochlorid (13)

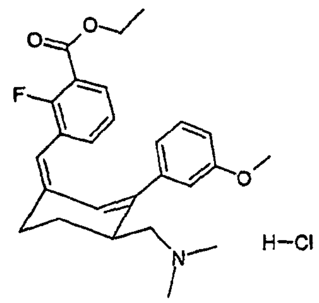

(13)

Unter Einsatz von:

(2-Fluor-3-benzoesäureethylester)-methyl-triphenylphosphoniumchlorid

anstelle von 3-(Benzoesäuremethylester)-methyltriphenylphosphoniumchlorid in Beispiel 1 erhielt man nach der in Beispiel 1 beschriebenen Vorgehensweise ausschließlich *Z*-3-[4-Dimethylaminomethyl-3-(3-methoxy-phenyl)-cyclohexylidenmethyl]-2-fluor-benzoesäureethylester Hydrochlorid (13)

**Beispiel 22**

*E*-3-[4-Dimethylaminomethyl-3-(3-methoxy-phenyl)-cyclohex-2-enylidenmethyl]-benzoesäure-tert.-butylester; Hydrochlorid (21)

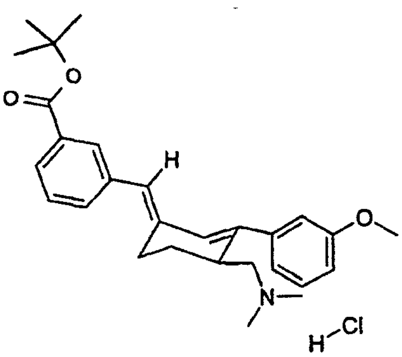

(21)

Unter Einsatz von:

(3-Benzoesäure-tert.butylester)-methyl-triphenylphosphoniumchlorid)

anstelle von 3-(Benzoesäuremethylester)-methyltriphenylphosphoniumchlorid in Beispiel 1 erhielt man nach der in Beispiel 1 beschriebenen Vorgehensweise ausschließlich *E*-3-[4-Dimethylaminomethyl-3-(3-methoxy-phenyl)-cyclohex-2-enylidenmethyl]-benzoesäure-tert.-butytester; Hydrochlorid (21)

**Beispiel 23**

*E*-3-[4-Dimethylaminomethyl-3-(3-methoxy-phenyl)-cyclohex-2-enylidenmethyl]-benzoesäureethylester; Hydrochlorid (33)

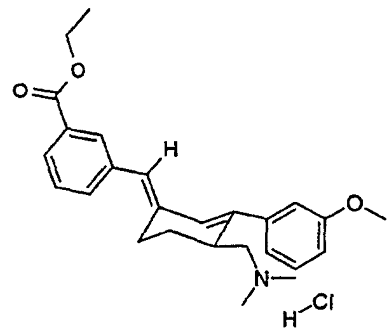

(33)

Unter Einsatz von:

(3-Benzoesäure-ethylester)-methyltriphenylphosphoniumchlorid

anstelle von 3-(Benzoesäuremethylester)-methyltriphenylphosphoniumchlorid in Beispiel 1 erhielt man nach der in Beispiel 1 beschriebenen Vorgehensweise ausschließlich *E*-3-[4-Dimethylaminomethyl-3-(3-methoxy-phenyl)-cyclohex-2-enylidenmethyl]-benzoesäureethylester; Hydrochlorid (33)

**Beispiel 24**

*Z*-3-[4-Dimethylaminomethyl-3-(3-methoxy-phenyl)-cyclohex-2-enylidenmethyl]-benzoesäureisobutylester; Hydrochlorid (39)

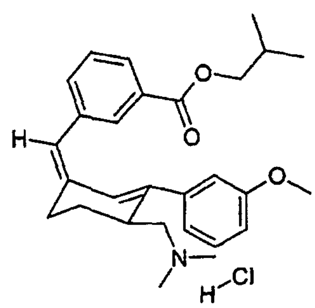

(39)

Unter Einsatz von:

(3-Benzoesäure-isobutylester)-methyl-triphenylphosphoniumchlorid

anstelle von 3-(Benzoesäuremethylester)-methyltriphenylphosphoniumchlorid in Beispiel 1 erhielt man nach der in Beispiel 1 beschriebenen Vorgehensweise ausschließlich Z-3-[4-Dimethylaminomethyl-3-(3-methoxy-phenyl)-cyclohex-2-enylidenmethyl]-benzoesäureisobutylester; Hydrochlorid (39)

**Beispiel 25:**

*rac-trans-E*-3-[4-Dimethylaminomethyl-3-(3-methoxy-phenyl)-cyclohexylidenmethyl]-benzoesäuretert-butylester; Hydrochlorid (28)

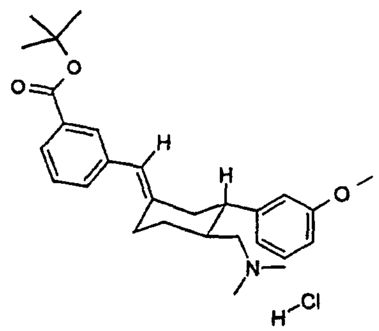

(28)

Unter Einsatz von:

(3-Benzoesäure-tert.butylester)-methyl-triphenylphosphoniumchlorid
anstelle von 3-(Benzoesäuremethylester)-methyltriphenylphosphoniumchlorid in Beispiel 2 erhielt man nach der in Beispiel 2 beschriebenen Vorgehensweise ausschließlich *rac-trans-E*-3-[4-Dimethylaminomethyl-3-(3-methoxy-phenyl)-cyclohexylidenmethyl]-benzoesäuretert-butylester; Hydrochlorid (28)

**Beispiel 26:**

*rac-cis-Z*-3-[4-Dimethylaminomethyl-3-(3-methoxy-phenyl)-cyclohexylidenmethyl]-benzoesäureethylester; Hydrochlorid (30)

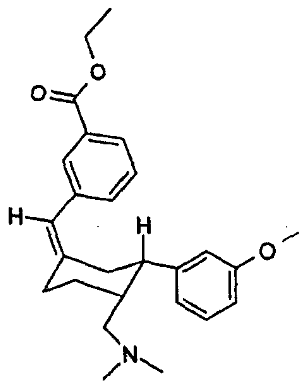

(30)

Unter Einsatz von:

(3-Benzoesäure-ethylester)-methyl-triphenylphosphoniumchlorid

anstelle von 3-(Benzoesäuremethylester)-methyltriphenylphosphoniumchlorid in Beispiel 3 erhielt man nach der in Beispiel 3 beschriebenen Vorgehensweise ausschließlich rac-cis-Z-3-[4-Dimethylaminomethyl-3-(3-methoxy-phenyl)-cyclohexylidenmethyl]-benzoesäureethylester; Hydrochlorid (30)

**Beispiel 27**

*Z*-[4-(4-Chloro-benzyliden)-2-(3-methoxy-phenyl)-cyclohex-2-enylmethyl]-dimethyl-amin; Hydrochlorid (41) und E-[4-(4-Chlorbenzyliden)-2-(3-methoxy-phenyl)-cyclohex-2-enylmethyl]-dimethylamin; Hydrochlorid (46)

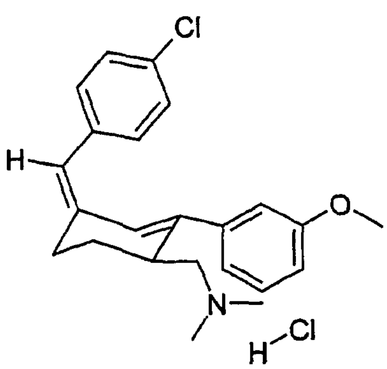

(41)

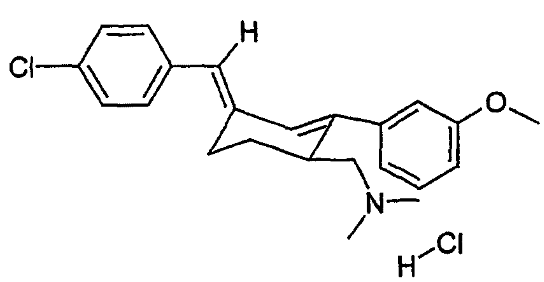

(46)

21.6 g Kalium-tert.butylat und 65 g 4-Chlorbenzyltriphenylphosphoniumchlorid wurden unter Stickstofatmosphäre bei Raumtemperatur in 800 ml Toluol p.a. suspendiert und anschließend eine Stunde bei 70 °C gerührt. Bei dieser Temperatur wurden 11.5 g 4-Dimethylaminomethyl-3-(3-methoxyphenyl)-cyclo-hex-2-enon in 100 ml Toluol p. a. zugegeben und 3 Tage bei 70 °C gerührt. Das Gemisch wurde mit 500 ml Wasser gequenscht. Die Phasen wurden getrennt und die wässrige Phase wurde 3 Mal mit je 200 ml Essigsäureethylester gewaschen. Die vereinten organischen Phasen wurden über Magnesiumsulfat getrocknet und anschließend im Vakuum vom Lösemittel befreit. Der ausgefallene Feststoff wurde abfiltriert und mit Diisoether gewaschen. Die vereinten organischen Phasen wurden im Vakuum vom Lösemittel befreit. Der Rückstand wurde säulenchromatographisch an Kieselgel mit Essigsäureethylester/Methanol = 9/1 gereinigt. Als erste Produktfraktion erhielt man 5.2 g *E*-[4-(4-Chlor-benzyliden)-2-(3-methoxy-phenyl)-cyclohex-2-enylmethyl]-dimethyl-amin Base. Zur Darstellung des Hydrochlorids wurde die Base in 50 ml Aceton gelöst und mit einer äquimolaren Menge Trimethylchlorsilan und Wasser versetzt. Man erhielt so 4.7 g (27.6 % d. Th.) der Titelverbindung 46 in Form weißer Kristalle. Als zweite Produktfraktion erhielt man 3.5 g Z-[4-(4-Chlorobenzyliden)-2-(3-methoxy-phenyl)-cyclohex-2-enylmethyl]-dimethyl-amin Base. Zur Freisetzung des Hydrochlorids wurde die Base in 50 ml Aceton gelöst und mit einer äquimolaren Menge Wasser und Trimethylchlorsilan versetzt. Man erhielt so 3 g (17.6 % d. Th.) der Titelverbindung 41 in Form weißer Kristalle.

**Beispiel 28**

*E*-3-[3-(4-Chlor-benzyliden)-6-dimethylaminomethyl-cyclohex-1-enyl]-phenol; Hydrochlorid (47)

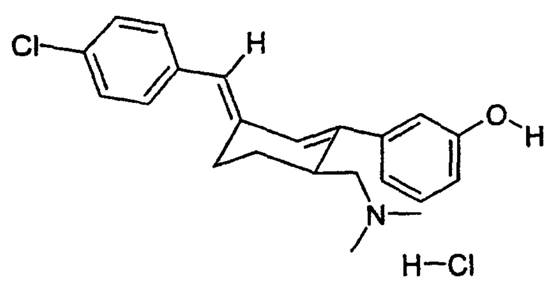

**(47)**

Zu 50 ml Diisobutylaluminiumhydrid-Lösung (25 Gew.% Lsg in Toluol) wurden 850 mg *E*-[4-(4-Chlor-benzyliden)-2-(3-methoxy-phenyl)-cyclohex-2-enylmethyl]-dimethyl-amin Base, die gemäß Beispiel 27 dargestellt wurde, gelöst in 50 ml Toluol p.a. zugegeben. Es wurde 8 Stunden unter Rückfluß erhitzt. Anschließend wurde die Reaktionslösung mit 100 ml Ethanol, dann mit 100 ml Wasser gequenscht. Der ausgefallene Niederschlag wurde agbesaugt und mit Toluol gewaschen. Die vereinten organischen Phasen wurden über Magnesiumsulfat getrocknet und anschließend das Lösemittel im Vakuum evaporiert. Der Rückstand wurde an Kieselgel mit Essigsäureethylester/ Methanol = 9/1 gereinigt. Man erhielt so 500 mg der Titelverbindung 47 als Base. Zur Freisetzung des Hydrochlorids wurde die Titelverbindung in 50 ml Aceton gelöst und mit einer äquimolaren Menge Trimethylchlorsilan und Wasser versetzt. So erhielt man 430 mg (52.5 % d. Th.) *E*-3-[3-(4-Chlor-benzyliden)-6-dimethylaminomethyl-cyclohex-1-enyl]-phenol; Hydrochlorid (47) in Form weißer Kristalle.

**Beispiel 29**

Z-[4-(4-Fluoro-benzyliden)-2-(3-methoxy-phenyl)-cyclohex-2-enylmethyl]-dimethyl-amin; Hydrochlorid (43) und *E*-[4-(4-Fluorobenzyliden)-2-(3-methoxy-phenyl)-cyclohex-2-enylmethyl)-dimethylamin; Hydrochlorid (42)

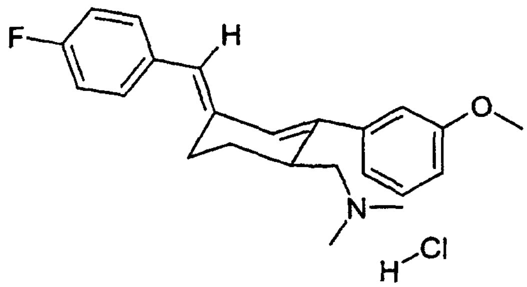

(42)

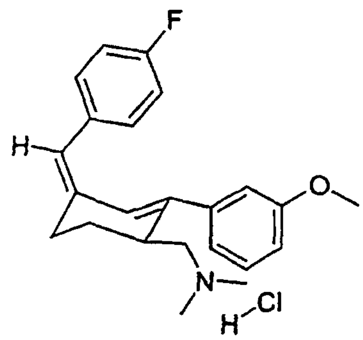

(43)

21.6 g Kalium-tert.butylat und 70 g 4-Fluorbenzyltriphenylphosphoniumchlorid wurden unter Stickstofatmosphäre bei Raumtemperatur in 800 ml Toluol p.a. suspendiert und anschließend eine Stunde bei 70 °C gerührt. Bei dieser Temperatur wurden 11.5 g 4-Dimethylaminomethyl-3-(3-methoxyphenyl)-cyclo-hex-2-enon in 100 ml Toluol p. a. zugegeben und 3 Tage bei 70 °C gerührt. Das Gemisch wurde mit 500 ml Wasser gequenscht. Die Phasen wurden getrennt und die wässrige Phase wurde 3 Mal mit je 200 ml Essigsäureethylester gewaschen. Die vereinten organischen Phasen wurden über Magnesiumsulfat getrocknet und anschließend im Vakuum vom Lösemittel befreit. Der ausgefallene Feststoff wurde abfiltriert und mit Diisoether gewaschen. Die vereinten organischen Phasen wurden im Vakuum vom Lösemittel befreit. Der Rückstand wurde säulenchromatographisch an Kieselgel mit Essigsäureethylester/Methanol = 9/1 gereinigt. Als erste Produktfraktion erhielt man 5.4 g *E-[*4-(4-Fluoro-benzyliden)-2-(3-methoxy-phenyl)-cyclohex-2-enylmethyl]-dimethyl-amid. Zur Darstellung des Hydrochlorids wurde die Base in 50 ml Aceton gelöst und mit einer äquimolaren Menge Trimethylchlorsilan und Wasser versetzt. Man erhielt so 5.1 g (30 % d. Th.) der Titelverbindung 42 in Form weißer Kristalle. Als zweite Produktfraktion erhielt man 4.2 g Z-[4-(4-Fluorobenzyliden)-2-(3-methoxy-phenyl)-cyclohex-2-enylmethyl]-dimethylamin. Zur Freisetzung des Hydrochlorids wurde die Base in 50 ml Aceton gelöst und mit einer äquimolaren Menge Wasser und Trimethylchlorsilan versetzt. Man erhielt so 3.9 g (22.9 % d. Th.) der Titelverbindung 43 in Form weißer Kristalle.

**Beispiel 30**

Z-3-[6-Dimethylaminomethyl-3-(4-fluor-benzyliden)-cyclohex-1-enyl]-phenol; Hydrochlorid (45)

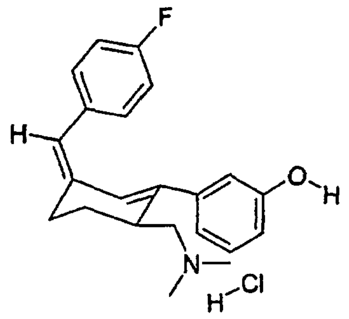

(45)

Unter Einsatz von:

Z-[4-(4-Fluoro-benzyliden)-2-(3-methoxy-phenyl)-cyclohex-2-enylmethyl]-dimethyl-amin Base

anstelle von *E*-[4-(4-Chlor-benzyliden)-2-(3-methoxy-phenyl)-cyclohex-2-enylmethyl]-dimethyl-amin Base in Beispiel 28 erhielt man nach der in Beispiel 28 beschriebenen Vorgehensweise *Z*-3-[6-Dimethylaminomethyl-3-(4-fluor-benzyliden)-cyclohex-1-enyl]-phenol; Hydrochlorid (45).

**Beispiel 31**

*E*-3-[6-Dimethylaminomethyl-3-(4-fluor-benzyliden)-cyclohex-1-enyl]-phenol; Hydrochlorid (44)

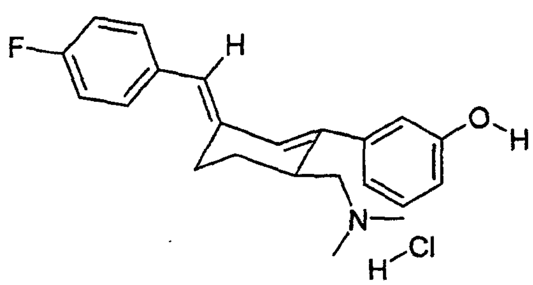

(44)

Unter Einsatz von:

*E*-[4-(4-Fluoro-benzyliden)-2-(3-methoxy-phenyl)-cyclohex-2-enylmethyl]-dimethyl-amin Base

anstelle von *E*-[4-(4-Chlor-benzyliden)-2-(3-methoxy-phenyl)-cyclohex-2-enylmethyl]-dimethyl-amin Base in Beispiel 28 erhielt man nach der in Beispiel 28 beschriebenen VorgehensweiseEZ-3-[6-Dimethylaminomethyl-3-(4-flu-

or-benzyliden)-cyclohex-1-enyl]-phenol; Hydrochlorid (44).

**Beispiel 32**

Z-3-[4-Dimethylaminomethyl-3-(3-hydroxy-phenyl)-cyclohex-2-enylidenmethyl]-benzoesäuremethylester Hydrochlorid (38) und E-3-[4-Dimethylaminomethyl-3-(3-hydroxy-phenyl)-cyclohex-2-enylidenmethyl]-benzoesäuremethylester Hydrochlorid (37)

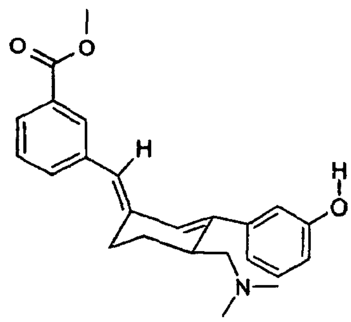

(37)

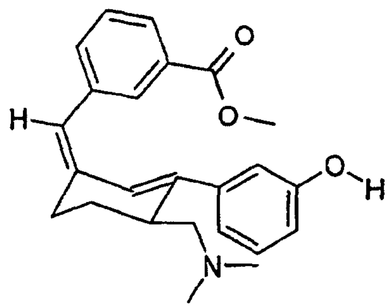

(38)

8.1 g Kalium-tert.butylat und 35.4 g 3-(Benzoesäuremethylester)-methyltriphenylphosphoniumchlorid wurden unter Stickstoffatmosphäre bei Raumtemperatur in 800 ml Toluol p.a. suspendiert und anschließend eine Stunde bei 70 °C gerührt. Bei dieser Temperatur wurden 7 g 3-(3-(tert.-Butyl-diphenyl-silanyloxy)-cyclohex-2-enon in 100 ml Toluol p.a. zugegeben und 3 Tage bei 70 °C gerührt. Das Gemisch wurde mit 500 ml Wasser gequenscht. Die Phasen wurden getrennt und die wässrige Phase wurde 3 Mal mit je 200 ml Essigsäureethylester gewaschen. Die vereinten organischen Phasen wurden über Magnesiumsulfat getrocknet und anschließend im Vakuum von Lösungsmittel befreit. Der so erhaltene Rückstand wurde in einem Gemisch aus 150 ml Essigsäureethylester und 150 ml Diisoether aufgenommen. Der ausgefallene Feststoff wurde abfiltriert und mit Diisoether gewaschen. Die vereinten organischen Phasen wurden im Vakuum vom Lösemittel befreit. Der Rückstand (16.7 g) wurde in 70 ml Tetrahydrofuran gelöst und mit 16.7 ml Tetrabutylammoniumfluorid versetzt. Nach 10 Minuten Reaktionszeit wurde die Reaktionslösung mit 50 ml Wasser gequenscht und drei Mal mit je 50 ml Essigsäureethylester extrahiert. Die vereinten organischen Phasen wurden über Magnesiumsulfat getrocknet und anschließend im Vakuum vom Lösemittel befreit. Der Rückstand wurde säulenchromatographisch an Kieselgel mit Essigsäureethylester/Methanol = 9/1 gereinigt. Als erste Produktfraktion erhielt man 2.5 g *E*-3-[4-Dimethylaminomethyl-3-(3-hydroxy-phenyl)-cyclohex-2-enylidenmethyl]-benzoesäuremethylester Base. Zur Freisetzung des Hydrochlorids wurde die Base in 50 ml Aceton gelöst und mit einer äquimolaren Menge Trimethylchlorsilan und Wasser versetzt. So erhielt man 2 g (36.8 % d. Th.) *E*-3-[4-Dimethylaminomethyl-3-(3-hydroxyphenyl)-cyclohex-2-enylidenmethyl]-benzoesäuremethylester Hydrochlorid (37). Als zweite Produktfraktion erhielt man 1.8 g (27.6 % d. Th.) *Z*-3-[4-Dimethylaminomethyl-3-(3-hydroxy-phenyl)-cyclohex-2-enylidenemethyl]-benzoesäuremethylester. Zur Freisetzung des Hydrochlorids wurde die Base in 50 ml Aceton gelöst und mit einer äquimolaren Menge Trimethylchlorsilan und Wasser versetzt. So erhielt man 1.5 g (27.6 % d. Th.) Z-3-[4-Dimethylaminomethyl-3-(3-hydroxyphenyl)-cyclohex-2-enylidenmethyl]-benzoesäuremethylester Hydrochlorid (38).

**Beispiel 33**

*rac-cis-Z*-3-[4-Dimethylaminomethyl-3-(3-hydroxy-phenyl)-cyclohexylidenmethyl]-benzoesäuremethylester; Hydrochlorid (51) und *rac-cis-E* 3-[4-Dimethylaminomethyl-3-(3-hydroxy-phenyl)-cyclohexylidenmethyl]-benzoesäuremethylester; Hydrochlorid (50)

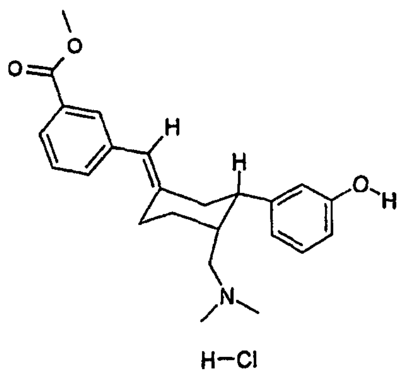

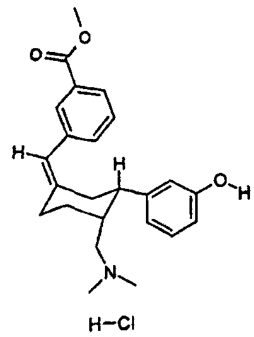

(50) (51)

Unter Einsatz von

*rac-cis*-3-(3-(tert-Butyl-diphenyl-silanyloxy)-phenyl)-cyclohexanon

anstelle von 3-(3-(tert.-Butyl-diphenyl-silanyloxy)-cyclohex-2-enon in Beispiel 32 erhielt man nach der in Beispiel 32 beschriebenen Vorgehensweise *rac-cis-Z*-3-[4-Dimethylaminomethyl-3-(3-hydroxyphenyl)-cyclohexylidenmethyl]-benzoesäuremethylester; Hydrochlorid (51) und *rac-cis-E*-3-[4-Dimethylaminomethyl-3-(3-hydroxy-phenyl)-cyclohexylidenmethyl]-benzoesäuremethylester; Hydrochlorid (50)

**Beispiel 34**

Z-3-[4-Dimethylaminomethyl-3-(3-hydroxy-phenyl)-cyclohex-2-enylidenmethyl]-benzoesäure Hydrochlorid (48)

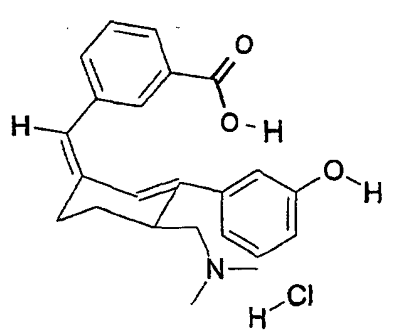

(48)

3 g des nach Beispiel 32 hergestellten Z-3-[4-Dimethylaminomethyl-3-(3-hydroxy-phenyl)-cyclohex-2-enylidenemethyl]-benzoesäuremethylester als Base wurden in 30 ml Methanol gelöst und mit 30 ml 1 n Kaliumhydroxid-Lösung versetzt. Es wurde 2 Stunden bei 60 °C gerührt. Nach Abkühlen der Reaktionsmischung auf Raumtemperatur wurde das Gemisch mit 1 n Salzsäure solange versetzt bis ein pH-Wert von 4 eingestellt wurde. Die Phasen wurden getrennt und die wäßrige Phase wurde 3 Mal mit je 20 ml Essigsäureethylester gewaschen. Die vereinten organischen Phasen wurden über Magnesiumsulfat getrocknet und im Vakuum vom Lösemittel befreit. Man erhielt so Z-3-[4-Dimethylaminomethyl-3-(3-hydroxy-phenyl)-cyclohex-2-enylidenmethyl]-benzoesäure 2.7 g in Form eines orange-gelben Öles. Zur Darstellung des Hydrochlorids wurde die Base in 10 ml Aceton gelöst und mit einer äquimolaren Menge Trimethylchlorsilan und Wasser versetzt. So erhielt man 2.4 g ( 77.7 % d. Th.) Z-3-[4-Dimethylaminomethyl-3-(3-hydroxy-phenyl)-cyclohex-2-enylidenmethyl]-benzoesäure Hydrochlorid.

**Beispiel 35**

*E*-3-[4-Dimethylaminomethyl-3-(3-hydroxy-phenyl)-cyclohex-2-enylidenmethyl]-benzoesäure; Hydrochlorid (49)

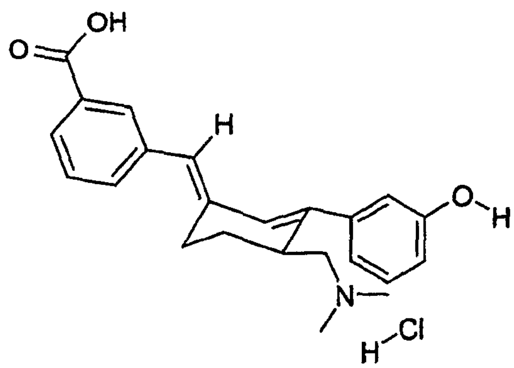

(49)

Unter Einsatz von

*E*-3-[4-Dimethylaminomethyl-3-(3-hydroxy-phenyl)-cyclohex-2-enylidenemethyl]-benzoesäuremethylester als Base, die gemäß Beispiel 32 hergestellt wurde,

anstelle von Z-3-[4-Dimethylaminomethyl-3-(3-hydroxy-phenyl)-cyclohex-2-enylidenemethyl)-benzoesäuremethylester Base in Beispiel 34 erhielt man nach der in Beispiel 34 beschriebenen Vorgehensweise *E*-3-[4-Dimethylaminomethyl-3-(3-hydroxy-phenyl)-cyclohex-2-enylidenmethyl]-benzoesäure; Hydrochlorid (49)

**Beispiel 36**

*rac-trans*-Z-3-[4-Dimethylaminomethyl-3-(3-hydrox$_y$-phenyl)-cyclohexylidenmethyl]-benzoesäure; Hydrochlorid (52)

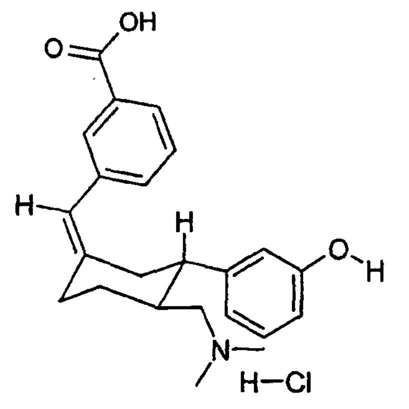

(52)

Unter Einsatz von

*rac-trans-Z*-3-[4-Dimethylaminomethyl-3-(3-hydroxy-phenyl)-cyclohex-2-enylidenemethyl]-benzoesäuremethylester als Base,
anstelle von Z-3-[4-Dimethylaminomethyl-3-(3-hydroxy-phenyl)-cyclohex-2-enylidenemethyl]-benzoesäuremethylester Base in Beispiel 34 erhielt man nach der in Beispiel 34 beschriebenen Vorgehensweise *rac-trans*-Z-3-[4-Dimethylaminomethyl-3-(3-hydroxy-phenyl)-cyclohexylidenmethyl]-benzoesäure; Hydrochlorid (52)

**Beispiel 37**

*rac-trans-E*--[4-Dimethylaminomethyl-3-(3-hydroxy-phenyl)-cyclohexylidenmethyl]-benzoesäure Hydrochlorid (53)

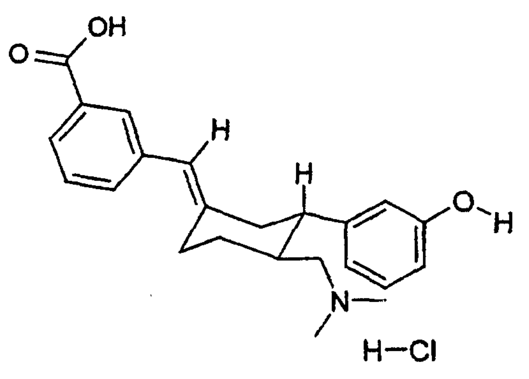

(53)

Unter Einsatz von

*rac-trans-E*-3-[4-Dimethylaminomethyl-3-(3-hydroxy-phenyl)-cyclohex-2-enylidenemethyl]-benzoesäuremethylester als Base,

anstelle von Z-3-[4-Dimethylaminomethyl-3-(3-hydroxy-phenyl)-cyclohex-2-enylidenemethyl]-benzoesäuremethylester Base in Beispiel 34 erhielt man nach der in Beispiel 34 beschriebenen Vorgehensweise *rac-trans-E*-3-[4-Dimethylaminomethyl-3-(3-hydroxy-phenyl)-cyclohexylidenmethyl]-benzoesäure; Hydrochlorid (53)

**<u>Beispiel 38</u>**

*rac-trans-Z*-[4-Benzyliden-2-(3-methoxy-phenyl)-cyclohexylmethyl]-dimethylamin Hydrochlorid (3) und *rac-trans-E*-[4-Benzyliden-2-(3-methoxy-phenyl)-cyclohexylmethyl]-dimethylamin Hydrochlorid (2)

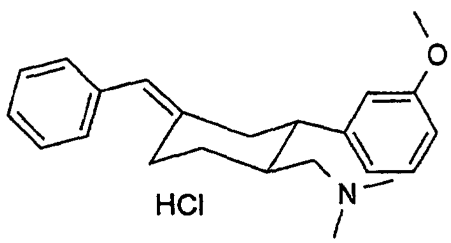

(2)

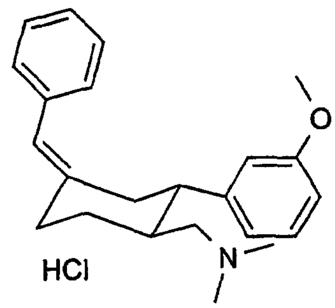

(3)

Unter Einsatz von

*rac-trans*-4-Dimethylaminomethyl-3-(3-methoxy-phenyl)-cyclohexanon anstelle von Dimethylaminomethyl-3-(3-methoxy-phenyl)-cyclo-hex-2-enon in Beispiel 1

und Benzyltriphenylphosphoniumchlorid anstelle von 3-(Benzoesäuremethylester)-methyltriphenylphosphoniumchlorid in Beispiel 1

erhielt man nach der in Beispiel 1 beschriebenen Verfahrensweise:

*rac-trans-Z*-[4-Benzyliden-2-(3-methoxy-phenyl)-cyclohexylmethyl]-dimethylamin Hydrochlorid (3) und *rac-trans-E*-[4-Benzyliden-2-(3-methoxy-phenyl)-cyclohexylmethyl]-dimethylamin Hydrochlorid (2)

**Beispiel 39**

*rac-trans-Z*-3-(5-Benzyliden-2-dimethylaminomethyl-cyclohexyl)-phenol; Hydrochlorid (23)

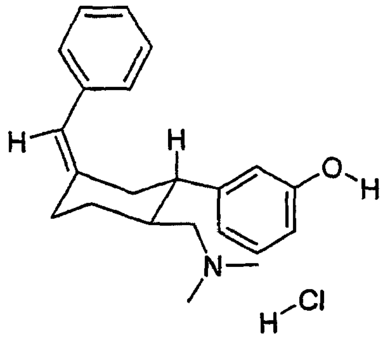

(23)

Unter Stickstoffatmosphäre wurden 140 ml Diisobutylaluminiumhydrid-Lösung (25 Gew.% Lsg in Toluol) vorgelegt. Anschließend wurden bei Raumtemperatur 2.4 *rac-trans-Z*-[4-Benzyliden-2-(3-methoxy-phenyl)-cyclohexylmethyl]-dimethylamin Base, die gemäß Beispiel 39 hergestellt wurde, gelöst in 20 ml Toluol p.a. zugetropft. Es wurde zwei Stunden unter Rückfluß erhitzt. Nach Beendigung der Reaktion wurde das Gemisch auf 0°C abgekühlt und mit einem Gemisch aus 25 ml Ethanol und 20 ml Wasser gequenscht. Der ausgefallene Niederschlag wurde abgesaugt und mit Essigester gewaschen. Die organische Phase wurde über Magnesiumsulfat getrocknet und anschließend im Vakuum vom Lösemittel befreit. Der Rückstand wurde säulenchromatographisch an Kieselgel mit Essigsäureethylester/Methanol = 9/1 gereinigt. Man erhielt so 1 g der Titelverbindung als Base. Zur Freisetzung des Hydrochlorids wurde die Base in 20 ml Aceton gelöst und mit einer äquimolaren Menge Trimethylchlorsilan und Wasser versetzt. So erhielt man 300 mg (17.1 % d. Th.) *rac-trans-Z*-3-(5-Benzyliden-2-dimethylaminomethylcyclohexyl)-phenol; Hydrochlorid (23) in Form weißer Kristalle.

**Beispiel 40**

*rac-trans-E*-3-(5-Benzyliden-2-dimethylaminomethyl-cyclohexyl)-phenol; Hydrochlorid (20)

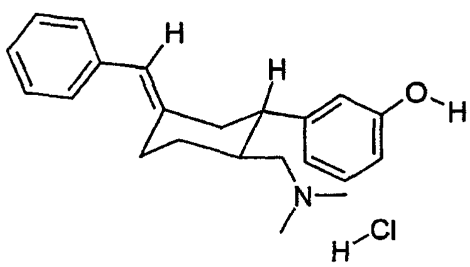

(20)

Unter Einsatz von:

*rac-trans-E*-[4-Benzyliden-2-(3-methoxy-phenyl)-cyclohexylmethyl]-dimethylamin Base

anstelle von *rac-trans-Z*-[4-Benzyliden-2-(3-methoxy-phenyl)-cyclohexylmethyl]-dimethylamin Base in Beispiel 39 erhielt man nach der in Beispiel 39 beschriebenen Vorgehensweise *rac-trans-E*-3-(5-Benzyliden-2-dimethylaminomethyl-cyclohexyl)-phenol; Hydrochlorid (20)

**Beispiel 41**

*rac-cis-Z*-[4-Benzyliden-2-(3-methoxy-phenyl)-cyclohexylmethyl]-dimethylamin Hydrochlorid (4) und *rac-cis-E*-[-4-Benzyliden-2-(3-methoxy-phenyl)-cyclohexylmethyl]-dimethylamin Hydrochlorid (1)

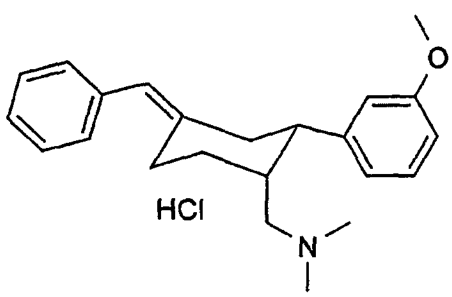

(1)

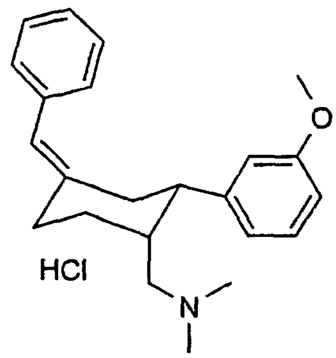

(4)

Unter Einsatz von

*rac-cis*-4-Dimethylaminomethyl-3-(3-methoxy-phenyl)-cyclohexanon anstelle von Dimethylaminomethyl-3-(3-methoxy-phenyl)-cyclo-hex-2-enon in Beispiel 1

und Benzyltriphenylphosphoniumchlorid anstelle von 3-(Benzoesäuremethylester)-methyltriphenylphosphoniumchlorid in Beispiel 1

erhielt man nach der in Beispiel 1 beschriebenen Verfahrensweise:

*rac-cis-Z*-[4-Benzyliden-2-(3-methoxy-phenyl)-cyclohexylmethyl]-dimethylamin Hydrochlorid (4) und *rac-cis-E*-[-4-Benzyliden-2-(3-methoxy-phenyl)-cyclohexylmethyl]-dimethylamin Hydrochlorid (1)

**Beispiel 42**

*rac-trans-E*-[3-(4-Dimethylaminomethyl-3-(3-methoxy-phenyl)-cyclohexylidenmethyl)-phenyl)-methanol]; Hydrochlorid (29)

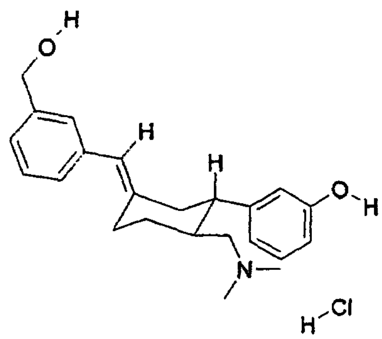

(29)

Unter Einsatz von

*rac-trans-E*-3-[4-Dimethylaminomethyl-3-(3-methoxy-phenyl)-cyclohexylidenemethyl]-benzoesäuremethylester, das gemäß Beispiel 2 dargestellt wurde,

anstelle von Z-3-[4-Dimethylaminomethyl-3-(3-methoxy-phenyl)-2-methyl-cyclohex-2-enylidenmethyl]-benzoesäuremethylester in Beispiel 15 erhielt man nach der in Beispiel 15 beschriebenen Vorgehensweise *rac-trans-E*-3-[4-Dimethylaminomethyl-3-(3-methoxy-phenyl)-cyclohexylidenmethyl)-phenyl)-methanol]; Hydrochlorid (29)

**Beispiel 43**

Die folgenden Verbindungen wurden nach den vorangehend beschriebenen Vorschriften hergestellt. Die jeweilige Struktur ist durch ein NMR-Spektrum belegt:

rac-cis-Z-3-[4-Dimethylaminomethyl-3-(3-hydroxy-phenyl)-cyclohexylidenmethyl]-benzoesäure,
rac-cis-Z-3-[4-Dimethylaminomethyl-3-hydroxy-3-(3-methoxy-phenyl)-cyclohexylidenemethyl]-benzoesäuremethylester,
rac-cis-E-3-[3-Chloro-4-dimethylaminomethyl-3-(3-methoxy-phenyl)-cyclohexylidenmethyl]-benzoesäuremethylester,
rac-cis-E-3-[4-Dimethylaminomethyl-3-hydroxy-3-(3-methoxy-phenyl)-cyclohexylidenmethyl]-benzoesäuremethylester,
rac-cis-Z-3-[4-Dimethylaminomethyl-3-hydroxy-3-(3-methoxy-phenyl)-cyclohexylidenemethyl]-benzoesäuremethylester,
(+)-trans-E-3-[4-Dimethylaminomethyl-3-(3-methoxy-phenyl)-cyclohexylidenmethyl]-benzoesäure,
(-)-trans-E-3-[4-Dimethylaminomethyl-3-(3-methoxy-phenyl)-cyclohexylidenmethyl]-benzoesäure,
rac-trans-E-3-(4-Dimethylaminomethyl-3-phenylcyclohexylidenemethyl)-benzoesäuremethylester,
. rac-trans-Z-3-(4-Dimethylaminomethyl-3-phenyl-cyclohexylidenmethyl)-benzoesäuremethylester,
rac-cis-E-3-(4-Dimethylaminomethyl-3-phenyl-cyclohexylidenmethyl)-benzoesäure,
rac-cis-E-3-(4-Dimethylaminomethyl-3-phenyl-cyclohexylidenmethyl)-benzoesäure,
*rac-trans-Z*-3-(4-Dimethylaminomethyl-3-phenylcyclohexylidenmethyl)benzoesäure,
rac-trans-E-3-(4-Dimethylaminomethyl-3-phenylcyclohexylidenemethyl)-benzoesäure,
rac-trans-E-3-[4-Dimethylaminomethyl-3-(3-trifluoromethyl-phenyl)-cyclohexylidenmethyl]-benzoesäure,
rac-trans-Z-3-[4-Dimethylaminomethyl-3-(3-trifluoromethyl-phenyl)-cyclohexylidenmethyl]-benzoesäure,
rac-trans-E-3-[4-Dimethylaminomethyl-3-(3-fluoro-phenyl)-cyclohexylidenmethyl]-benzoesäuremethylester,

rac-trans-Z-3-[4-Dimethylaminomethyl-3-(3-fluoro-phenyl)-cyclohexylidenmethyl]benzoesäuremethylester,
rac-cis-E-3-[4-Dimethylaminomethyl-3-(3-fluoro-phenyl)-cyclohexylidenemethyl]-benzoesäuremethylester,
rac-trans-E-3-[4-Dimethylaminomethyl-3-(3-fluoro-phenyl)-cyclohexylidenemethyl]-benzoesäure,
rac-trans-Z-3-[4-Dimethylaminomethyl-3-(3-fluoro-phenyl)-cyclohexylidenemethyl]-benzoesäure,
rac-cis-E-3-[4-Dimethylaminomethyl-3-(3-fluoro-phenyl)-cyclohexylidenemethyl]-benzoesäure
E-[4-Ethyliden-2-(3-methoxy-phenyl)-cyclohex-2-enylmethyl]-dimethylamin
[4-Isopropyliden-2-(3-methoxy-phenyl)-cyclohex-2-enylmethyl]-dimethylamin,
E-[2-(3-Methoxy-phenyl)-4-propyliden-cyclohex-2-enylmethyl]-dimethylamin,
E-[4-Butyliden-2-(3-methoxy-phenyl)-cyclohex-2-enylmethyl]-dimethylamin.

**Beispiel 44**

**Pharmakologische Untersuchungen**

**Writhing-Test**

Die antinociceptive Wirksamkeit der erfindungsgemäßen Verbindungen wurde im Phenylchinon-induzierten Writhing-Test, modifiziert nach I.C. Hendershot, J. Forsaith, J.Pharmacol. Exp. Ther. 125, 237 - 240 (1959) an der Maus untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0.3 ml/ Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die physiologische Kochsalzlösung i.v. und Phenyylchinon i.v. erhielten.

Alle Substanzen wurden in der Standarddosis von 10 mg/kg getestet. Die prozentuale Hemmung (% Hemmung) der Writhingreaktionen durch eine Substanz wurde nach folgender Formel berechnet:

%Hemmung = 100 -[WR behandelte Tiere/WR Kontrolle x 100]

Alle untersuchten erfindungsgemäßen Verbindungen zeigten eine mittelstarke bis starke analgetische Wirkung.
Die Ergebnisse ausgewählter Wrihting-Untersuchungen sind in der Tabelle 1 zusammengefaßt

Tabelle 1: Analgesieprüfung im Writhing-Test an der Maus

| Verbindung | % Hemmung der Writhing-Reaktionen 10 mg/kg i.v. |
|---|---|
| 8 | 98 |
| 10 | 84 |
| 13 | 85 |
| 14 | 94 |
| 15 | 100 |
| 16 | 98 |
| 17 | 85 |
| 18 | 100 |
| 19 | 82 |
| 20 | 84 |
| 21 | 79 |
| 22 | 93 |
| 23 | 88 |

| Verbindung | % Hemmung der Writhing-Reaktionen 10 mg/kg i.v. |
|---|---|
| 24 | 100 |
| 25 | 100 |
| 26 | 87 |
| 28 | 100 |
| 29 | 94 |
| 30 | 84 |
| 32 | 100 |
| 33 | 90 |
| 34 | 78 |
| 35 | 100 |
| 36 | 100 |
| 37 | 91 |
| 39 | 78 |
| 48 | 98 |

## Patentansprüche

1. Substituierte Aminomethyl-Phenyl-Cyclohexanderivate der allgemeinen Formel I bzw. Ia auch in Form ihrer Diastereomere oder Enantiomere sowie ihrer freien Base oder eines mit einer physiologisch verträglichen Säure gebildeten Salzes, insbesondere des Hydrochloridsalzes,

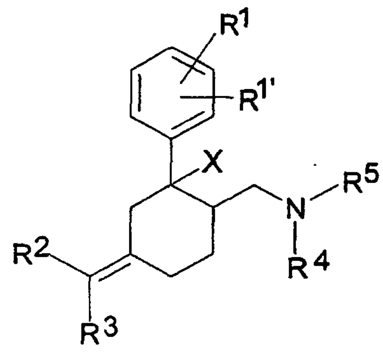

I

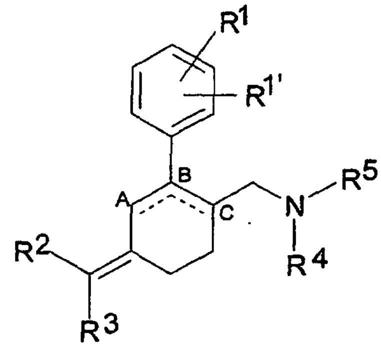

Ia

, worin

$R^1$ und $R^{1'}$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; F, Cl, Br, I, $NR^6R^{6'}$, $NO_2$, CN, $OR^6$, $SR^6$, $OC(O)R^6$, $C(O)OR^6$, C(O) $R^6$ oder $C(O)NR^6R^{6'}$, wobei $R^6$ und $R^{6'}$ ausgewählt sind aus

H; $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^1$ und $R^{1'}$ zusammen -CH=CH-CH=CH- bilden, wobei das entstehende Naphthylsystem ein- oder mehrfach substituiert sein kann,

X ausgewählt ist aus

H, F, Cl, Br I, $CF_3$, $OS(O_2)C_6H_4$-$pCH_3$, $OR^7$ oder $OC(O)R^7$, wobei $R^7$ ausgewählt ist aus

H; $C_1$-$C_{10}$-Alkyl, $C_2$-$C_{10}$-Alkeny) oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder,

wenn die Verbindung kein X aufweist, gemäß Formel Ia zwischen C-Atom A und C-Atom B oder C-Atom B und C-Atom C eine Doppelbindung ausgebildet ist,

$R^4$, $R^5$ unabhängig voneinander ausgewählt sind aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^4$ und $R^5$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^8$ ersetzt ist, mit $R^8$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

und

$R^2$, $R^3$ unabhängig voneinander augewählt sind aus

$R^9$ oder $YR^9$ mit Y = $C_1$-$C_{10}$-Alkyl, $C_2$-$C_{10}$-Alkenyl oder $C_2$-$C_{10}$-Alkinyl, verzweigt oder unverzweigt, einfach oder mehrfach substituiert oder unsubstituiert, wobei $R^9$ ausgewählt ist aus

H, F, Cl, Br, I, CN, $NO_2$, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{10}$ ersetzt ist, mit $R^{10}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

$OR^{11}$, $OC(O)R^{11}$, $OC(O)OR^{11}$, $OC(S)R^{11}$, $C(O)R^{11}$, $C(O)OR^{11}$, $C(S)R^{11}$, $C(S)OR^{11}$, $SR^{11}$, $S(O)R^{11}$ bzw. $S(O_2)R^{11}$, wobei $R^{11}$ ausgewählt ist aus

H, $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{12}$ ersetzt ist, mit $R^{12}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

$NR^{13}R^{14}$, $NR^{13}C(O)R^{14}$, $C(O)NR^{13}R^{14}$ oder $S(O_2)NR^{13}R^{14}$, wobei $R^{13}$ und $R^{14}$ unabhängig voneinander ausgewählt sind aus

H, O; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{15}$ ersetzt ist, mit $R^{15}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^{13}$ und $R^{14}$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{16}$ ersetzt ist, mit $R^{16}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

oder

Alkylaryl, Aryl oder Heteroaryl, jeweils einfach oder mehrfach substituiert oder unsubstituiert; insbesondere

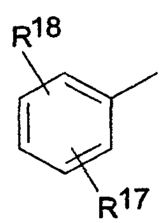

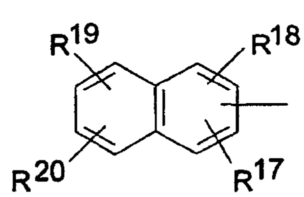

oder

, wobei $R^{17}$, $R^{18}$, $R^{19}$ und $R^{20}$ unabhängig voneinander ausgewählt sind aus

$R^{21}$ oder $ZR^{21}$ mit Z = $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert, wobei $R^{21}$ ausgewählt ist aus

H, F, Cl, Br, I, CN, $NO_2$; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{22}$ ersetzt ist, mit $R^{22}$ ausgewählt aus H, $C_1$-$C_{10}$-Alkyl, $C_2$-$C_{10}$--Alkenyl oder $C_2$-$C_{10}$-Alkinyl, jeweils verzweigt oder unverzweigt, einfach oder mehrfach substituiert oder unsubstituiert;

Alkylaryl, Aryl oder Heteroaryl, jeweils einfach oder mehrfach substituiert oder unsubstituiert;

$OR^{23}$, $OC(O)R^{23}$, $OC(O)OR^{23}$, $OC(S)R^{23}$, $C(O)R^{23}$, $C(O)OR^{23}$, $C(S)R^{23}$, $C(S)OR^{23}$, $SR^{23}$, $S(O)R^{23}$ bzw. $S(O_2)R^{23}$, wobei $R^{23}$ ausgewählt ist aus

H, $C_1$-$C_{18}$-Alkyl, $C_2$-$C_{18}$-Alkenyl bzw. $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{24}$ ersetzt ist, mit $R^{24}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

$NR^{25}R^{26}$, $NR^{25}C(O)R^{26}$, $C(O)NR^{25}R^{26}$ oder $S(O_2)NR^{25}R^{26}$, wobei $R^{25}$ und $R^{26}$ unabhängig voneinander ausgewählt sind aus

H; $C_1$-$C_{18}$-Alkyl, $C_2$-$C_{18}$-Alkenyl oder $C_2$-$C_{18}$-Alkinyl, jeweils verzweigt oder unverzweigt, einfach oder mehrfach substituiert oder unsubstituiert; $C_3$-$C_7$-Cycloalkyl, gesättigt oder ungesättigt, einfach oder mehrfach substituiert oder unsubstituiert, bzw. einem entsprechenden Heterocyclus, bei dem ein C-Atom im Ring durch S, O oder $NR^{27}$ ersetzt ist, mit $R^{27}$ ausgewählt aus

H, $C_1$-$C_{10}$-Alkyl, $C_2$-$C_{10}$-Alkenyl oder $C_2$-$C_{10}$--Alkinyl, jeweils verzweigt oder unverzweigt, einfach oder mehrfach substituiert oder unsubstituiert;

Alkylaryl, gesättigt oder ungesättigt, einfach oder mehrfach substituiert oder unsubstituiert; Aryl oder Heteroaryl, jeweils einfach oder mehrfach substituiert oder unsubstituiert;

oder

$R^{25}$ und $R^{26}$ zusammen ein $C_3$-$C_7$-Cycloalkyl, gesättigt oder ungesättigt, einfach oder mehrfach substituiert oder unsubstituiert,bilden, bzw. einen entsprechenden Heterocyclus, bei dem ein C-Atom im Ring durch S, O oder $NR^{28}$ ersetzt ist, mit $R^{27}$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert oder unsubstituiert.

2. Substituierte Aminomethyl-Phenyl-Cyclohexanderivate nach Anspruch 1, **dadurch gekennzeichnet, daß** $R^2$ und $R^3$ unterschiedliche Bedeutung haben und/oder $R^3$ = H oder $CH_3$, vorzugsweise H, ist, während $R^1$, $R^{1'}$, $R^2$, $R^4$, $R^5$ und X eine der in den vorangehenden Ansprüchen genannten Bedeutungen haben.

3. Substituierte Aminomethyl-Phenyl-Cyclohexanderivate nach einem der Ansprüche 1 oder 2, **dadurch gekennzeichnet, daß**
$R^2$ ausgewählt ist aus

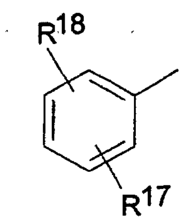

oder

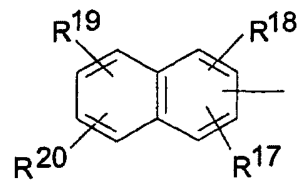

, wobei $R^{17}$, $R^{18}$, $R^{19}$ und $R^{20}$ sowie $R^1$, $R^{1'}$, $R^3$ , $R^4$, $R^5$ und X eine der in den vorangehenden Ansprüchen genannten Bedeutungen haben
oder $R^2$ ausgewählt ist aus $C_{1-3}$-Alkyl.

4. Substituierte Aminomethyl-Phenyl-Cyclohexanderivate nach einem der Ansprüche 1-3, **dadurch gekennzeichnet, daß** $R^2$

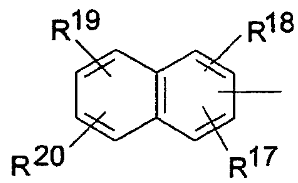

ist und $R^{19}$ und $R^{20}$ H sind, während $R^1$, $R^{1'}$, $R^3$, $R^4$, $R^5$, X sowie $R^{17}$ und $R^{18}$ eine der in den vorangehenden Ansprüchen genannten Bedeutungen haben.

5. Substituierte Aminomethyl-Phenyl-Cyclohexanderivate nach einem der Ansprüche 1-4, **dadurch gekennzeichnet, daß** $R^2$ ausgewählt ist aus

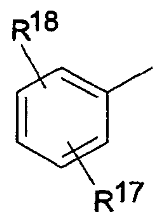

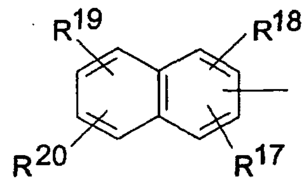

oder

, $R^{17}$ und $R^{18}$ unterschiedliche Bedeutung haben und/oder $R^{18}$ = $R^{21}$ mit $R^{21}$ ausgewählt aus

H, F, Cl, Br, I, $CF_3$ oder $OR^{23}$ mit $R^{23}$ = H, Methyl- Ethyl-, Propyl-, Isopropyl-, Butyl- oder Isobutyl-,

während $R^1$, $R^{1'}$, $R^3$, $R^4$, $R^5$ und X sowie $R^{17}$, $R^{19}$ und $R^{20}$ eine der in den vorangehenden Ansprüchen genannten Bedeutungen haben.

6. Substituierte Aminomethyl-Phenyl-Cyclohexanderivate nach einem der Ansprüche 1 - 5, **dadurch gekennzeichnet, daß** $R^2$ ausgewählt ist aus

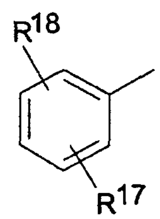

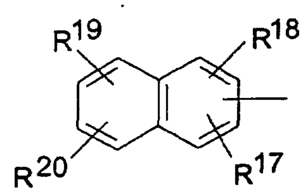

oder

, wobei $R^{17}$ ausgewählt ist aus

$R^{21}$, wobei $R^{21}$ ausgewählt ist aus

H, F, Cl, Br, I, $CF_3$, $OR^{23}$, $OC(O)R^{23}$, $C(O)R^{23}$ oder $C(O)OR^{23}$,vorzugsweise H, F, Cl, $C(O)OR^{23}$,

wobei $R^{23}$ ausgewählt ist aus

H; $C_1$-$C_6$-Alkyl, $C_2$-$C_8$-Alkenyl oder $C_2$-$C_8$-Alkinyl, insbesondere $C_1$-$C_4$-Alkyl, verzweigt oder unverzweigt, einfach oder mehrfach substituiert oder unsubstituiert; vorzugsweise H, Methyl- Ethyl-, Propyl-, Isopropyl-, Butyl-. oder Isobutyl-, insbesondere H, $CH_3$, $C_2H_5$, oder Isobutyl-;

$C(O)NR^{25}R^{26}$, wobei $R^{25}$ und $R^{26}$ unabhängig voneinander ausgewählt sind aus

H, O; $C_1$-$C_{18}$-Alkyl, insbesondere $C_1$-$C_4$-Alkyl, verzweigt oder unverzweigt, gesättigt oder ungesättigt, einfach oder mehrfach substituiert oder unsubstituiert, vorzugsweise H, Methyl-Ethyl-, Propyl-, Isopropyl-, Butyl- oder Isobutyl-, insbesondere $C_2H_5$;

oder $ZR^{21}$ mit Z = $CH_2$ oder $C_2H_4$, wobei $R^{21}$ ausgewählt ist aus

$OR^{23}$, $OC(O)R^{23}$, $C(O)R^{23}$ oder $C(O)OR^{23}$, vorzugsweise $OR^{23}$, wobei $R^{23}$, ausgewählt ist aus

H; $C_1$-$C_6$-Alkyl, $C_2$-$C_8$-Alkenyl oder $C_2$-$C_8$-Alkinyl, insbesondere $C_{1-}C_4$-Alkyl, verzweigt oder unverzweigt, einfach oder mehrfach substituiert oder unsubstituiert, vorzugsweise H, Methyl- Ethyl-, Propyl-, Isopropyl-, Butyloder Isobutyl-, insbesondere H;

C(O)$NR^{25}R^{26}$, wobei $R^{25}$ und $R^{26}$ unabhängig voneinander ausgewählt sind aus

H, O, $C_1$-$C_{18}$-Alkyl, insbesondere $C_1$-$C_4$-Alkyl, verzweigt oder unverzweigt, gesättigt oder ungesättigt, einfach oder mehrfach substituiert oder unsubstituiert, vorzugsweise H, Methyl-Ethyl-, Propyl-, Isopropyl-, Butyl- oder Isobutyl-;

während $R^1$, $R^{1'}$, $R^3$, $R^4$, $R^5$ und X sowie $R^{18}$, $R^{19}$ und $R^{20}$ eine der in den vorangehenden Ansprüchen genannten Bedeutungen haben.

7. Substituierte Aminomethyl-Phenyl-Cyclohexanderivate nach einem der Ansprüche 1 bis 6, **dadurch gekennzeichnet, daß**

$R^1$ ausgewählt ist aus

H, F, Cl, Br, I, $CF_3$, $SCH_3$ oder $OR^6$, vorzugsweise $OR^6$, wobei $R^6$ ausgewählt ist aus

H; $C_1$-$C_4$-Alkyl, verzweigt oder unverzweigt, einfach oder mehrfach substituiert oder unsubstituiert; vorzugsweise H oder $CH_3$;

und/oder

$R^{1'}$ ausgewählt ist aus

H, F, Cl, $SCH_3$ oder $OCH_3$, vorzugsweise H

und/oder

X ausgewählt ist aus

H, F, Br, I, Cl, $OR^7$, vorzugsweise H, F, Cl, $OR^7$, mit $R^7$ = H oder $CH_3$, vorzugsweise H;

oder,

wenn die Verbindung kein X aufweist, gemäß Formel Ia zwischen C-Atom A und C-Atom B oder C-Atom B und C-Atom C eine Doppelbindung ausgebildet ist;

und/oder

$R^4$ und $R^5$ unabhängig voneinander ausgewählt sind aus

$C_1$-$C_4$-Alkyl, verzweigt oder unverzweigt, einfach oder mehrfach substituiert oder unsubstituiert, vorzugsweise $CH_3$,

während $R^2$ und $R^3$ eine der in den vorangehenden Ansprüchen genannten Bedeutungen haben.

8. Substituierte Aminomethyl-Phenyl-Cyclohexanderivate, gemäß einem der Ansprüche 1 bis 5, **dadurch gekennzeichnet, daß** es sich um

*rac-cis-E*-[-4-Benzyliden-2-(3-methoxy-phenyl)-cyclohexylmethyl]-dimethylamin,
*rac-trans-E*-[4-Benzyliden-2-(3-methoxy-phenyl)-cyclohexylmethyl]-dimethylamin,
*rac-trans-Z*-[4-Benzyliden-2-(3-methoxy-phenyl)-cyclohexylmethyl]-dimethylamin,
*rac-cis-Z*-[4-Benzyliden-2-(3-methoxy-phenyl)-cyclohexylmethyl]-dimethylamin,
*rac-cis-E*-3-[4-Dimethylaminomethyl-3-(3-methoxy-phenyl)-cyclohexylidenmethyl]-benzoesäuremethylester,
*rac-cis-Z*-3-[4-Dimethylaminomethyl-3-(3-methoxy-phenyl)-cyclohexylidenmethyl]-benzoesäuremethylester,
*rac-trans-Z*-3-[4-Dimethylaminomethyl-3-(3-methoxy-phenyl)-cyclohexylidenmethyl]-benzoesäuremethylester,
*rac-trans-E*-3-[4-Dimethylaminomethyl-3-(3-methoxy-phenyl)-cyclohexylidenmethyl]-benzoesäuremethylester,
*Z*-3-[4-Dimethylaminomethyl-3-(3-methoxy-phenyl)-2-methyl-cyclohex-2-enylidenmethyl]-benzoe-säuremethylester,
*E*-3-[4-Dimethylaminomethyl-3-(3-methoxy-phenyl)-2-methyl-cyclohex-2-enylidenmethyl]-benzoesäuremethylester,
*Z*-3-[4-Dimethylaminomethyl-3-(3-methoxy-phenyl)-2-methyl-cyclohex-2-enylidenmethyl]-naphthalen-1-carbonsäureethylester,
*E*-3-[4-Dimethylaminomethyl-3-(3-methoxy-phenyl)-2-methyl-cyclohex-2-enylidenmethyl]-naphthalen-1-carbonsäureethylester,
*Z*-3-[4-Dimethylaminomethyl-3-(3-methoxy-phenyl)-cyclohexylidenmethyl]-2-fluor -benzoesäureethylester

*Z*-3-[4-Dimethylaminomethyl-3-(3-methoxy-phenyl)-cyclohex-2-enylidenmethyl]-benzoesäure,
*E*-3-[4-Dimethylaminomethyl-3-(3-methoxy-phenyl)-cyclohex-2-enylidenmethyl]-benzoesäure,
*E*-{3-[4-Dimethylaminomethyl-3-(3-methoxy-phenyl)-cyclohex-2-enylidenmethyl]-phenyl}-methanol,
*rac-trans*-Z-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäure,
*rac-trans-E*-[3-(2-Dimethylaminomethyl-5-(3-hydroxymethyl-benzyliden)-cyclohexyl)-phenol,
*rac-trans-E*-3-(5-Benzyliden-2-dimethylaminomethyl-cyclohexyl)-phenol,
*E*-3-[4-Dimethylaminomethyl-3-(3-methoxy-phenyl)-cyclohex-2-enylidenmethyl]-benzoesäure-tert.-butylester,
*E*-3-[6-Dimethylaminomethyl-3-(3-hydroxymethyl-benzyliden)-cyclohex-1-enyl]-phenol,
*rac-trans-Z*-3-(5-Benzyliden-2-dimethylaminomethyl-cyclohexyl)-phenol,
*Z*-3-[2-Dimethylaminomethyl-5-(3-hydroxymethyl-benzyliden)-cyclohex-1-enyl]-phenol,
*Z*-{3-[4-Dimethylaminomethyl-3-(3-methoxy-phenyl)-cyclohex-2-enylidenmethyl]-phenyl}-methanol,
*rac-cis-E*-4-Dimethylaminomethyl-3-(3-methoxy-phenyl)-cyclohexylidenmethyl]-benzoesäure,
*rac-cis-Z*-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäuretert-butylester,
*rac-trans-E*-13-[4-Dimethylaminomethyl-3-(3-methoxy-phenyl)-cyclohexylidenmethyl)-phenyl)-methanol],
*rac-cis-Z*-3-[4-Dimethylaminomethyl-3-(3-methoxy-phenyl)-cyclohexylidenmethyl]-benzoesäureethylester,
*E*-3-[4-Dimethylaminomethyl-3-(3-methoxy-phenyl)-cyclohex-2-enylidenmethyl]-N,N-diethyl-benzamid
*rac-trans-E*-3-[4-Dimethylaminomethyl-3-(3-methoxy-phenyl)-cyclohex-2-enylidenmethyl]-N,N-diethyl-benzamid,
*E*-3-[4-Dimethylaminomethyl-3-(3-methoxy-phenyl)-cyclohex-2-enylidenmethyl]-benzoesäureethylester
*Z*-3-[4-Dimethylaminomethyl-3-(3-methoxy-phenyl)-cyclohex-2-enylidenmethyl]-N,N-diethyl-benzamid,
*rac-cis-E*-3-[4-Dimethylaminomethyl-3-(3-methoxy-phenyl)-cyclohexylidenmethyl]-N,N-diethyl-benzamid,
*rac-trans-Z*-3-[4-Dimethylaminomethyl-3-(3-methoxy-phenyl)-cyclohexylidenmethyl]-N,N-diethyl-benzamid,
*E*-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-hydroxy-phenyl)-cyclohex-2-enylidenmethyl]-benzoesäuremethylester,
*Z* 3-[4-Dimethylaminomethyl-3-(3-methoxy-phenyl)-cyclohex-2-enylidenmethyl]-benzoesäureisobutylester,
*Z*-3-[6-Dimethylaminomethyl-3-(3-hydroxymethyl-benzyliden)-cyclohex-1-enyl]-phenol
*Z*-[4-(4-Chloro-benzyliden)-2-(3-methoxy-phenyl)-cyclohex-2-enylmethyl]-dimethyl-amin,
*E-[4-(* 4-Fluoro-benzyliden )-2-(3-methoxy-phenyl)-cyclohex-2-enylmethyl]-dimethyl-amin,
*Z*-[4-(4-Fluoro-benzyliden)-2-(3-methoxy-phenyl)-cyclohex-2-enylmethyl]-dimethyl-amin,
*E*-3-[6-Dimethylaminomethyl-3-(4-fluor-benzyliden)-cyclohex-1-enyl]-phenol,
*Z*-3-[6-Dimethylaminomethyl-3-(4-fluor-benzyliden)-cyclohex-1-enyl]-phenol,
*E*-[4-(4-Chlor-benzyliden)-2-(3-methoxy-phenyl)-cyclohex-2-enylmethyl]-dimethyl-amin
*E*-3-[3-(4-Chlor-benzyliden)-6-dimethylaminomethyl-cyclohex-1-enyl]-phenol,
*Z*-3-[4-Dimethylaminomethyl-3-(3-hydroxy-phenyl)-cyclohex-2-enylidenmethyl]-benzoesäuremethylester,
*E*-3-[4-Dimethylaminomethyl-3-(3-hydroxy-phenyl)-cyclohex-2-enylidenmethyl]-benzoesäure,
*rac-cis-E*-3-[4-Dimethylaminomethyl-3-(3-hydroxy-phenyl)-cyclohexylidenmethyl]-benzoesäuremethylester,
*rac-cis-Z*-3-[4-Dimethylaminomethyl-3-(3-hydroxy-phenyl)-cyclohexylidenmethyl]-benzoesäuremethylester,
*rac-trans-Z*-3-[4-Dimethylaminomethyl-3-(3-hydroxy-phenyl)-cyclohexylidenmethyl-benzoesäure,
*rac-trans-E*-[4-Dimethylaminomethyl-3-(3-hydroxy-phenyl)-cyclohexylidenmethyl]-benzoesäure,
rac-cis-Z-3-[4-Dimethylaminomethyl-3-(3-hydroxy-phenyl)-cyclohexylidenmethyl]-benzoesäure,
rac-cis-Z-3-[4-Dimethylaminomethyl-3-hydroxy-3-(3-methoxy-phenyl)-cyclohexylidenemethyl]-benzoesäuremethylester,
rac-cis-E-3-[3-Chloro-4-dimethylaminomethyl-3-(3-methoxy-phenyl)-cyclohexylidenmethyl]-benzoesäuremethylester,
rac-cis-E-3-[4-Dimethylaminomethyl-3-hydroxy-3-(3-methoxy-phenyl)-cyclohexylidenmethyl]-benzoesäuremethylester,
rac-cis-Z-3-[4-Dimethylaminomethyl-3-hydroxy-3-(3-methoxy-phenyl)-cyclohexylidenemethyl]-benzoesäuremethylester,
(+)-trans-E-3-[4-Dimethylaminomethyl-3-(3-methoxy-phenyl)-cyclohexylidenmethyl]-benzoesäure,
(-)-trans-E-3-[4-Dimethylaminomethyl-3-(3-methoxy-phenyl)-cyclohexylidenmethyl]-benzoesäure,
rac-trans-E-3-(4-Dimethylaminomethyl-3-phenylcyclohexylidenemethyl)-benzoesäuremethylester,
rac-trans-Z-3-(4-Dimethylaminomethyl-3-phenyl-cyclohexylidenmethyl)-benzoesäuremethylester,
rac-cis-E-3-(4-Dimethylaminomethyl-3-phenyl-cyclohexylidenmethyl)-benzoesäure,
rac-cis-E-3-(4-Dimethylaminomethyl-3-phenyl-cyclohexylidenmethyl)-benzoesäure,

rac-trans-Z-3-(4-Dimethylaminomethyl-3-phenylcyclohexylidenmethyl)benzoesäure,
rac-trans-E-3-(4-Dimethylaminomethyl-3-phenylcyclohexylidenemethyl)-benzoesäure,
rac-trans-E-3-(4-Dimethylaminomethyl-3-(3-trifluoromethyl-phenyl)-cyclohexylidenmethyl]-benzoesäure,
rac-trans-Z-3-[4-Dimethylaminomethyl-3-(3-trifluoromethyl-phenyl)-cyclohexylidenmethyl]-benzoesäure,
rac-trans-E-3-[4-Dimethylaminomethyl-3-(3-fluoro-phenyl)-cyclohexylidenmethyl]-benzoesäuremethylester,
rac-trans-Z-3-[4-Dimethylaminomethyl-3-(3-fluoro-phenyl)-cyclohexylidenmethyl]benzoesäuremethylester,
rac-cis-E-3-[4-Dimethylaminomethyl-3-(3-tluoro-phenyl)-cyclohexylidenemethyl]-benzoesäuremethylester,
rac-trans-E-3-[4-Dimethylaminomethyl-3-(3-fluoro-phenyl)-cyclohexylidenemethyl]-benzoesäure,
rac-trans-Z-3-[4-Dimethylaminomethyl-3-(3-fluoro-phenyl)-cyclohexylidenemethyl]-benzoesäure,
rac-cis-E-3-[4-Dimethylaminomethyl-3-(3-fluoro-phenyl)-cyclohexylidenemethyl]-benzoesäure
E-(4-Ethyliden-2-(3-methoxy-phenyl)-cyclohex-2-enylmethyl]-dimethylamin
[4-Isopropyliden-2-(3-methoxy-phenyl)-cyclohex-2-enylmethyl]-dimethylamin,
E-(2-(3-Methoxy-phenyl)-4-propyliden-cyclohex-2-enylmethyl]-dimethylamin,
E-[4-Butyliden-2-(3-methoxy-phenyl)-cyclohex-2-enylmethyl]-dimethylamin

sowie deren Salze, insbesondere Hydrochloridsalze, handelt.

**9.** Verfahren zur Herstellung substituierter Aminomethyl-Phenyl-Cyclohexanderivate der allgemeinen Formel I bzw. Ia

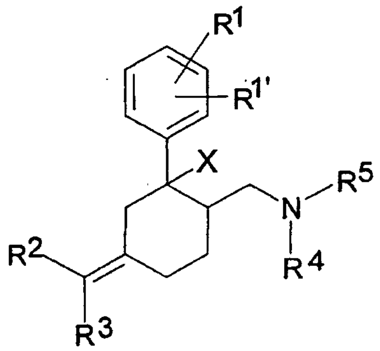

I

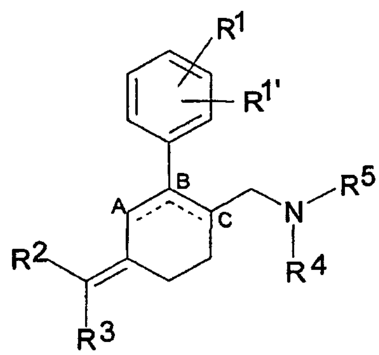

Ia

, in denen $R^1$, $R^{1'}$, $R^2$, $R^3$, $R^4$, $R^5$ und X eine der in Anspruch 1 genannten Bedeutungen haben, **dadurch gekennzeichnet, daß** Cyclohexanone der Formel II bzw. IIa

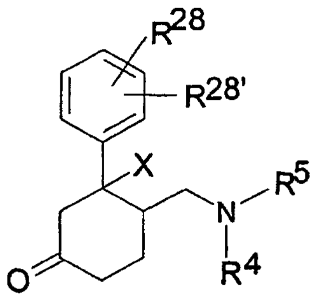

II

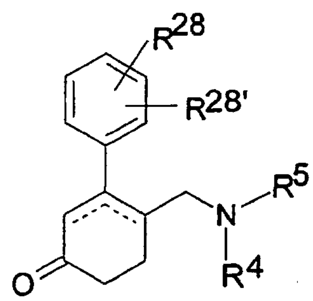

IIa

, in denen $R^4$, $R^5$ und X eine der in Anspruch 1 genannten Bedeutungen haben und $R^{28}$ eine der in Anspruch 1 für $R^1$ und $R^{28'}$ eine der in Anspruch 1 für $R^{1'}$ genannten Bedeutungen hat,
in einer Wittig-Reaktion in einem organischen Lösungsmittel in Gegenwart einer Base mit Alkyltriphenylphosphoniumsalzen der Formel III umgesetzt werden,

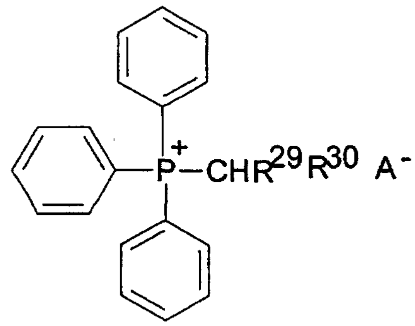

III

, wobei A Chlorid oder Bromid bedeutet und unabhängig voneinander $R^{29}$ eine der für $R^2$ und $R^{30}$ eine der für $R^3$ in Anspruch 1 genannten Bedeutungen haben.

**10.** Verfahren nach Anspruch 9, **dadurch gekennzeichnet, daß** das organische Lösungsmittel Benzol, Toluol oder ein chlorierter Kohlenwasserstoff ist und/oder als Base Kaliumtert.butylat oder Natriumhydrid verwendet werden und/oder die Reaktionstemperatur, insbesondere während der Wittig-Reaktion, zwischen 50°C und 90°C gehalten wird.

**11.** Verfahren gemäß einem der Ansprüche 9 oder 10, **dadurch gekennzeichnet, daß** in $R^{28}$ und/oder $R^{28'}$ gemäß Formel II bzw. IIa und/oder $R^{29}$ und/oder $R^{30}$ gemäß Formel III, mindestens eine OH-Gruppe durch eine OSi $(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wurde und nach Abschluß der Wittig-Reaktion mindestens eine $OSi(Ph)_2$tert.but.-Gruppe mit Tetrabutylammoniumfluorid in Tetrahydrofuran und/oder mindestens eine p-Methoxybenzylgruppe mit einem Metallamin, bevorzugt Natriumamin, abgespalten und/oder mindestens eine $NO_2$-Gruppe zu $NH_2$ reduziert wird.

**12.** Verfahren nach einem der Ansprüche 9 - 11 **dadurch gekennzeichnet, daß** nach der Wittig-Reaktion ein Verfahrensprodukt mit mindestens einer $C(O)OCH_3$- und/oder $C(S)OCH_3$-Gruppe mit KOH-Lösung bzw. NaOH-Lösung in Methanol bei 40°C - 60°C verseift wird.

**13.** Arzneimittel enthaltend als Wirkstoff mindestens ein substituiertes Aminomethyl-Phenyl-Cyclohexanderivat der allgemeinen Formel I bzw. Ia

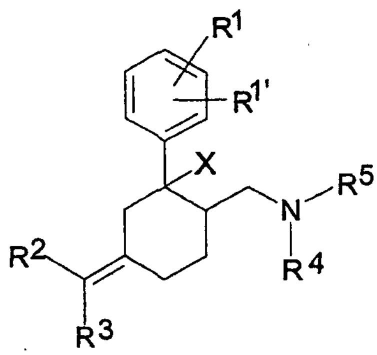

I

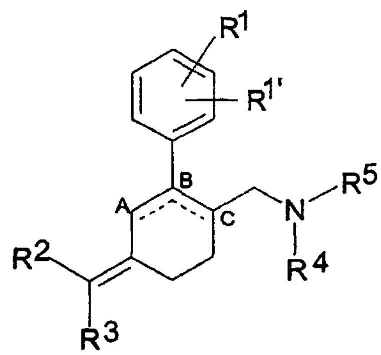

Ia

, in der $R^1$, $R^{1'}$, $R^2$, $R^3$, $R^4$, $R^5$ und X eine der in Anspruch 1 genannten Bedeutungen haben, in Form seiner Diastereomere oder Enantiomere sowie seiner freien Base oder eines mit einer physiologisch verträglichen Säure

gebildeten Salzes, insbesondere des Hydrochloridsalzes.

**14.** Arzneimittel gemäß Anspruch 13 enthaltend als Wirkstoff mindestens ein substituiertes Aminomethyl-Phenyl-Cyclohexanderivat ausgewählt aus der Gruppe

*rac-cis-E*-[-4-Benzyliden-2-(3-methoxy-phenyl)-cyclohexylmethyl]-dimethylamin,
*rac-trans-E* [4-Benzyliden-2-(3-methoxy-phenyl)-cyclohexylmethyl]-dimethylamin,
*rac-trans*-Z-[4-Benzyliden-2-(3-methoxy-phenyl)-cyclohexylmethyl]-dimethylamin,
*rac-cis-Z*-[4-Benzyliden-2-(3-methoxy-phenyl)-cyclohexylmethyl]-dimethylamin,
*rac-cis-E*-3-[4-D)methylaminomethyl-3-(3-methoxy-phenyl)-cyclohexylidenmethyl]-benzoesäuremethylester,
*rac-cis*-Z-3-[4-Dimethylaminomethyl-3-(3-methoxy-phenyl)-cyclohexylidenmethyl]-benzoesäuremethylester,
*rac-trans*-Z-3-[4-Dimethylaminomethyl-3-(3-methoxy-phenyl)-cyclohexylidenmethyl]-benzoesäuremethylester,
*rac-trans-E*-3-[4-Dimethylaminomethyl-3-(3-methoxy-phenyl)-cyclohexylidenmethyl]-benzoesäuremethylester,
*Z*-3-[4-Dimethylaminomethyl-3-(3-methoxy-phenyl)-2-methyl-cyclohex-2-enylidenmethyl]-benzoe-säuremethylester,
*E*-3-[4-Dimethylaminomethyl-3-(3-methoxy-phenyl)-2-methyl-cyclohex-2-enylidenmethyl]-benzoesäuremethylester,
*Z*-3-[4-Dimethylaminomethyl-3-(3-methoxy-phenyl)-2-methyl-cyclohex-2-enylidenmethyl]-naphthalen-1-carbonsäureethylester,
*E*-3-[4-Dimethylaminomethyl-3-(3-methoxy-phenyl)-2-methyl-cyclohex-2-enylidenmethyl]-naphthalen-1-carbonsäureethylester,
*Z*-3-[4-Dimethylaminomethyl-3-(3-methoxy-phenyl)-cyclohexylidenmethyl]-2-fluor-benzoesäureethylester
*Z*-3-[4-Dimethylaminomethyl-3-(3-methoxy-phenyl)-cyclohex-2-enylidenmethyl]-benzoesäure,
*E*-3-[4-Dimethylaminomethyl-3-(3-methoxy-phenyl)-cyclohex-2-enylidenmethyl]-benzoesäure,
*E*-{3-[4-Dimethylaminomethyl-3-(3-methoxy-phenyl)-cyclohex-2-enylidenmethyl]-phenyl}-methanol,
*rac-trans-Z*-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäure,
*rac-trans-E*-[3-(2-Dimethylaminomethyl-5-(3-hydroxymethyl-benzyliden)-cyclohexyl)-phenol,
*rac-trans-E*-3-(5-Benzyliden-2-dimethylaminomethyl-cyclohexyl)-phenol,
*E*-3-[4-Dimethylaminomethyl-3-(3-methoxy-phenyl)-cyclohex-2-enylidenmethyl]-benzoesäure-tert.-butytester,
*E*-3-[6-Dimethylaminomethyl-3-(3-hydroxymethyl-benzyliden)-cyclohex-1-enyl]-phenol,
*rac-trans-Z*-3-(5-Benzyliden-2-dimethylaminomethyl-cyclohexyl)-phenol,
*Z*-3-[2-Dimethylaminomethyl-5-(3-hydroxymethyl-benzyliden)-cyclohex-1-enyl]-phenol,
*Z*-{3-[4-Dimethylaminomethyl-3-(3-methoxy-phenyl)-cyclohex-2-enylidenmethyl]-phenyl]-methanol,
*rac-cis-E*-4-Dimethylaminomethyl-3-(3-methoxy-phenyl)-cyclohexylidenmethyl]-benzoesäure,
*rac-cis-Z*-3-[4-Dimethylaminomethyl-3-(3-methoxy-phenyl)-cyclohexylidenmethyl]-benzoesäure,
*rac-trans-E*-3-[4-Dimethylaminomethyl-3-(3-methoxy-phenyl)-cyclohexylidenmethyl]-benzoesäuretert-butylester,
*rac-trans-E*-[3-[4-Dimethylaminomethyl-3-(3-methoxy-phenyl)-cyclohexylidenmethyl)-phenyl)-methanol],
*rac-cis-Z*-3-[4-Dimethylaminomethyl-3-(3-methoxy-phenyl)-cyclohexylidenmethyl]-benzoesäureethylester,
*E*-3-[4-Dimethylaminomethyl-3-(3-methoxy-phenyl)-cyclohex-2-enylidenmethyl]-N,N-diethyl-benzamid
*rac-trans-E*-3-[4-Dimethylaminomethyl-3-(3-methoxy-phenyl)-cyclohex-2-enylidenmethyl]-N,N-diethyl-benzamid,
*E*-3-[4-Dimethylaminomethyl-3-(3-methoxy-phenyl)-cyclohex-2-enylidenmethyl]-benzoesäureethylester
*Z*-3-[4-Dimethylaminomethyl-3-(3-methoxy-phenyl)-cyclohex-2-enylidenmethyl]-N,N-diethyl-benzamid,
*rac-cis-E*-3-[4-Dimethylaminomethyl-3-(3-methoxy-phenyl)-cyclohexylidenmethyl]-N,N-diethyl-benzamid,
*rac-trans-Z*-3-[4-Dimethylaminomethyl-3-(3-methoxy-phenyl)-cyclohexylidenmethyl]-N,N-diethyl-benzamid,
*E*-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-hydroxy-phenyl)-cyclohex-2-enylidenmethyl]-benzoesäuremethylester,
*Z*-3-[4-Dimethylaminomethyl-3-(3-methoxy-phenyl)-cyclohex-2-enylidenmethyl]-benzoesäureisobutylester,
*Z*-3-[6-Dimethylaminomethyl-3-(3-hydroxymethyl-benzyliden)-cyclohex-1-enyl]-phenol
Z-[4-(4-Chloro-benzyliden)-2-(3-methoxy-phenyl)-cyclohex-2-enylmethyl]-dimethyl-amin,
*E-[*4-(4-Fluoro-benzyliden)-2-(3-methoxy-phenyl)-cyclohex-2-enylmethyl]-dimethyl-amin,
*Z*-[4-(4-Fluoro-benzyliden)-2-(3-methoxy-phenyl)-cyclohex-2-enylmethyl]-dimethyl-amin,
*E*-3-[6-Dimethylaminomethyl-3-(4-fluor-benzyliden)-cyclohex-1-enyl]-phenol,

*Z*-3-[6-Dimethylaminomethyl-3-(4-fluor-benzyliden)-cyclohex-1-enyl]-phenol,
*E*-[4-(4-Chlor-benzyliden)-2-(3-methoxy-phenyl)-cyclohex-2-enylmethyl]-dimethyl-amin
*E*-3-[3-(4-Chlor-benzyliden)-6-dimethylaminomethyl-cyclohex-1-enyl]-phenol,
*Z*-3-[4-Dimethylaminomethyl-3-(3-hydroxy-phenyl)-cyclohex-2-enylidenmethyl]-benzoesäuremethylester,
*E*-3-[4-Dimethylaminomethyl-3-(3-hydroxy-phenyl)-cyclohex-2-enylidenmethyl]-benzoesäure,
*rac-cis-E*-3-[4-Dimethylaminomethyl-3-(3-hydroxy-phenyl)-cyclohexylidenmethyl]-benzoesäuremethylester,
*rac-cis-Z*-3-[4-Dimethylaminomethyl-3-(3-hydroxy-phenyl)-cyclohexylidenmethyl]-benzoesäuremethylester,
*rac-trans-Z*-3-[4-Dimethylaminomethyl-3-(3-hydroxy-phenyl)-cyclohexylidenmethyl]-benzoesäure,
*rac-trans-E*-[4-Dirnethylaminomethyl-3-(3-hydroxy-phenyl)-cyclohexylidenmethyl]-benzoesäure,
*rac-cis-Z*-3-[4-Dimethylaminomethyl-3-(3-hydroxy-phenyl)-cyclohexylidenmethyl]-benzoesäure,
rac-cis-Z-3-[4-Dimethylaminomethyl-3-hydroxy-3-(3-methoxy-phenyl)-cyclohexylidenemethyl]-benzoesäuremethylester,
rac-cis-E-3-[3-Chloro-4-dimethylaminomethyl-3-(3-methoxy-phenyl)-cyclohexylidenmethyl]-benzoesäuremethylester,
rac-cis-E-3-[4-Dimethylaminomethyl-3-hydroxy-3-(3-methoxy-phenyl)-cyclohexylidenmethyl]-benzoesäuremethylester,
rac-cis-Z-3-[4-Dimethylaminomethyl-3-hydroxy-3-(3-methoxy-phenyl)-cyclohexylidenemethyl]-benzoesäuremethylester,
(+)-trans-E-3-[4-Dimethylaminomethyl-3-(3-methoxy-phenyl)-cyclohexylidenmethyl]-benzoesäure,
(-)-trans-E-3-[4-Dimethylaminomethyl-3-(3-methoxy-phenyl)-cyclohexylidenmethyl]-benzoesäure,
rac-trans-E-3-(4-Dimethylaminomethyl-3-phenylcyclohexylidenemethyl)-benzoesäuremethylester,
rac-trans-Z-3-(4-Dimethylaminomethyl-3-phenyl-cyclohexylidenmethyl)-benzoesäuremethylester,
rac-cis-E-3-(4-Dimethylaminomethyl-3-phenyl-cyclohexylidenmethyl)-benzoesäure,
rac-cis-E-3-(4-Dimethylaminomethyl-3-phenyl-cyclohexylidenmethyl)-benzoesäure,
rac-trans-Z-3-(4-Dimethylaminomethyl-3-phenylcyclohexylidenmethyl)benzoesäure,
rac-trans-E-3-(4-Dimethylaminomethyl-3-phenylcyclohexylidenemethyl)-benzoesäure,
rac-trans-E-3-[4-Dimethylaminomethyl-3-(3-trifluoromethyl-phenyl)-cyclohexylidenmethyl]-benzoesäure,
rac-trans-Z-3-[4-Dimethylaminomethyl-3-(3-trifluoromethyl-phenyl)-cyclohexylidenmethyl]-benzoesäure,
rac-trans-E-3-[4-Dimethylaminomethyl-3-(3-fluoro-phenyl)-cyclohexylidenmethyl]-benzoesäuremethylester,
rac-trans-Z-3-[4-Dimethylaminomethyl-3-(3-fluoro-phenyl)-cyclohexylidenmethyl]benzoesäuremethylester,
rac-cis-E-3-[4-Dimethylaminomethyl-3-(3-fluoro-phenyl)-cyclohexylidenemethyl]-benzoesäuremethylester,
rac-trans-E-3-[4-Dimethylaminomethyl-3-(3-fluoro-phenyl)-cyclohexylidenemethyl]-benzoesäure,
rac-trans-Z-3-[4-Dimethylaminomethyl-3-(3-fluoro-phenyl)-cyclohexylidenemethyl]-benzoesäure,
rac-cis-E-3-[4-Dimethylaminomethyl-3-(3-fluoro-phenyl)-cyclohexylidenemethyl]-benzoesäure
E-[4-Ethyliden-2-(3-methoxy-phenyl)-cyclohex-2-enylmethyl]-dimethylamin
[4-Isopropyliden-2-(3-methoxy-phenyl)-cyclohex-2-enylmethyl]-dimethylamin,
E-[2-(3-Methoxy-phenyl)-4-propyliden-cyclohex-2-enylmethyl]-dimethylamin,
E-[4-Butyliden-2-(3-methoxy-phenyl)-cyclohex-2-enylmethyl]-dimethylaminals freie Base oder in Form eines mit einer physiologisch verträglichen Säure gebildeten Salzes, insbesondere des Hydrochloridsalzes.

**15.** Verwendung mindestens eines substituierten Aminomethyl-Phenyl-Cyclohexanderivats der allgemeinen Formel I bzw. Ia

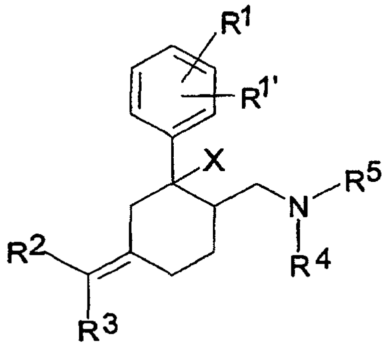

I

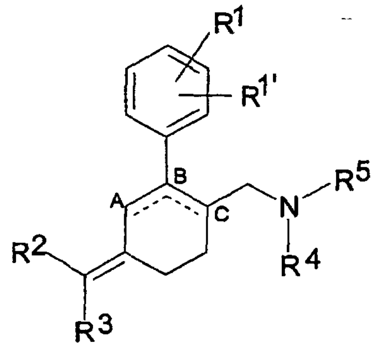

Ia

, in der $R^1$, $R^{1'}$, $R^2$, $R^3$, $R^4$, $R^5$ und X eine der in Anspruch 1 genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von Schmerz.

**16.** Verwendung mindestens eines substituierten Aminomethyl-Phenyl-Cyclohexanderivats der allgemeinen Formel I bzw. Ia

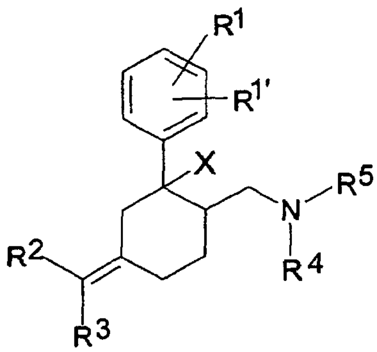

I

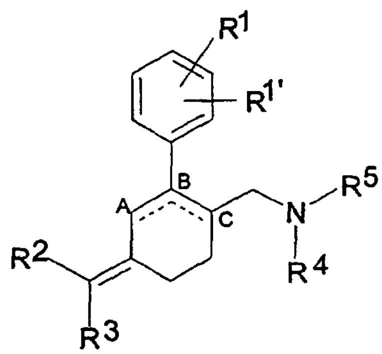

Ia

, in der $R^1$, $R^{1'}$, $R^2$, $R^3$, $R^4$, $R^5$ und X eine der in Anspruch 1 genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von Haminkontinenz, Juckreiz und/oder Diarrhoe.

**17.** Verwendung mindestens eines substituierten Aminomethyl-Phenyl-Cyclohexanderivats der allgemeinen Formel I bzw. Ia

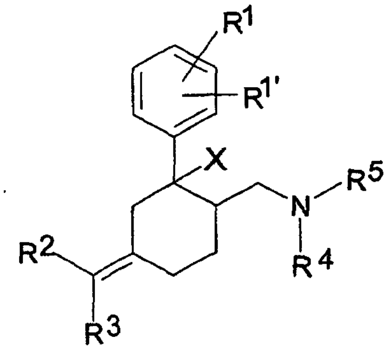

I

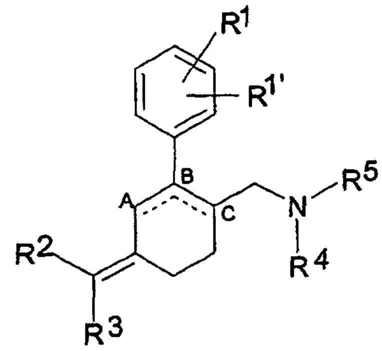

Ia

, in der $R^1$, $R^{1'}$, $R^2$, $R^3$, $R^4$, $R^5$ und X eine der in Anspruch 1 genannten Bedeutungen haben, in Form seiner Diastereomere oder Enantiomere sowie seiner freien Base oder eines mit einer physiologisch verträglichen Säure gebildeten Salzes, insbesondere des Hydrochloridsalzes, zur Herstellung eines Arzneimittels zur Behandlung von inflammatorischen und allergischen Reaktionen, Depressionen, Drogen- und/oder Alkoholmißbrauch, Gastritis, cardiovaskulären Erkrankungen, Atemwegserkrankungen, Husten, seelischen Erkrankungen und/oder Epilepsie.

## Claims

**1.** Substituted aminomethyl-phenyl-cyclohexane derivatives of the general formula I and Ia, also in the form of their diastereomers or enantiomers and of their free base or of a salt form with a physiologically tolerated acid, in particular the hydrochloride salt

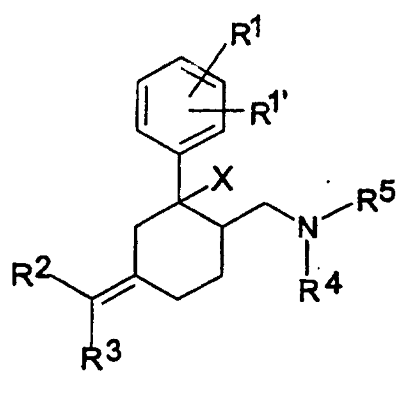

I

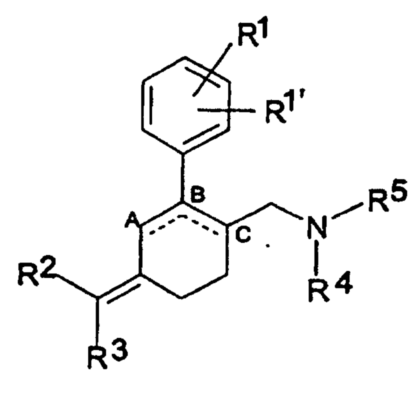

Ia

wherein

$R^1$ and $R^{1'}$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; F, Cl, Br, I, $NR^6$ $R^{6'}$, $NO_2$, CN, $OR^6$, $SR^6$, OC (O) $R^6$, C (O) $OR^6$, $C(O)R^6$ or $C(O)NR^6R^{6'}$, wherein $R^6$ and $R^{6'}$ are chosen from

H; $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^1$ and $R^{1'}$ together form -CH=CH-CH=CH-, wherein the naphthyl system formed can be mono- or polysubstituted,

X is chosen from

H, F, Cl, Br, I, $CF_3$, $OS(O_2)C_6H_4$-p$CH_3$, $OR^7$ or $OC(O)R^7$, wherein $R^7$ is chosen from

H; $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

if the compound contains no X, according to formula Ia a double bond is formed between C atom A and C atom B or C atom B and C atom C,

$R^4$, $R^5$ independently of one another are chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by N, S or O; alkylaryl, saturated or unsaturated and mono- or polysubstituted or unsubstituted; aryl or heteroaryl, in each case mono- or polysubstituted or unsubstituted;

or

$R^4$ and $R^5$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^8$, where $R^8$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

and

$R^2$, $R^3$ independently of one another are chosen from

$R^9$ or $YR^9$, where Y = $C_1$-$C_{10}$-alkyl, $C_2$-$C_{10}$-alkenyl or $C_2$-$C_{10}$-alkinyl, branched or unbranched and mono- or polysubstituted or unsubstituted, wherein $R^9$ is chosen from

H, F, Cl, Br, I, CN, $NO_2$, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{10}$, where $R^{10}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

$OR^{11}$, $OC(O)R^{11}$, $OC(O)OR^{11}$, $OC(S)R^{11}$, $C(O)R^{11}$, $C(O)OR^{11}$, $C(S)R^{11}$, $C(S)OR^{11}$, $SR^{11}$, $S(O)R^{11}$ or $S(O_2)R^{11}$, wherein $R^{11}$ is chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{12}$, where $R^{12}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

$NR^{13}R^{14}$, $NR^{13}C(O)R^{14}$, $C(O)NR^{13}R^{14}$ or $S(O_2)NR^{13}R^{14}$, wherein $R^{13}$ and $R^{14}$ independently of one another are chosen from

H, O; $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{15}$, where $R^{15}$ is chosen from

H, $C_1$-$C_{10}$-alkyl, $C_2$-$C_{10}$-alkenyl or $C_2$-$C_{10}$--alkinyl, in each case branched or unbranched and mono- or polysubstituted or unsubstituted;

alkylaryl, saturated or unsaturated and mono- or polysubstituted or unsubstituted; aryl or

heteroaryl, in each case mono- or polysubstituted or unsubstituted;

or

$R^{13}$ and $R^{14}$ together form a $C_3$-$C_7$-cycloalkyl, saturated or unsaturated and mono- or polysubstituted or unsubstituted, or a corresponding heterocyclic radical, in which one C atom in the ring is replaced by S, O or $NR^{16}$, where $R^{16}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted;

or

alkylaryl, aryl or heteroaryl, in each case mono- or polysubstituted or unsubstituted; in particular

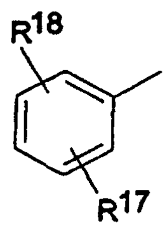

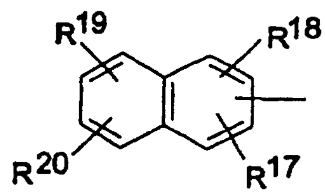

or

wherein $R^{17}$, $R^{18}$, $R^{19}$ and $R^{20}$ independently of one another are chosen from

$R^{21}$ or $ZR^{21}$, where Z = $C_1$-$C_{10}$-alkyl, $C_2$-$C_{10}$-alkenyl or $C_2$-$C_{10}$-alkinyl, in each case branched or unbranched and mono- or polysubstituted or unsubstituted, wherein $R^{21}$ is chosen from

H, F, Cl, Br, I, CN, $NO_2$; $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{22}$, where $R^{22}$ is chosen from

H, $C_1$-$C_{10}$-alkyl, $C_2$-$C_{10}$--alkenyl or $C_2$-$C_{10}$--alkinyl, in each case branched or unbranched and mono- or polysubstituted or unsubstituted;

alkylaryl, aryl or heteroaryl, in each case mono- or polysubstituted or unsubstituted;

$OR^{23}$, $OC(O)R^{23}$, $OC(O)OR^{23}$, $OC(S)R^{23}$, $C(O)R^{23}$, $C(O)OR^{23}$, $C(S)R^{23}$, $C(S)OR^{23}$, $SR^{23}$, $S(O)R^{23}$ or $S(O_2)R^{23}$, wherein $R^{23}$ is chosen from

H, $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{24}$, where $R^{24}$ is chosen from

H, $C_1$-$C_{10}$-alkyl, $C_2$-$C_{10}$--alkenyl or $C_2$-$C_{10}$--alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

$NR^{25}R^{26}$, $NR^{25}C(O)R^{26}$, $C(O)NR^{25}R^{26}$ or $S(O_2)NR^{25}R^{26}$, wherein $R^{25}$ and $R^{26}$ independently of one another are chosen from

H; $C_1$-$C_{18}$-alkyl, $C_2$-$C_{18}$-alkenyl or $C_2$-$C_{18}$-alkinyl, in each case branched or unbranched and mono- or polysubstituted or unsubstituted; $C_3$-$C_7$-cycloalkyl, saturated or unsaturated and mono- or polysubstituted or unsubstituted, or a corresponding heterocyclic radical, in which one C atom in the ring is replaced by S, O or $NR^{27}$, where $R^{27}$ is chosen from H, $C_1$-$C_{10}$-alkyl, $C_2$-$C_{10}$-alkenyl or $C_2$-$C_{10}$--alkinyl, in each case branched or unbranched and mono- or polysubstituted or unsubstituted;

alkylaryl, saturated or unsaturated and mono- or polysubstituted or unsubstituted; aryl or heteroaryl, in each case mono- or polysubstituted or unsubstituted;

or

$R^{25}$ and $R^{26}$ together form a $C_3$-$C_7$-cycloalkyl, saturated or unsaturated and mono- or polysubstituted or unsubstituted, or a corresponding heterocyclic radical in which one C atom in the ring is replaced by S, O or $NR^{28}$ [sic], where $R^{27}$ is chosen from

H, $C_1$-$C_{10}$-alkyl, $C_2$-$C_{10}$-alkenyl or $C_2$-$C_{10}$-alkinyl, in each case branched or unbranched and mono- or polysubstituted or unsubstituted.

2. Substituted aminomethyl-phenyl-cyclohexane derivatives according to claim 1, **characterized in that** $R^2$ and $R^3$ have a different meaning and/or $R^3$ = H or $CH_3$, preferably H, while $R^1$, $R^{1'}$, $R^2$, $R^4$, $R^5$ and X have one of the meanings mentioned in the preceding claims.

3. Substituted aminomethyl-phenyl-cyclohexane derivatives according to one of claims 1 or 2, **characterized in that** $R^2$ is chosen from

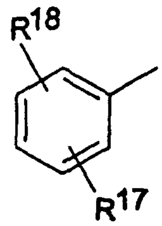

or

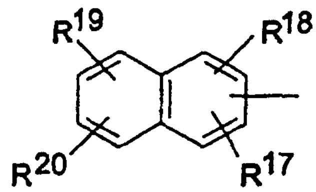

wherein $R^{17}$, $R^{18}$, $R^{19}$ and $R^{20}$ and $R^1$, $R^{1'}$, $R^3$, $R^4$, $R^5$ and X have one of the meanings mentioned in the preceding claims
or $R^2$ is chosen from $C_{1-3}$-alkyl.

4. Substituted aminomethyl-phenyl-cyclohexane derivatives according to one of claims 1-3, **characterized in that** $R^2$ is

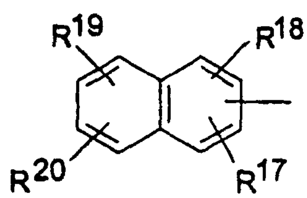

and $R^{19}$ and $R^{20}$ are H, while $R^1$, $R^{1'}$, $R^3$, $R^4$, $R^5$, X and $R^{17}$ and $R^{18}$ have one of the meanings mentioned in the preceding claims.

5. Substituted aminomethyl-phenyl-cyclohexane derivatives according to one of claims 1-4, **characterized in that** $R^2$ is chosen from

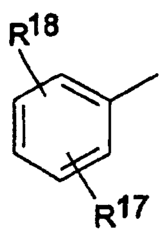

or

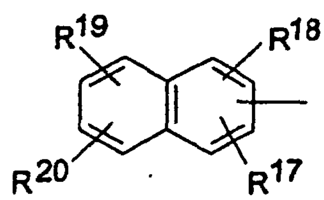

$R^{17}$ and $R^{18}$ have a different meaning and/or $R^{18}$ = $R^{21}$, where $R^{21}$ is chosen from
H, F, Cl, Br, I, $CF_3$ or $OR^{23}$, where $R^{23}$ = H, methyl ethyl, propyl, isopropyl, butyl or isobutyl,
while $R^1$ $R^{1'}$, $R^3$, $R^4$, $R^5$ and X and $R^{17}$, $R^{19}$ and $R^{20}$ have one of the meanings mentioned in the preceding claims.

6. Substituted aminomethyl-phenyl-cyclohexane derivatives according to one of claims 1-5, **characterized in that** $R^2$ is chosen from

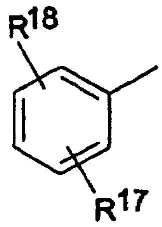

or

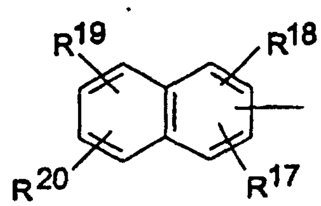

wherein $R^{17}$ is chosen from

$R^{21}$, wherein $R^{21}$ is chosen from

H, F, Cl, Br, I, $CF_3$, $OR^{23}$, $OC(O)R^{23}$, $C(O)R^{23}$ or $C(O)OR^{23}$, preferably H, F, Cl, $C(O)OR^{23}$, wherein $R^{23}$ is chosen from

H; $C_1$-$C_6$-alkyl, $C_2$-$C_8$-alkenyl or $C_2$-$C_8$-alkinyl, in particular $C_1$-$C_4$-alkyl, branched or unbranched and mono- or polysubstituted or unsubstituted; preferably H, methyl ethyl, propyl, isopropyl, butyl or isobutyl, in particular H, $CH_3$, $C_2H_5$ or isobutyl;

$C(O)NR^{25}R^{26}$, wherein $R^{25}$ and $R^{26}$ independently of one another are chosen from

H, O; $C_1$-$C_{18}$-alkyl, in particular $C_1$-$C_4$-alkyl, branched or unbranched, saturated or unsaturated and mono- or polysubstituted or unsubstituted, preferably H, methyl ethyl, propyl, isopropyl, butyl or isobutyl, in particular $C_2H_5$;

or

$ZR^{21}$, where Z = $CH_2$ or $C_2H_4$, wherein $R^{21}$ is chosen from

$OR^{23}$, $OC(O)R^{23}$, $C(O)R^{23}$ or $C(O)OR^{23}$, preferably $OR^{23}$, wherein $R^{23}$ is chosen from

H; $C_1$-$C_6$-alkyl, $C_2$-$C_8$-alkenyl or $C_2$-$C_8$-alkinyl, in particular $C_1$-$C_4$-alkyl, branched or unbranched and mono- or polysubstituted or unsubstituted, preferably H, methyl ethyl, propyl, isopropyl, butyl or isobutyl, in particular H;

$C(O)NR^{25}R^{26}$, wherein $R^{25}$ and $R^{26}$ independently of one another are chosen from

H, O, $C_1$-$C_{18}$-alkyl, in particular $C_1$-$C_4$-alkyl, branched or unbranched, saturated or unsaturated and mono- or polysubstituted or unsubstituted, preferably H, methyl ethyl, propyl, isopropyl, butyl or isobutyl; while $R^1$ $R^{1'}$, $R^3$ $R^4$ $R^5$ and X and $R^{18}$, $R^{19}$ and $R^{20}$ have one of the meanings mentioned in the preceding claims.

**7.** Substituted aminomethyl-phenyl-cyclohexane derivatives according to one of claims 1 to 6, **characterized in that**

$R^1$ is chosen from

H, F, Cl, Br, I, $CF_3$, $SCH_3$ or $OR^6$, preferably $OR^6$, wherein $R^6$ is chosen from

H; $C_1$-$C_4$-alkyl, branched or unbranched and mono- or polysubstituted or unsubstituted; preferably H or $CH_3$;

and/or

$R^{1'}$ is chosen from

H, F, Cl, $SCH_3$ or $OCH_3$, preferably H

and/or

X is chosen from

H, F, Br, I, Cl or $OR^7$, preferably H, F, Cl or $OR^7$, where $R^7$ = H or $CH_3$, preferably H;

or

if the compound contains no X, according to formula Ia a double bond is formed between C atom A and C atom B or C atom B and C atom C;

and/or

$R^4$ and $R^5$ independently of one another are chosen from $C_1$-$C_4$-alkyl, branched or unbranched and mono- or polysubstituted or unsubstituted, preferably $CH_3$,

while $R^2$ and $R^3$ have one of the meanings mentioned in the preceding claims.

**8.** Substituted aminomethyl-phenyl-cyclohexane derivatives according to one of claims 1 to 5, **characterized in that**

they are

*rac-cis-E*-(4-benzylidene-2-(3-methoxy-phenyl)-cyclohexylmethyl]-dimethylamine,
*rac-trans-E*-[4-benzylidene-2-(3-methoxy-phenyl)-cyclohexylmethyl]-dimethylamine,
*rac-trans-Z*-[4-benzylidene-2-(3-methoxy-phenyl)-cyclohexylmethyl]-dimethylamine,
*rac-cis-Z*-[9-benzylidene-2-(3-methoxy-phenyl)-cyclohexylmethyl]-dimethylamine,
*rac-cis-E*-3-[4-dimethylaminomethyl-3-(3-methoxyphenyl)-cyclohexylidenemethyl]-benzoic acid methyl ester,
*rac-cis-Z*-3-[4-dimethylaminomethyl-3-(3-methoxyphenyl)-cyclohexylidenemethyl]-benzoic acid methyl ester,
*rac-trans-Z*-3-[4-dimethylaminomethyl-3-(3-methoxyphenyl)-cyclohexylidenemethyl]-benzoic acid methyl ester,
*rac-trans-E*-3-[4-dimethylaminomethyl-3-(3-methoxyphenyl)-cyclohexylidenemethyl]-benzoic acid methyl ester,
*Z*-3-[4-dimethylaminomethyl-3-(3-methoxy-phenyl)-2-methyl-cyclohex-2-enylidenemethyl]-benzoic acid methyl ester,
*E*-3-[4-dimethylaminomethyl-3-(3-methoxy-phenyl)-2-methyl-cyclohex-2-enylidenemethyl]-benzoic acid methyl ester,
*Z*-3-[4-dimethylaminomethyl-3-(3-methoxy-phenyl)-2-methyl-cyclohex-2-enylidenemethyl]-naphthalene-1-carboxylic acid ethyl ester,
*E*-3-[4-dimethylaminomethyl-3-(3-methoxy-phenyl)-2-methyl-cyclohex-2-enylidenemethyl]-naphthalene-1-carboxylic acid ethyl ester,
*Z*-3-[4-dimethylaminomethyl-3-(3-methoxy-phenyl)-cyclohexylidenemethyl]-2-fluoro-benzoic acid ethyl ester,
*Z*-3-[4-dimethylaminomethyl-3-(3-methoxy-phenyl)cyclohex-2-enylidenemethyl]-benzoic acid,
*E*-3-[4-dimethylaminomethyl-3-(3-methoxy-phenyl)-cyclohex-2-enylidenemethyl]-benzoic acid,
*E*-{3-[4-dimethylaminomethyl-3-(3-methoxy-phenyl)-cyclohex-2-enylidenemethyl]-phenyl}-methanol,
*rac-trans-Z*-3-[4-dimethylaminomethyl-3-(3-methoxyphenyl)-cyclohexylidenemethyl]-benzoic acid,
*rac-trans-E*-3-(4-dimethylaminomethyl-3-(3-methoxyphenyl)-cyclohexylidenemethyl]-benzoic acid,
*rac-trans-E*-[3-(2-dimethylaminomethyl-5-(3-hydroxymethyl-benzylidene)-cyclohexyl)-phenol,
*rac-trans-E*-3-(5-benzylidene-2-dimethylaminomethylcyclohexyl)-phenol,
*E*-3-[4-dimethylaminomethyl-3-(3-methoxy-phenyl)-cyclohex-2-enylidenemethyl]-benzoic acid tert-butyl ester,
*E*-3-[6-dimethylaminomethyl-3-(3-hydroxymethylbenzylidene)-cyclohex-1-enyl]-phenol,
*rac-trans-Z*-3-(5-benzylidene-2-dimethylaminomethylcyclohexyl)-phenol,
*Z*-3-[2-dimethylaminomethyl-5-(3-hydroxymethylbenzylidene)-cyclohex-1-enyl)-phenol,
*Z*-{3-[4-dimethylaminomethyl-3-(3-methoxy-phenyl)-cyclohex-2-enylidenemethyl]-phenyl}-methanol,
*rac-cis-E*-4-dimethylaminomethyl-3-(3-methoxy-phenyl)-cyclohexylidenemethyl]-benzoic [sic] acid,
*rac-cis-Z*-3-[4-dimethylaminomethyl-3-(3-methoxyphenyl)-cyclohexylidenemethyl)-benzoic acid,
*rac-trans-E*-3-[4-dimethylaminomethyl-3-(3-methoxyphenyl)-cyclohexylidenemethyl]-benzoic acid tert-butyl ester,
*rac-trans-E-[*3-[4-dimethylaminomethyl-3-(3-methoxyphenyl)-cyclohexylidenemethyl)-phenyl)-methanol],
*rac-cis-Z*-3-[4-dimethylaminomethyl-3-(3-methoxyphenyl)-cyclohexylidenemethyl]-benzoic acid ethyl ester,
*E*-3-(4-dimethylaminomethyl-3-(3-methoxy-phenyl)-cyclohex-2-enylidenemethyl]-N,N-diethyl-benzamide
*rac-trans-E*-3-[4-dimethylaminomethyl-3-(3-methoxyphenyl)-cyclohex-2-enylidenemethyl]-N,N-diethylbenzamide,
*E*-3-[4-dimethylaminomethyl-3-(3-methoxy-phenyl)-cyclohex-2-enylidenemethyl]-benzoic acid ethyl ester
*Z*-3-[4-dimethylaminomethyl-3-(3-methoxy-phenyl)-cyclohex-2-enylidenemethyl]-N,N-diethyl-benzamide,
*rac-cis-E*-3-[4-dimethylaminomethyl-3-(3-methoxyphenyl)-cyclohexylidenemethyl]-N,N-diethyl-benzamide,
*rac-trans-Z*-3-[4-dimethylaminomethyl-3-(3-methoxyphenyl)-cyclohexylidenemethyl]-N,N-diethyl-benzamide,
*E*-3-(4-dimethylaminomethyl-3-(3-hydroxy-phenyl)-cyclohex-2-enylidenemethyl]-benzoic acid methyl ester,
*Z*-3-[4-dimethylaminomethyl-3-(3-hydroxy-phenyl)-cyclohex-2-enylidenemethyl]-benzoic acid methyl ester,
*Z*-3-[4-dimethylaminomethyl-3-(3-methoxy-phenyl)-cyclohex-2-enylidenemethyl]-benzoic acid isobutyl ester,
*Z*-3-[6-dimethylaminomethyl-3-(3-hydroxymethylbenzylidene)-cyclohex-1-enyl]-phenol,
*Z*-[4-(4-chloro-benzylidene)-2-(3-methoxy-phenyl)-cyclohex-2-enylmethyl]-dimethyl-amine,
*E*-[4-(4-fluoro-benzylidene)-2-(3-methoxy-phenyl)-cyclohex-2-enylmethyl]-dimethyl-amine,
*Z*-[4-(4-fluoro-benzylidene)-2-(3-methoxy-phenyl)-cyclohex-2-enylmethyl]-dimethyl-amine,
*E*-3-[6-dimethylaminomethyl-3-(4-fluoro-benzylidene)-cyclohex-1-enyl]-phenol,
*Z*-3-[6-dimethylaminomethyl-3-(4-fluoro-benzylidene)-cyclohex-1-enyl]-phenol,
*E*-[4-(4-chloro-benzylidene)-2-(3-methoxy-phenyl)-cyclohex-2-enylmethyl]-dimethyl-amine,
*E*-3-[3-(4-chloro-benzylidene)-6-dimethylaminomethylcyclohex-1-enyl]-phenol,

*Z*-3-[4-dimethylaminomethyl-3-(3-hydroxy-phenyl)-cyclohex-2-enylidenemethyl]-benzoic acid methyl ester,
*E*-3-[4-dimethylaminomethyl-3-(3-hydroxy-phenyl)-cyclohex-2-enylidenemethyl]-benzoic acid,
*rac-cis-E*-3-[4-dimethylaminomethyl-3-(3-hydroxyphenyl)-cyclohexylidenemethyl]-benzoic acid methyl ester,
*rac-cis-Z*-3-[4-dimethylaminomethyl-3-(3-hydroxyphenyl)-cyclohexylidenemethyl]-benzoic acid methyl ester,
*rac-trans-Z*-3-[4-dimethylaminomethyl-3-(3-hydroxyphenyl)-cyclohexylidenemethyl]-benzoic acid,
*rac-trans-E*--[4-dimethylaminomethyl-3-(3-hydroxyphenyl)-cyclohexylidenemethyl]-benzoic [sic] acid,
*rac-cis-Z*-3-[4-dimethylaminomethyl-3-(3-hydroxyphenyl)-cyclohexylidenemethyl]-benzoic acid,
rac-cis-Z-3-[4-dimethylaminomethyl-3-hydroxy-3-(3-methoxy-phenyl)-cyclohexylidenemethyl]-benzoic acid methyl ester,
rac-cis-E-3-[3-chloro-4-dimethylaminomethyl-3-(3-methoxy-phenyl)-cyclohexylidenemethyl]-benzoic acid methyl ester,
rac-cis-E-3-[4-dimethylaminomethyl-3-hydroxy-3-(3-methoxy-phenyl)-cyclohexylidenemethyl]-benzoic acid methyl ester,
rac-cis-Z-3-[4-dimethylaminomethyl-3-hydroxy-3-(3-methoxy-phenyl)-cyclohexylidenemethyl)-benzoic acid methyl ester,
(+)-trans-E-3-[4-dimethylaminomethyl-3-(3-methoxyphenyl)-cyclohexylidenemethyl)-benzoic acid,
(-)-trans-E-3-(4-dimethylaminomethyl-3-(3-methoxyphenyl)-cyclohexylidenemethyl]-benzoic acid,
rac-trans-E-3-(4-dimethylaminomethyl-3-phenylcyclohexylidenemethyl)-benzoic acid methyl ester,
rac-trans-Z-3-(4-dimethylaminomethyl-3-phenylcyclohexylidenemethyl)-benzoic acid methyl ester,
rac-cis-E-3-(4-dimethylaminomethyl-3-phenylcyclohexylidenemethyl)-benzoic acid,
rac-cis-E-3-(4-dimethylaminomethyl-3-phenylcyclohexylidenemethyl)-benzoic acid,
rac-trans-Z-3-(4-dimethylaminomethyl-3-phenylcyclohexylidenemethyl)benzoic acid,
rac-trans-E-3-(4-dimethylaminomethyl-3-phenylcyclohexylidenemethyl)-benzoic acid,
rac-trans-E-3-[4-dimethylaminomethyl-3-(3-triflluoromethyl-phenyl)-cyclohexylidenemethyl]-benzoic acid,
rac-trans-Z-3-[4-dimethylaminomethyl-3-(3-trifluoromethyl-phenyl)-cyclohexylidenemethyl]-benzoic acid,
rac-trans-E-3-[4-dimethylaminomethyl-3-(3-fluorophenyl)-cyclohexylidenemethyl]-benzoic acid methyl ester,
rac-trans-Z-3-[4-dimethylaminomethyl-3-(3-fluorophenyl)-cyclohexylidenemethyl)benzoic acid methyl ester,
rac-cis-E-3-[4-dimethylaminomethyl-3-(3-fluorophenyl)-cyclohexylidenemethyl]-benzoic acid methyl ester,
rac-trans-E-3-[4-dimethylaminomethyl-3-(3-fluorophenyl)-cyclohexylidenemethyl]-benzoic acid,
rac-trans-Z-3-[4-dimethylaminomethyl-3-(3-fluorophenyl)-cyclohexylidenemethyl)-benzoic acid,
rac-cis-E-3-[4-dimethylaminomethyl-3-(3-fluorophenyl)-cyclohexylidenemethyl]-benzoic acid
E-[4-ethylidene-2-(3-methoxy-phenyl)-cyclohex-2-enylmethyl]-dimethylamine
[4-isopropylidene-2-(3-methoxy-phenyl)-cyclohex-2-enylmethyl]-dimethylamine,
E-[2-(3-methoxy-phenyl)-4-propylidene-cyclohex-2-enylmethyl]-dimethylamine,
E-[4-butylidene-2-(3-methoxy-phenyl)-cyclohex-2-enylmethyl]-dimethylamine

and salts thereof, in particular hydrochloride salts.

**9.** Process for the preparation of substituted aminomethyl-phenyl-cyclohexane derivatives of the general formula I or Ia

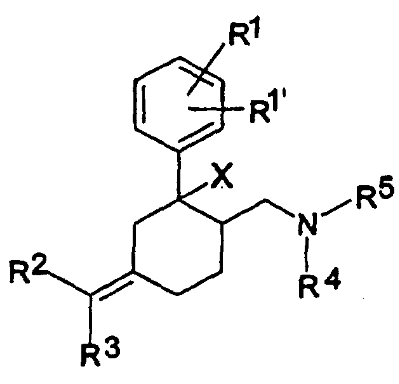

I

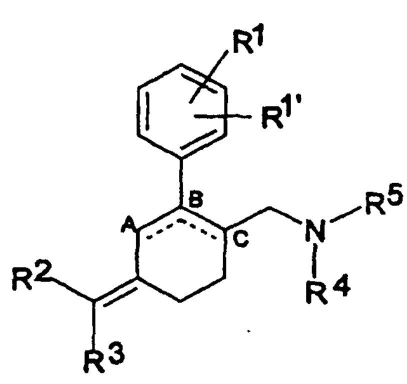

Ia

in which $R^1$, $R^{1'}$, $R^2$, $R^3$, $R^4$, $R^5$ and X have one of the meanings mentioned in claim 1, **characterized in that** cyclohexanones of the formula II or IIa

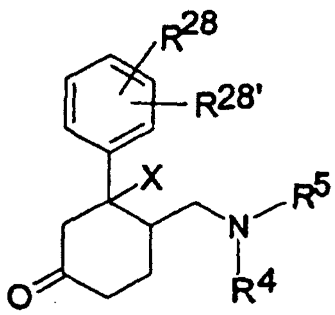

II

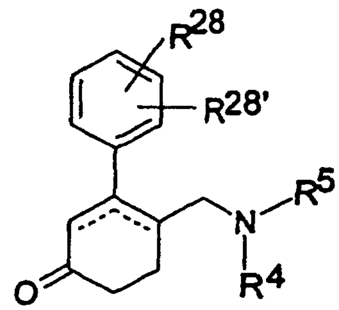

IIa

in which $R^4$, $R^5$ and X have one of the meanings mentioned in claim 1 and $R^{28}$ has one of the meanings mentioned in claim 1 for $R^1$ and $R^{28'}$ has one of the meanings mentioned in claim 1 for $R^{1'}$,
are reacted in a Wittig reaction in an organic solvent in the presence of a base with alkyltriphenylphosphonium salts of the formula III

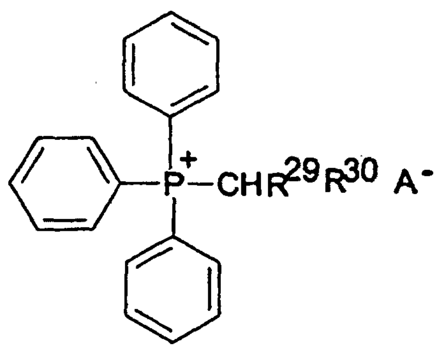

III

wherein A denotes chloride or bromide and, independently of one another, $R^{29}$ has one of the meanings mentioned for $R^2$ and $R^{30}$ has one of the meanings mentioned for $R^3$ in claim 1.

**10.** Process according to claim 9, **characterized in that** the organic solvent is benzene, toluene or a chlorinated hydrocarbon and/or potassium tert-butylate or sodium hydride are used as the base and/or the reaction temperature, in particular during the Wittig reaction, is kept between 50°C and 90°C.

**11.** Process according to one of claims 9 or 10, **characterized in that** in $R^{28}$ and/or $R^{28'}$ according to formula II or IIa and/or $R^{29}$ and/or $R^{30}$ according to formula III at least one OH group has been replaced by an $OSi(Ph)_2$tert-but group, at least one SH group has been replaced by an S-p-methoxybenzyl group and/or at least one $NH_2$ group has been replaced by an $NO_2$ group, and, after conclusion of the Wittig reaction, at least one OSi $(Ph)_2$ tert-but group is split off with tetrabutylammonium fluoride in tetrahydrofuran and/or at least one p-methoxybenzyl group is split off with a metal amine, preferably sodium amine, and/or at least one $NO_2$ group is reduced to $NH_2$.

**12.** Process according to one of claims 9 - 11, **characterized in that** after the Wittig reaction a process product with at least one $C(O)OCH_3$ and/or $C(S)OCH_3$ group is hydrolysed with KOH solution or NaOH solution in methanol at 40°C - 60°C.

13. Medicaments comprising as the active compound at least one substituted aminomethyl-phenyl-cyclohexane derivative of the general formula I or Ia

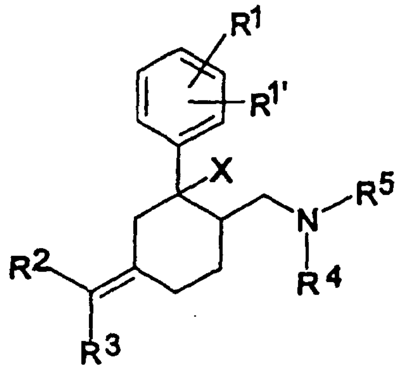

I

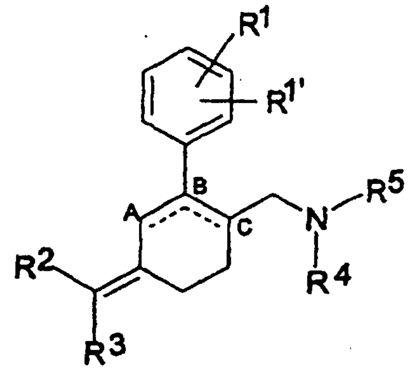

Ia

in which $R^1$, $R^{1'}$, $R^2$, $R^3$, $R^4$, $R^5$ and X have one of the meanings mentioned in claim 1, in the form of its diastereomers or enantiomers and of its free base or of a salt formed with a physiologically tolerated acid, in particular the hydrochloride salt.

14. Medicaments according to claim 13, comprising as the active compound at least one substituted aminomethyl-phenyl-cyclohexane derivative chosen from the group consisting of

*rac-cis-E*-[4-benzylidene-2-(3-methoxy-phenyl)-cyclohexylmethyl]-dimethylamine,
*rac-trans-E*-[4-benzylidene-2-(3-methoxy-phenyl)-cyclohexylmethyl]-dimethylamine,
*rac-trans-Z*-[4-benzylidene-2-(3-methoxy-phenyl)-cyclohexylmethyl]-dimethylamine,
*rac-cis-Z*-[4-benzylidene-2-(3-methoxy-phenyl)-cyclohexylmethyl]-dimethylamine,
*rac-cis-E*-3-[4-dimethylaminomethyl-3-(3-methoxyphenyl)-cyclohexylidenemethyl]-benzoic acid methyl ester,
*rac-cis-Z*-3-[4-dimethylaminomethyl-3-(3-methoxyphenyl)-cyclohexylidenemethyl]-benzoic acid methyl ester,
*rac-trans*-Z-3-[4-dimethylaminomethyl-3-(3-methoxyphenyl)-cyclohexylidenemethyl]-benzoic acid methyl ester,
*rac-trans-E*-3-[4-dimethylaminomethyl-3-(3-methoxyphenyl)-cyclohexylidenemethyl]-benzoic acid methyl ester,
*Z*-3-[4-dimethylaminomethyl-3-(3-methoxy-phenyl)-2-methyl-cyclohex-2-enylidenemethyl]-benzoic acid methyl ester,
*E*-3-[4-dimethylaminomethyl-3-(3-methoxy-phenyl)-2-methyl-cyclohex-2-enylidenemethyl]-benzoic acid methyl ester,
*Z*-3-[4-dimethylaminomethyl-3-(3-methoxy-phenyl)-2-methyl-cyclohex-2-enylidenemethyl]-naphthalene-1-carboxylic acid ethyl ester,
*E*-3-(4-dimethylaminomethyl-3-(3-methoxy-phenyl)-2-methyl-cyclohex-2-enylidenemethyl)-naphthalene-1-carboxylic acid ethyl ester,
*Z*-3-[4-dimethylaminomethyl-3-(3-methoxy-phenyl)-cyclohexylidenemethyl]-2-fluoro-benzoic acid ethyl ester,
*Z*-3-[4-dimethylaminomethyl-3-(3-methoxy-phenyl)-cyclohex-2-enylidenemethyl)-benzoic acid,
*E*-3-[4-dimethylaminomethyl-3-(3-methoxy-phenyl)-cyclohex-2-enylidenemethyl]-benzoic acid,
*E*-(3-[4-dimethylaminomethyl-3-(3-methoxy-phenyl)-cyclohex-2-enylidenemethyl]-phenyl}-methanol,
*rac-trans-Z*-3-[4-dimethylaminomethyl-3-(3-methoxyphenyl)-cyclohexylidenemethyl]-benzoic acid,
*rac-trans-E*-3-[4-dimethylaminomethyl-3-(3-methoxyphenyl)-cyclohexylidenemethyl]-benzoic acid,
*rac-trans-E*-[3-(2-dimethylaminomethyl-5-(3-hydroxymethyl-benzylidene)-cyclohexyl)-phenol,
rac-trans-E-3-(5-benzylidene-2-dimethylaminomethylcyclohexyl)-phenol,
*E*-3-[4-dimethylaminomethyl-3-(3-methoxy-phenyl)-cyclohex-2-enylidenemethyl]-benzoic acid tert-butyl ester,
*E*-3-[6-dimethylaminomethyl-3-(3-hydroxymethylbenzylidene)-cyclohex-1-enyl]-phenol,
*rac-trans-Z*-3-(5-benzylidene-2-dimethylaminomethylcyclohexyl)-phenol,

*Z*-3-[2-dimethylaminomethyl-5-(3-hydroxymethylbenzylidene)-cyclohex-1-enyl]-phenol,
*Z*-(3-(4-dimethylaminomethyl-3-(3-methoxy-phenyl)-cyclohex-2-enylidenemethyl]-phenyl}-methanol,
*rac-cis-E*-4-dimethylaminomethyl-3-(3-methoxy-phenyl)-cyclohexylidenemethyl)-benzoic acid,
*rac-cis-Z*-3-[4-dimethylaminomethyl-3-(3-methoxyphenyl)-cyclohexylidenemethyl]-benzoic acid,
*rac-trans-E*-3-[4-dimethylaminomethyl-3-(3-methoxyphenyl)-cyclohexylidenemethyl]-benzoic acid tert-butyl ester,
*rac-trans-E-[*3-[4-dimethylaminomethyl-3-(3-methoxyphenyl)-cyclohexylidenemethyl)-phenyl)-methanol],
*rac-cis-Z*-3-[4-dimethylaminomethyl-3-(3-methoxyphenyl)-cyclohexylidenemethyl]-benzoic acid ethyl ester,
*E*-3-[4-dimethylaminomethyl-3-(3-methoxy-phenyl)-cyclohex-2-enylidenemethyl]-N,N-diethyl-benzamide
*rac-trans-E*-3-[4-dimethylaminomethyl-3-(3-methoxyphenyl)-cyclohex-2-enylidenemethyl]-N,N-diethylbenzamide,
*E*-3-(4-dimethylaminomethyl-3-(3-methoxy-phenyl)-cyclohex-2-enylidenemethyl]-benzoic acid ethyl ester
*Z*-3-[4-dimethylaminomethyl-3-(3-methoxy-phenyl)-cyclohex-2-enylidenemethyl]-N,N-diethyl-benzamide,
*rac-cis-E*-3-[4-dimethylaminomethyl-3-(3-methoxyphenyl)-cyclohexylidenemethyl]-N,N-diethyl-benzamide,
*rac-trans-Z*-3-[4-dimethylaminomethyl-3-(3-methoxyphenyl)-cyclohexylidenemethyl]-N,N-diethyl-benzamide,
*E*-3-[4-dimethylaminomethyl-3-(3-hydroxy-phenyl)-cyclohex-2-enylidenemethyl]-benzoic acid methyl ester,
*Z*-3-[4-dimethylaminomethyl-3-(3-hydroxy-phenyl)-cyclohex-2-enylidenemethyl]-benzoic acid methyl ester,
*Z*-3-[4-dimethylaminomethyl-3-(3-methoxy-phenyl)-cyclohex-2-enylidenemethyl]-benzoic acid isobutyl ester,
*Z*-3-[6-dimethylaminomethyl-3-(3-hydroxymethylbenzylidene)-cyclohex-1-enyl]-phenol,
*Z*-[4-(4-chloro-benzylidene)-2-(3-methoxy-phenyl)-cyclohex-2-enylmethyl]-dimethyl-amine,
*E*-[4-(4-fluoro-benzylidene)-2-(3-methoxy-phenyl)-cyclohex-2-enylmethyl]-dimethyl-amine,
*Z*-[4-(4-fluoro-benzylidene)-2-(3-methoxy-phenyl)-cyclohex-2-enylmethyl]-dimethyl-amine,
*E*-3-[6-dimethylaminomethyl-3-(4-fluoro-benzylidene)-cyclohex-1-enyl]-phenol,
*Z*-3-[6-dimethylaminomethyl-3-(4-fluoro-benzylidene)-cyclohex-1-enyl]-phenol,
*E*-[4-(4-chloro-benzylidene)-2-(3-methoxy-phenyl)-cyclohex-2-enylmethyl]-dimethyl-amine,
*E*-3-[3-(4-chloro-benzylidene)-6-dimethylaminomethylcyclohex-1-enyl]-phenol,
*Z*-3-[4-dimethylaminomethyl-3-(3-hydroxy-phenyl)-cyclohex-2-enylidenemethyl)-benzoic acid methyl ester,
*E*-3-[4-dimethylaminomethyl-3-(3-hydroxy-phenyl)-cyclohex-2-enylidenemethyl]-benzoic acid,
*rac-cis-E*-3-[4-dimethylaminomethyl-3-(3-hydroxyphenyl)-cyclohexylidenemethyl)-benzoic acid methyl ester,
*rac-cis-Z*-3-[4-dimethylaminomethyl-3-(3-hydroxyphenyl)-cyclohexylidenemethyl)-benzoic acid methyl ester,
*rac-trans-Z*-3-[4-dimethylaminomethyl-3-(3-hydroxyphenyl)-cyclohexylidenemethyl]-benzoic acid,
*rac-trans-E*--[4-dimethylaminomethyl-3-(3-hydroxyphenyl)-cyclohexylidenemethyl]-benzoic [sic] acid,
rac-cis-Z-3-[4-dimethylaminomethyl-3-(3-hydroxyphenyl)-cyclohexylidenemethyl)-benzoic acid,
rac-cis-Z-3-[4-dimethylaminomethyl-3-hydroxy-3-(3-methoxy-phenyl)-cyclohexylidenemethyl]-benzoic acid methyl ester,
rac-cis-E-3-[3-chloro-4-dimethylaminomethyl-3-(3-methoxy-phenyl)-cyclohexylidenemethyl)-benzoic acid methyl ester,
rac-cis-E-3-[4-dimethylaminomethyl-3-hydroxy-3-(3-methoxy-phenyl)-cyclohexylidenemethyl]-benzoic acid methyl ester,
rac-cis-Z-3-[4-dimethylaminomethyl-3-hydroxy-3-(3-methoxy-phenyl)-cyclohexylidenemethyl]-benzoic acid methyl ester,
(+)-trans-E-3-[4-dimethylaminomethyl-3-(3-methoxyphenyl)-cyclohexylidenemethyl]-benzoic acid,
(-)-trans-E-3-[4-dimethylaminomethyl-3-(3-methoxyphenyl)-cyclohexylidenemethyl)-benzoic acid,
rac-trans-E-3-(4-dimethylaminomethyl-3-phenylcyclohexylidenemethyl)-benzoic acid methyl ester,
rac-trans-Z-3-(4-dimethylaminomethyl-3-phenylcyclohexylidenemethyl)-benzoic acid methyl ester,
rac-cis-E-3-(4-dimethylaminomethyl-3-phenylcyclohexylidenemethyl)-benzoic acid,
rac-cis-E-3-(4-dimethylaminomethyl-3-phenylcyclohexylidenemethyl)-benzoic acid,
rac-trans-Z-3-(4-dimethylaminomethyl-3-phenylcyclohexylidenemethyl)benzoic acid,
rac-trans-E-3-(4-dimethylaminomethyl-3-phenylcyclohexylidenemethyl)-benzoic acid,
rac-trans-E-3-[4-dimethylaminomethyl-3-(3-triflluoromethyl-phenyl)-cyclohexylidenemethyl]-benzoic acid,
rac-trans-Z-3-[4-dimethylaminomethyl-3-(3-trifluoromethyl-phenyl)-cyclohexylidenemethyl]-benzoic acid,
rac-trans-E-3-[4-dimethylaminomethyl-3-(3-fluorophenyl)-cyclohexylidenemethyl]-benzoic acid methyl ester,
rac-trans-Z-3-[4-dimethylaminomethyl-3-(3-fluorophenyl)-cyclohexylidenemethyl]benzoic acid methyl ester,
rac-cis-E-3-[4-dimethylaminomethyl-3-(3-fluorophenyl)-cyclohexylidenemethyl]-benzoic acid methyl ester,
rac-trans-E-3-[4-dimethylaminomethyl-3-(3-fluorophenyl)-cyclohexylidenemethyl]-benzoic acid,
rac-trans-Z-3-[4-dimethylaminomethyl-3-(3-fluorophenyl)-cyclohexylidenemethyl]-benzoic acid,
rac-cis-E-3-[4-dimethylaminomethyl-3-(3-fluorophenyl)-cyclohexylidenemethyl]-benzoic acid
E-[4-ethylidene-2-(3-methoxy-phenyl)-cyclohex-2-enylmethyl]-dimethylamine

[4-isopropylidene-2-(3-methoxy-phenyl)-cyclohex-2-enylmethyl)-dimethylamine,
E-[2-(3-methoxy-phenyl)-4-propylidene-cyclohex-2-enylmethyl]-dimethylamine,
E-[4-butylidene-2-(3-methoxy-phenyl)-cyclohex-2-enylmethyl]-dimethylamineas the free base or in the form of a salt formed with a physiologically tolerated acid, in particular the hydrochloride salt.

**15.** Use of at least one substituted aminomethyl-phenyl-cyclohexane derivative of the general formula I or Ia

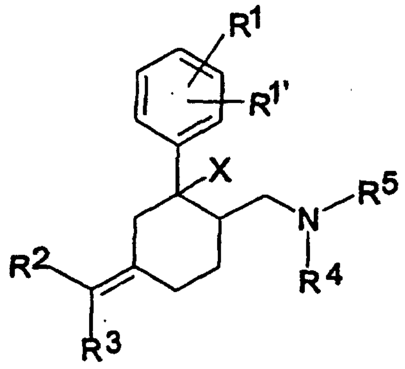

I

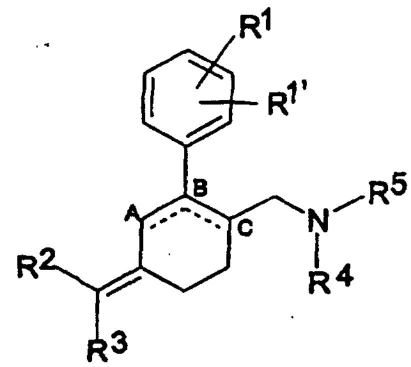

Ia

in which $R^1$, $R^{1'}$, $R^2$, $R^3$, $R^4$, $R^5$ and X have one of the meanings mentioned in claim 1, in the form of its diastereomers or enantiomers and of its free base or of a salt formed with a physiologically tolerated acid, in particular the hydrochloride salt, for the preparation of a medicament for treatment of pain.

**16.** Use of at least one substituted aminomethyl-phenyl-cyclohexane derivative of the general formula I or Ia

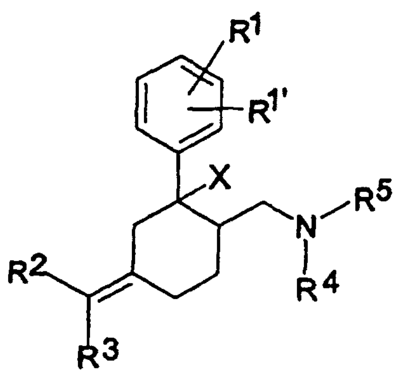

I

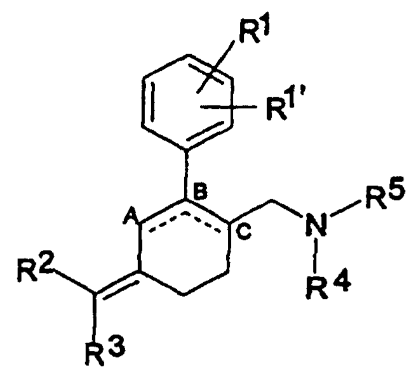

Ia

in which $R^1$, $R^{1'}$, $R^2$, $R^3$, $R^4$, $R^5$ and X have one of the meanings mentioned in claim 1, in the form of its diastereomers or enantiomers and of its free base or of a salt formed with a physiologically tolerated acid, in particular the hydrochloride salt, for the preparation of a medicament for treatment of urinary incontinence, itching and/or diarrhoea.

**17.** Use of at least one substituted aminomethyl-phenyl-cyclohexane derivative of the general formula I or Ia

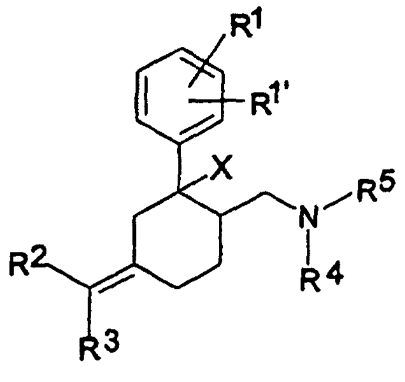

I

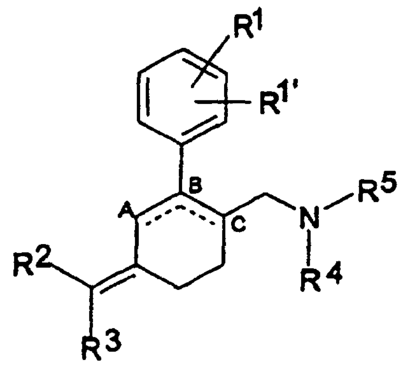

Ia

in which $R^1$, $R^{1'}$, $R^2$, $R^3$, $R^4$, $R^5$ and X have one of the meanings mentioned in claim 1, in the form of its diastereomers or enantiomers and of its free base or of a salt formed with a physiologically tolerated acid, in particular the hydrochloride salt, for the preparation of a medicament for treatment of inflammatory and allergic reactions, depression, drug and/or alcohol abuse, gastritis, cardiovascular diseases, respiratory tract diseases, coughing, mental illnesses and/or epilepsy.

## Revendications

**1.** Dérivés substitués d'aminométhyl-phénylcyclohexane de formule générale I ou Ia, également sous forme de leurs diastéréoisomères ou de leurs énantiomères ainsi que de leurs bases libres ou d'un sel formé avec un acide acceptable du point de vue physiologique, notamment sous forme du chlorhydrate,

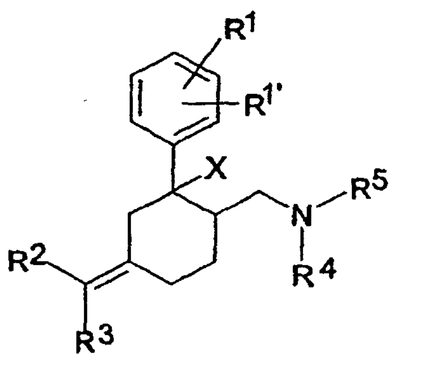

I

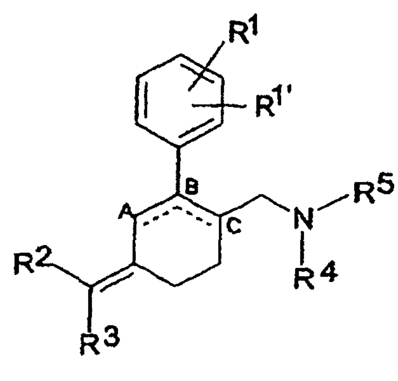

Ia

formule dans laquelle

$R^1$ et $R^{1'}$ sont choisis, indépendamment l'un de l'autre, entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; F, Cl, Br, I, $NR^6R^{6'}$, $NO_2$, CN, $OR^6$, $SR^6$, $OC(O)R^6$, $C(O)OR^6$, $C(O)R^6$ ou $C(O)NR^6R^{6'}$, où $R^6$ et $R^{6'}$ sont choisis entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O; alkylaryle, saturé ou non saturé, substitué une ou plusieurs fois ou non substitué ; aryle ou hétéroaryle, chacun substitué une ou plusieurs fois ou non substitué ;

Ou bien,

$R^1$ et $R^{1'}$ forment ensemble un groupe -CH=CH-CH=CH-, le système naphtyle formé pouvant être substitué une ou plusieurs fois,

X est choisi entre

H, F, Cl, Br, I, $CF_3$, $OS(O_2)C_6H_4$-$PCH_3$, $OR^7$ ou $OC(O)R^7$, où $R^7$ est choisi entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou,

lorsque le composé ne présente pas de substituant X, une double liaison est formée entre l'atome de carbone A et l'atome de carbone B ou entre l'atome de carbone B et l'atome de carbone C selon la formule Ia,

$R^4$, $R^5$ sont choisis, indépendamment l'un de l'autre, entre

H ; alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par N, S ou O ; alkylaryle, saturé ou non saturé, substitué une ou plusieurs fois ou non substitué ; aryle ou hétéroaryle, chacun substitué une ou plusieurs fois ou non substitué ;

ou bien

$R^4$ et $R^5$ forment ensemble un groupe cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^8$, $R^8$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

et

$R^2$, $R^3$ sont choisis indépendamment l'un de l'autre entre

$R^9$ ou $YR^9$, avec Y = alkyle en $C_1$ à $C_{10}$, alcényle en $C_2$ à $C_{10}$ ou alcynyle en $C_2$ à $C_{10}$, ramifié ou non ramifié, substitué une ou plusieurs fois ou non substitué, $R^9$ étant choisi entre

H, F, Cl, Br, I, CN, $NO_2$, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^{10}$, $R^{10}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

$OR^{11}$, $OC(O)R^{11}$, $OC(O)OR^{11}$, $OC(S)R^{11}$, $C(O)R^{11}$, $C(O)OR^{11}$, $C(S)R^{11}$, $C(S)OR^{11}$, $SR^{11}$, $S(O)R^{11}$ ou $S(O_2)R^{11}$, $R^{11}$ étant choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^{12}$, $R^{12}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué;

$NR^{13}R^{14}$, $NR^{13}C(O)R^{14}$, $C(O)\ NR^{13}R^{14}$ ou $S(O_2)NR^{13}R^{14}$, $R^{13}$ et $R^{14}$ étant choisis indépendamment l'un de l'autre entre

H, O ; alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant dans lequel un atome de carbone du noyau est remplacé par S, O ou $NR^{15}$, $R^{15}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^{13}$ et $R^{14}$ forment ensemble un groupe cycloalkyle en $C_3$ à $C_7$, saturé ou non saturé, substitué une ou plusieurs fois ou non substitué, ou un hétérocycle correspondant, dans lequel un atome de carbone

du noyau est remplacé par S, O ou $NR^{16}$, $R^{16}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

ou bien

alkylaryle, aryle ou hétéroaryle, chacun substitué une ou plusieurs fois ou non substitué ; en particulier

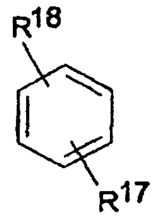

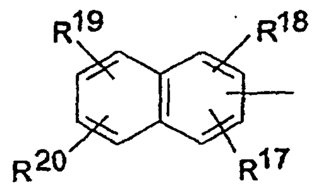

,

ou

où $R^{17}$, $R^{18}$, $R^{19}$ et $R^{20}$ sont choisis indépendamment les uns des autres entre

$R^{21}$ ou $ZR^{21}$ avec Z = alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué, $R^{21}$ étant choisi entre

H, F, Cl, Br, I, CN, $NO_2$; alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{22}$, $R^{22}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, aryle ou hétéroaryle, chacun substitué une ou plusieurs fois ou non substitué ;

$OR^{23}$ , $OC(O)R^{23}$, $OC(O)OR^{23}$, $OC(S)R^{23}$, $C(O)R^{23}$, $C(O)OR^{23}$, $C(S)R^{23}$, $C(S)OR^{23}$, $SR^{23}$, $S(O)R^{23}$ ou $S(O_2)R^{23}$, où $R^{23}$ est choisi entre

H, alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé on non saturé, substitué une ou plusieurs fois ou non substitué, ou un hétérocycle correspondant, dans lequel un atome de carbone du noyau est remplacé par S, O ou $NR^{24}$, $R^{24}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

$NR^{25}R^{26}$, $NR^{25}C(O)R^{26}$, $C(O)NR^{25}R^{26}$ ou $S(O_2)NR^{25}R^{26}$, où $R^{25}$ et $R^{26}$ sont choisis indépendamment l'un de l'autre entre

H ; alkyle en $C_1$ à $C_{18}$, alcényle en $C_2$ à $C_{18}$ ou alcynyle en $C_2$ à $C_{18}$, chacun ramifié ou non ramifié, substitué une ou plusieurs fois ou non substitué ; cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{27}$, $R^{27}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué ;

alkylaryle, saturé ou non saturé, substitué une ou plusieurs fois ou non substitué ; aryle ou hétéroaryle, chacun substitué une ou plusieurs fois ou non substitué ;

ou bien

$R^{25}$ et $R^{26}$ forment ensemble un groupe cycloalkyle en $C_3$ à $C_7$, saturé ou non saturé, substitué une ou plusieurs fois ou non substitué, ou un hétérocycle correspondant, dans lequel un atome de carbone du noyau est remplacé par S, O ou $NR^{27}$, $R^{27}$ étant choisi entre

H, alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois ou non substitué.

2. Dérivés substitués d'aminométhyl-phénylcyclohexane suivant la revendication 1, **caractérisés en ce que** $R^2$ et $R^3$ ont une définition différente et/ou $R^3$ représente H ou $CH_3$, avantageusement H, tandis que $R^1$, $R^{1'}$, $R^2$, $R^4$, $R^5$

et X ont l'une des définitions mentionnées pour eux dans les revendications précédentes.

**3.** Dérivés substitués d'aminométhyl-phénylcyclohexane suivant l'une des revendications 1 ou 2, **caractérisés en ce que** $R^2$ est choisi entre

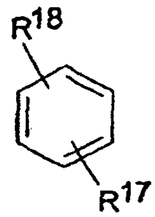

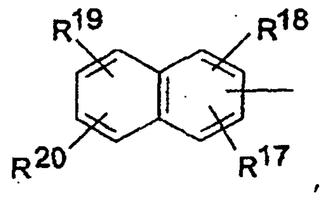

ou

où $R^{17}$, $R^{18}$, $R^{19}$ et $R^{20}$ de même que $R^1$, $R^{1'}$, $R^3$, $R^4$, $R^5$ et X ont l'une des définitions mentionnées dans les revendications précédentes

ou bien $R^2$ est choisi entre des restes alkyle en $C_1$ à $C_3$.

**4.** Dérivés substitués d'aminométhyl-phénylcyclohexane suivant l'une des revendications 1 à 3, **caractérisés en ce que** $R^2$ représente

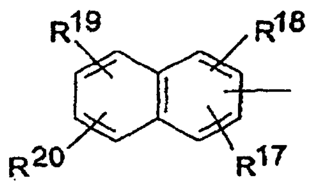

et $R^{19}$ et $R^{20}$ représentent H, tandis que $R^1$, $R^{1'}$, $R^3$, $R^4$, $R^5$, X ainsi que $R^{17}$ et $R^{18}$ ont l'une des définitions mentionnées dans les revendications précédentes.

**5.** Dérivés substitués d'aminométhyl-phénylcyclohexane suivant l'une des revendications 1 à 4, **caractérisés en ce que** $R^2$ est choisi entre

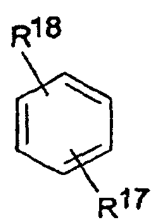

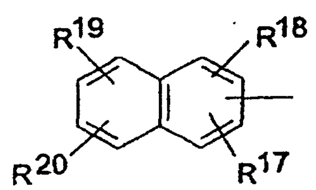

ou

$R^{17}$ et $R^{18}$ ont des définitions différentes et/ou $R^{18}$ est identique à $R^{21}$, $R^{21}$ étant choisi entre

H, F, C, Br, I, $CF_3$ ou $OR^{23}$ avec $R^{23}$ = H, méthyl-, éthyl-, propyl-, isopropyl-, butyl- ou isobutyl-,

tandis que $R^1$, $R^{1'}$, $R^3$, $R^4$, $R^5$ et X de même que $R^{17}$, $R^{19}$ et $R^{20}$ ont l'une des définitions déjà mentionnées dans les revendications précédentes.

**6.** Dérivés substitués d'aminométhyl-phénylcyclohexane suivant l'une des revendications 1 à 5, **caractérisés en ce que** $R^2$ est choisi entre

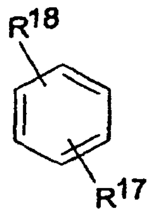

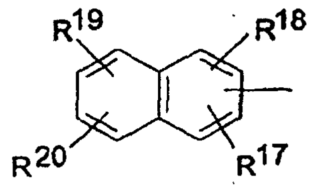

,

ou

où $R^{17}$ est choisi entre

$R^{21}$, $R^{21}$ étant choisi entre

H, F, Cl, Br, I, $CF_3$, $OR^{23}$, $OC(O)R^{23}$, $C(O)R^{23}$ ou $C(O)OR^{23}$, avantageusement H, F, Cl, $C(O)OR^{23}$, $R^{23}$ étant choisi entre

H ; alkyle en $C_1$ à $C_6$, alcényle en $C_2$ à $C_8$ ou alcynyle en $C_2$ à $C_8$, notamment alkyle en $C_1$ à $C_4$, ramifié ou non ramifié, substitué une ou plusieurs fois ou non substitué ; avantageusement H, méthyl-, éthyl-, propyl-, isopropyl-, butyl- ou isobutyl-, en particulier H, $CH_3$, $C_2H_5$ ou isobutyl- ;

$C(O)NR^{25}R^{26}$, où $R^{25}$ et $R^{26}$ sont choisis indépendamment l'un de l'autre entre

H, O ; alkyle en $C_1$ à $C_{18}$, notamment alkyle en $C_1$ à $C_4$, ramifié ou non ramifié, saturé ou non saturé, substitué une ou plusieurs fois ou non substitué, avantageusement H, méthyl-, éthyl-, propyl-, isopropyl-, butyl- ou isobutyl-, en particulier $C_2H_5$ ;

ou bien $ZR^{21}$, avec Z = $CH_2$ ou $C_2H_4$, $R^{21}$ étant choisi entre

$OR^{23}$, $OC(O)R^{23}$, $C(O)R^{23}$ ou $C(O)OR^{23}$, avantageusement $OR^{23}$, $R^{23}$ étant choisi entre

H ; alkyle en $C_1$ à $C_6$, alcényle en $C_2$ à $C_8$ ou alcynyle en $C_2$ à $C_8$, en particulier alkyle en $C_1$ à $C_4$, ramifié ou non ramifié, substitué une ou plusieurs fois ou non substitué, avantageusement H, méthyl-, éthyl-, propyl-, isopropyl-, butyl- ou isobutyl-, en particulier H ;

$C(O)NR^{25}R^{26}$, où $R^{25}$ et $R^{26}$ sont choisis indépendamment l'un de l'autre entre

H, O, alkyle en $C_1$ à $C_{18}$, en particulier alkyle en $C_1$ à $C_4$, ramifié ou non ramifié, saturé ou non saturé, substitué une ou plusieurs fois ou non substitué, avantageusement H, méthyl-, éthyl-, propyl-, isopropyl-, butyl- ou isobutyl- ;

tandis que $R^1$, $R^{1'}$, $R^3$, $R^4$ , $R^5$ et X de même que $R^{18}$ , $R^{19}$ et $R^{20}$ ont l'une des définitions mentionnées dans les revendications précédentes.

7. Dérivés substitués d'aminométhyl-phénylcyclohexane suivant l'une des revendications 1 à 6, **caractérisés en ce que**

$R^1$ est choisi entre

H, F, Cl, Br, I, $CF_3$, $SCH_3$ ou $OR^6$, avantageusement $OR^6$, où $R^6$ est choisi entre

H ; alkyle en $C_1$ à $C_4$, ramifié ou non ramifié, substitué une ou plusieurs fois ou non substitué ; avantageusement H ou $CH_3$ ;

et/ou

$R^{1'}$ est choisi entre

H, F, Cl, $SCH_3$ ou $OCH_3$, avantageusement H

et/ou

X est choisi entre

H, F, Br, I, Cl, $OR^7$, avantageusement H, F, Cl, $OR^7$, avec $R^7$ = H ou $CH_3$, avantageusement H ;

ou bien,

lorsque le composé ne porte pas de substituant X, une double liaison est formée entre l'atome de carbone A et l'atome de carbone B ou entre l'atome de carbone B et l'atome de carbone C selon la formule Ia ;

et/ou

$R^4$ et $R^5$ sont choisis indépendamment l'un de l'autre entre

des restes alkyle en $C_1$ à $C_4$, ramifiés ou non ramifiés, substitués une ou plusieurs fois ou non substitués, avantageusement $CH_3$,

tandis que $R^2$ et $R^3$ ont l'une des définitions mentionnées dans les revendications précédentes.

8. Dérivés substitués d'aminométhyl-phénylcyclohexane suivant l'une des revendications 1 à 5, **caractérisés en ce**

**qu'**il s'agit de :

*rac-cis*-E-[4-benzylidène-2-(3-méthoxy-phényl)-cyclohexylméthyl]-diméthylamine,
*rac-trans*-E-[4-benzylidène-2-(3-méthoxy-phényl)-cyclo-hexylméthyl)-diméthylamine,
*rac-trans-Z*-[4-benzylidène-2-(3-méthoxy-phényl)-cyclohexylméthyl]-diméthylamine,
*rac-cis-Z*-[4-benzylidène-2-(3-méthoxy-phényl)-cyclohexylméthyl]-diméthylamine,
ester méthylique d'acide *rac-cis*-E-3-[4-diméthylaminométhyl-3-(3-méthoxy-phényl)-cyclohexylidèneméthyl]-benzoïque,
ester méthylique d'acide *rac-cis*-Z-3-[4-diméthylaminométhyl-3-(3-méthoxy-phényl)-cyclohexylidèneméthyl]-benzoïque,
ester méthylique d'acide *rac-trans-Z*-3-[4-diméthylaminométhyl-3-(3-méthoxy-phényl)-cyclohexylidèneméthyl]-benzoïque,
ester méthylique d'acide *rac-trans*-E-3-[4-diméthylaminométhyl-3-(3-méthoxy-phényl)-cyclohexylidèneméthyl]-benzoïque,
ester méthylique d'acide Z-3-[4-diméthylaminométhyl-3-(3-méthoxy-phényl)-2-méthyl-cyclohex-2-énylidèneméthyl]-benzoïque,
ester méthylique d'acide E-3-[4-diméthylaminométhyl-3-(3-méthoxy-phényl)-2-méthyl-cyclohex-2-énylidèneméthyl]-benzoïque,
ester éthylique d'acide Z-3-[4-diméthylaminométhyl-3-(3-méthoxy-phényl)-2-méthyl-cyclohex-2-énylidèneméthyl]-naphtalène-1-carboxylique,
ester éthylique d'acide E-3-[4-diméthylaminométhyl-3-(3-méthoxy-phényl)-2-méthyl-cyclohex-2-énylidèneméthyl]-naphtalène-1-carboxylique,
ester éthylique d'acide Z-3-[4-diméthylaminométhyl-3-(3-méthoxy-phényl)-cyclohexylidèneméthyl]-2-fluorobenzoïque,
acide Z-3-[4-diméthylaminométhyl-3-(3-méthoxy-phényl)-cyclohex-2-énylidèneméthyl]-benzoïque,
acide E-3-[4-diméthylaminométhyl-3-(3-méthoxy-phényl)-cyclohex-2-énylidèneméthyl]-benzoïque,
E-{3-[4-diméthylaminométhyl-3-(3-méthoxy-phényl)-cyclohex-2-énylidèneméthyl]-phényl}-méthanol,
acide *rac-trans*-Z-3-[4-diméthylaminométhyl-3-(3-méthoxy-phényl)-cyclohexylidèneméthyl]-benzoïque,
acide *rac-trans*-E-3-[4-diméthylaminométhyl-3-(3-méthoxy-phényl)-cyclohexylidèneméthyl]-benzoïque,
*rac-trans*-E-[3-(2-diméthylaminométhyl)-5-(3-hydroxyméthyl-benzylidène)-cyclohexyl]-phénol,
*rac-trans*-E-3-(5-benzylidène-2-diméthylaminométhylcyclohexyl)-phénol,
ester tertiobutylique d'acide E-3-[4-diméthylaminométhyl-3-(3-méthoxy-phényl)-cyclohex-2-énylidèneméthyl]-benzoïque,
E-3-[6-diméthylaminométhyl-3-(3-hydroxyméthylbenzylidène)-cyclohex-1-ényl]-phénol,
*rac-trans*-Z-3-(5-benzylidène-2-diméthylaminométhylcyclohexyl)-phénol,
Z-3-[2-diméthylaminométhyl-5-(3-hydroxyméthylbenzylidène)-cyclohex-1-ényl]-phénol,
Z-{3-[4-diméthylaminométhyl-3-(3-méthoxy-phényl)-cyclohex-2-énylidèneméthyl]-phényl}-méthanol,
acide *rac-cis-E*-[4-diméthylaminométhyl-3-(3-méthoxyphényl)-cyclohexylidèneméthyl]-benzoïque,
acide *rac-cis*-Z-3-[4-diméthylaminométhyl-3-(3-méthoxy-phényl)-cyclohexylidèneméthyl]-benzoïque,
ester tertiobutylique d'acide *rac-trans-E-3-[4*-diméthylaminométhyl-3-(3-méthoxy-phényl)-cyclohexylidèneméthyl]-benzoïque,
*rac-trans*-E-[3-[4-diméthylaminométhyl-3-(3-méthoxyphényl)-cyclo-hexylidène-méthyl]-phényl]-méthanol,
ester éthylique d'acide *rac-cis*-Z-3-[4-diméthylaminométhyl-3-(3-méthoxy-phényl)-cyclohexylidèneméthyl)-benzoïque,
E-3-[4-diméthylaminométhyl-3-(3-méthoxy-phényl)-cyclohex-2-énylidèneméthyl]-N,N-diéthyl-benzamide,
*rac-trans*-E-3-[4-diméthylaminométhyl-3-(3-méthoxyphényl)-cyclohex-2-énylidèneméthyl]-N,N-diéthylbenzamide,
ester éthylique d'acide E-3-[4-diméthylaminométhyl-3-(3-méthoxy-phényl)-cyclohex-2-énylidèneméthyl]-benzoïque,
Z-3-[4-diméthylaminométhyl-3-(3-méthoxy-phényl)-cyclohex-2-énylidèneméthyl]-N,N-diéthyl-benzamide,
*rac-cis*-E-3-[4-diméthylaminométhyl-3-(3-méthoxyphényl)-cyclohexylidèneméthyl]-N,N-diéthyl-benzamide,
*rac-trans*-Z-3-[4-diméthylaminométhyl-3-(3-méthoxyphényl)-cyclohexylidèneméthyl]-N,N-diéthyl-benzamide,
ester méthylique d'acide E-3-[4-diméthylaminométhyl-3-(3-hydroxy-phényl)-cyclohex-2-énylidèneméthyl]-benzoïque,
ester méthylique d'acide Z-3-[4-diméthylaminométhyl-3-(3-hydroxy-phényl)-cyclohex-2-énylidèneméthyl]-benzoïque,
ester isobutylique d'acide Z-3-[4-diméthylaminométhyl-3-(3-méthoxy-phényl)-cyclohex-2-énylidèneméthyl]-benzoïque,

Z-3-[6-diméthylaminométhyl-3-(3-hydroxyméthylbenzylidène)-cyclohex-1-ényl]-phénol,
Z-[4-(4-chloro-benzylidène)-2-(3-méthoxy-phényl)-cyclohex-2-énylméthyl]-diméthyl-amine,
E-[4-(4-fluoro-benzylidène)-2-(3-méthoxy-phényl)-cyclohex-2-énylméthyl]-diméthyl-amine,
Z-[4-(4-fluoro-benzylidène)-2-(3-méthoxy-phényl)-cyclohex-2-énylméthyl]-diméthyl-amine,
E-3-[6-diméthylaminométhyl-3-(4-fluoro-benzylidène)-cyclohex-1-ényl]-phénol,
Z-3-[6-diméthylaminométhyl-3-(4-fluoro-benzylidène)-cyclohex-1-ényl]-phénol,
E-[4-(4-chloro-benzylidène)-2-(3-méthoxy-phényl)-cyclohex-2-énylméthyl]-diméthyl-amine,
E-3-[3-(4-chloro-benzylidène)-6-diméthylaminométhylcyclohex-1-ényl]-phénol,
ester méthylique d'acide Z-3-[4-diméthylaminométhyl-3-(3-hydroxy-phényl)-cyclohex-2-énylidèneméthyl]-benzoïque,
acide E-3-[4-diméthylaminométhyl-3-(3-hydroxy-phényl)-cyclohex-2-énylidèneméthyl]-benzoïque,
ester méthylique d'acide *rac-cis*-E-3-[4-diméthylaminométhyl-3-(3-hydroxy-phényl)-cyclohexylidèneméthyl]-benzoïque,
ester méthylique d'acide rac-cis-Z-3-[4-diméthylaminométhyl-3-(3-hydroxy-phényl)-cyclohexylidèneméthyl]-benzoïque,
acide *rac-trans*-Z-3-[4-diméthylaminométhyl-3-(3-hydroxy-phényl)-cyclohexylidèneméthyl]-benzoïque,
acide *rac-trans*-E-3-[4-diméthylaminométhyl-3-(3-hydroxy-phényl)-cyclohexylidèneméthyl]-benzoïque,
acide rac-cis-Z-3-[4-diméthylaminométhyl-3-(3-hydroxy-phényl)-cyclohexylidèneméthyl]-benzoïque,
ester méthylique d'acide rac-cis-Z-3-[4-diméthylaminométhyl-3-hydroxy-3-(3-méthoxy-phényl)-cyclohexylidèneméthyl]-benzoïque,
ester méthylique d'acide rac-cis-E-3-[3-chloro-4-diméthylaminométhyl-3-(3-méthoxy-phényl)-cyclohexylidèneméthyl]-benzoïque,
ester méthylique d'acide rac-cis-E-3-[4-diméthylaminométhyl-3-hydroxy-3-(3-méthoxy-phényl)-cyclohexylidèneméthyl]-benzoïque,
ester méthylique d'acide rac-cis-Z-3-[4-diméthylaminométhyl-3-hydroxy-3-(3-méthoxy-phényl)-cyclohexylidèneméthyl]-benzoïque,
acide (+)-trans-E-3-[4-diméthylaminométhyl-3-(3-méthoxy-phényl)-cyclohexylidèneméthyl]-benzoïque,
acide (-)-trans-E-3-[4-diméthylaminométhyl-3-(3-méthoxy-phényl)-cyclohexylidèneméthyl]-benzoïque,
ester méthylique d'acide rac-trans-E-3-(4-diméthylaminométhyl-3-phényl-cyclohexylidèneméthyl)-benzoïque,
ester méthylique d'acide rac-trans-Z-3-(4-diméthylaminométhyl-3-phényl-cyclohexylidèneméthyl)-benzoïque,
acide rac-cis-E-3-(4-diméthylaminométhyl-3-phénylcyclohexylidèneméthyl)-benzoïque,
acide rac-cis-E-3-(4-diméthylaminométhyl-3-phénylcyclohexylidèneméthyl)-benzoïque,
acide rac-trans-Z-3-(4-diméthylaminométhyl-3-phénylcyclohexylidèneméthyl)-benzoïque,
acide rac-trans-E-3-(4-diméthylaminométhyl-3-phénylcyclohexylidèneméthyl)-benzoïque,
acide rac-trans-E-3-[4-diméthylaminométhyl-3-(3-trifluorométhyl-phényl)-cyclohexylidèneméthyl]-benzoïque,
acide rac-trans-Z-3-[4-diméthylaminométhyl-3-(3-trifluorométhyl-phényl)-cyclohexylidèneméthyl]-benzoïque,
ester méthylique d'acide rac-trans-E-3-[4-diméthylaminométhyl-3-(3-fluoro-phényl)-cyclohexylidèneméthyl]-benzoïque,
ester méthylique d'acide rac-trans-Z-3-[4-diméthylaminométhyl-3-(3-fluoro-phényl)-cyclohexylidèneméthyl]-benzoïque,
ester méthylique d'acide rac-cis-E-3-[4-diméthylaminométhyl-3-(3-fluoro-phényl)-cyclohexylidèneméthyl]-benzoïque,
acide rac-trans-E-3-[4-diméthylaminométhyl-3-(3-fluoro-phényl)-cyclohexylidèneméthyl]-benzoïque,
acide rac-trans-Z-3-[4-diméthylaminométhyl-3-(3-fluoro-phényl)-cyclohexylidèneméthyl]-benzoïque,
acide rac-cis-E-3-[4-diméthylaminométhyl-3-(3-fluorophényl)-cyclohexylidèneméthyl]-benzoïque,
E-[4-éthylidène-2-(3-méthoxy-phényl)-cyclohex-2-énylméthyl]-diméthyl-amine,
[4-isopropylidène-2-(3-méthoxy-phényl)-cyclohex-2-énylméthyl]-diméthyl-amine,
E-[2-(3-méthoxy-phényl)-4-propylidène-cyclohex-2-énylméthyl]-diméthyl-amine,
E-[4-butylidène-2-(3-méthoxy-phényl)-cyclohex-2-énylméthyl]-diméthyl-amine

et de leurs sels, notamment de leurs chlorhydrates.

**9.** Procédé de production de dérivés substitués d'aminométhyl-phényl-cyclohexane de formule générale I ou Ia

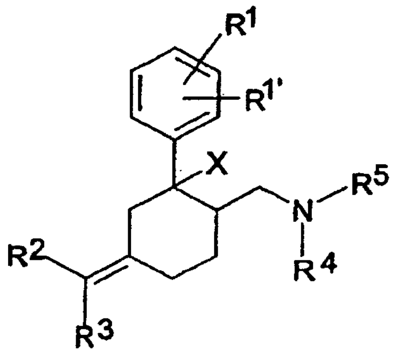

I

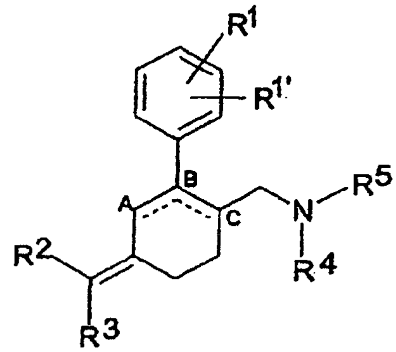

Ia ,

dans lesquelles $R^1$, $R^{1'}$, $R^2$, $R^3$, $R^4$, $R^5$ et X ont l'une des définitions mentionnées dans la revendication 1, **caractérisé en ce qu'**on fait réagir des cyclohexanones de formule II ou IIa

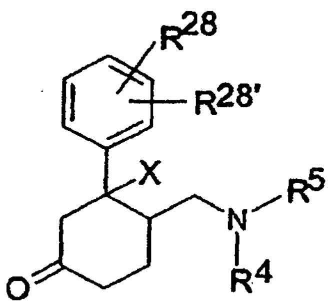

II

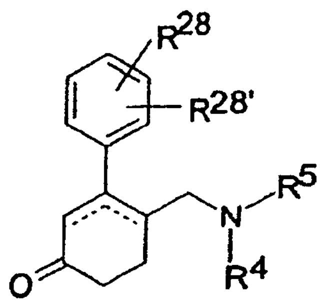

IIa ,

dans lesquelles $R^4$, $R^5$ et X ont l'une des définitions mentionnées dans la revendication 1, et $R^{28}$ a l'une des définitions mentionnées dans la revendication 1 pour $R^1$, et $R^{28'}$ a l'une des définitions mentionnées dans la revendication 1 pour $R^{1'}$ ,
dans une réaction de Wittig dans un solvant organique en présence d'une base, avec des sels d'alkyltriphénylphosphonium de formule III

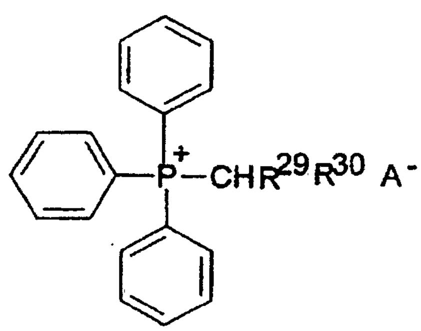

III

dans laquelle A désigne l'ion chlorure ou bromure et, indépendamment l'un de l'autre, $R^{29}$ a l'une des définitions mentionnées pour $R^2$ et $R^{30}$ a l'une des définitions mentionnées pour $R^3$ dans la revendication 1.

**10.** Procédé suivant la revendication 9, **caractérisé en ce que** le solvant organique est le benzène, le toluène ou un hydrocarbure chloré et/ou on utilise comme base le tertiobutylate de potassium ou l'hydrure de sodium et/ou la température de réaction, notamment pendant la réaction de Wittig, est maintenue entre 50 °C et 90 °C.

**11.** Procédé suivant l'une des revendications 9 ou 10, **caractérisé en ce que** dans $R^{28}$ et/ou dans $R^{28'}$ selon la formule II ou la formule IIa et/ou dans $R^{29}$ et/ou $R^{30}$ selon la formule III, au moins un groupe OH a été remplacé par un groupe $OSi(Ph)_2$tertiobutyle, au moins un groupe SH a été remplacé par un groupe S-p-méthoxybenzyle et/ou au moins un groupe $NH_2$ a été remplacé par un groupe $NO_2$ et, après la fin de la réaction de Wittig, au moins un groupe $OSi(Ph)_2$tertiobutyle est éliminé avec le fluorure de tétrabutylammonium dans le tétrahydrofuranne et/ou au moins un groupe p-méthoxybenzyle est éliminé avec une amine de métal, avantageusement l'amine de sodium et/ou au moins un groupe $NO_2$ est réduit en $NH_2$.

**12.** Procédé suivant l'une des revendications 9 à 11, **caractérisé en ce qu'**après la réaction de Wittig, un produit du procédé portant au moins un groupe $C(O)OCH_3$- et/ou un groupe $C(S)OCH_3$- est saponifié à 40°C-60°C avec une solution de KOH ou de NaOH dans le méthanol.

**13.** Médicament contenant comme substance active au moins un dérivé substitué d'aminométhyl-phénylcyclohexane de formule générale I ou Ia

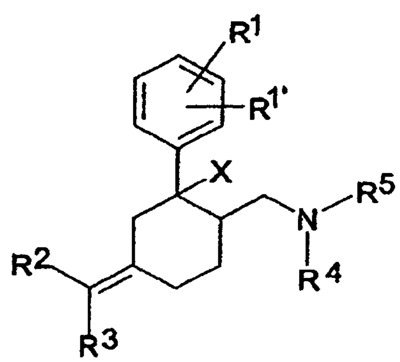

I

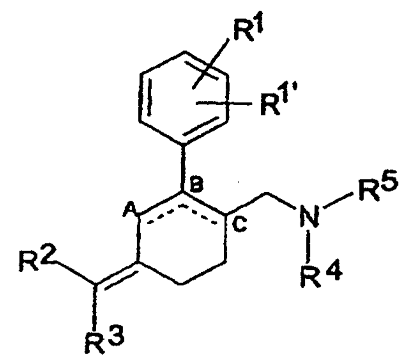

Ia ,

dans laquelle $R^1$, $R^{1'}$, $R^2$, $R^3$, $R^4$, $R^5$ et X ont l'une des définitions mentionnées dans la revendication 1, sous forme de ses diastéréoisomères ou énantiomères ainsi que de sa base libre ou d'un sel formé avec un acide acceptable du point de vue physiologique, en particulier sous forme du chlorhydrate.

**14.** Médicament suivant la revendication 13, contenant comme substance active au moins un dérivé substitué d'aminométhyl-phényl-cyclohexane choisi dans le groupe

*rac-cis*-E-[4-benzylidène-2-(3-méthoxy-phényl)-cyclohexylméthyl]-diméthylamine,
*rac-trans*-E-[4-benzylidène-2-(3-méthoxy-phényl)-cyclohexylméthyl]-diméthylamine,
*rac-trans*-Z-[4-benzylidène-2-(3-méthoxy-phényl)-cyclohexylméthyl]-diméthylamine,
*rac-cis*-Z-[4-benzylidène-2-(3-méthoxy-phényl)-cyclohexylméthyl]-diméthylamine,
ester méthylique d'acide *rac-cis*-E-3-[4-diméthylaminométhyl-3-(3-méthoxy-phényl)-cyclohexylidèneméthyl]-benzoïque,
ester méthylique d'acide *rac-cis*-Z-3-[4-diméthylaminométhyl-3-(3-méthoxy-phényl)-cyclohexylidèneméthyl]-benzoïque,
ester méthylique d'acide *rac-trans*-Z-3-[4-diméthylaminométhyl-3-(3-méthoxy-phényl)-cyclohexylidèneméthyl]-benzoïque,
ester méthylique d'acide *rac-trans*-E-3-[4-diméthylaminométhyl-3-(3-méthoxy-phényl)-cyclohexylidèneméthyl]-benzoïque,
ester méthylique d'acide Z-3-[4-diméthylaminométhyl-3-(3-méthoxy-phényl)-2-méthyl-cyclohex-2-énylidèneméthyl]-benzoïque,

ester méthylique d'acide E-3-[4-diméthylaminométhyl-3-(3-méthoxy-phényl)-2-méthyl-cyclohex-2-énylidèneméthyl]-benzoïque,
ester éthylique d'acide Z-3-[4-diméthylaminométhyl-3-(3-méthoxy-phényl)-2-méthyl-cyclohex-2-énylidèneméthyl]-naphtalène-1-carboxylique,
ester éthylique d'acide E-3-[4-diméthylaminométhyl-3-(3-méthoxy-phényl)-2-méthyl-cyclohex-2-énylidèneméthyl]-naphtalène-1-carboxylique,
ester éthylique d'acide Z-3-[4-diméthylaminométhyl-3-(3-méthoxy-phényl)-cyclohexylidèneméthyl]-2-fluorobenzoïque,
acide Z-3-[4-diméthylaminométhyl-3-(3-méthoxy-phényl)-cyclohex-2-énylidèneméthyl]-benzoïque,
acide E-3-[4-diméthylaminométhyl-3-(3-méthoxy-phényl)-cyclohex-2-énylidèneméthyl]-benzoïque,
E-{3-[4-diméthylaminométhyl-3-(3-méthoxy-phényl)-cyclohex-2-énylidèneméthyl]-phényl)-méthanol,
Acide *rac-trans*-Z-3-[4-diméthylaminométhyl-3-(3-méthoxyphényl)-cyclohexylidèneméthyl]-benzoïque,
acide *rac-trans*-E-3-[4-diméthylaminométhyl-3-(3-méthoxyphényl)-cyclohexylidèneméthyl]-benzoïque,
*rac-trans*-E-[3-(2-diméthylaminométhyl)-5-(3-hydroxyméthyl-benzylidène)-cyclohexyl]-phénol,
*rac-trans*-E-3-(5-benzylidène-2-diméthylaminométhylcyclohexyl)-phénol,
ester tertiobutylique d'acide E-3-[4-diméthylaminométhyl-3-(3-méthoxy-phényl)-cyclohex-2-énylidèneméthyl]-benzoïque,
E-3-[6-diméthylaminométhyl-3-(3-hydroxyméthylbenzylidène)-cyclohex-1-ényl]-phénol,
*rac-trans-Z*-3-(5-benzylidène-2-diméthylaminométhylcyclohexyl)-phénol,
Z-3-[2-diméthylaminométhyl-5-(3-hydroxyméthylbenzylidène)-cyclohex-1-ényl]-phénol,
Z-{3-[4-diméthylaminométhyl-3-(3-méthoxy-phényl)-cyclohex-2-énylidèneméthyl]-phényl}-méthanol,
acide *rac-cis-E*-[4-diméthylaminométhyl-3-(3-méthoxyphényl)-cyclohexylidèneméthyl]-benzoïque,
acide *rac-cis-Z*-3-[4-diméthylaminométhyl-3-(3-méthoxyphényl)-cyclohexylidèneméthyl]-benzoïque,
ester tertiobutylique d'acide *rac-trans-E*-3-[4-diméthylaminométhyl-3-(3-méthoxy-phényl)-cyclohexylidèneméthyl]-benzoïque,
*rac-trans*-E-[3-[4-diméthylaminométhyl-3-(3-méthoxyphényl)-cyclo-hexylidèneméthyl)-phényl]-méthanol,
ester éthylique d'acide *rac-cis*-Z-3-[4-diméthylaminométhyl-3-(3-méthoxy-phényl)-cyclohexylidèneméthyl]-benzoïque,
E-3-[4-diméthylaminométhyl-3-(3-méthoxy-phényl)-cyclohex-2-énylidèneméthyl]-N,N-diéthyl-benzamide,
*rac-trans*-E-3-[4-diméthylaminométhyl-3-(3-méthoxyphényl)-cyclohex-2-énylidèneméthyl]-N,N-diéthylbenzamide,
ester éthylique d'acide E-3-[4-diméthylaminométhyl-3-(3-méthoxy-phényl)-cyclohex-2-énylidèneméthyl]-benzoïque,
Z-3-[4-diméthylaminométhyl-3-(3-méthoxy-phényl)-cyclohex-2-énylidèneméthyl]-N,N-diéthyl-benzamide,
*rac-cis*-E-3-[4-diméthylaminométhyl-3-(3-méthoxyphényl)-cyclohexylidèneméthyl]-N,N-diéthyl-benzamide,
*rac-trans*-Z-3-[4-diméthylaminométhyl-3-(3-méthoxyphényl)-cyclohexylidèneméthyl]-N,N-diéthyl-benzamide,
ester méthylique d'acide E-3-[4-diméthylaminométhyl-3-(3-hydroxy-phényl)-cyclohex-2-énylidèneméthyl]-benzoïque,
ester méthylique d'acide Z-3-[4-diméthylaminométhyl-3-(3-hydroxy-phényl)-cyclohex-2-énylidèneméthyl]-benzoïque,
ester isobutylique d'acide Z-3-[4-diméthylaminométhyl-3-(3-méthoxy-phényl)-cyclohex-2-énylidèneméthyl]-benzoïque,
Z-3-[6-diméthylaminométhyl-3-(3-hydroxyméthylbenzylidène)-cyclohex-1-ényl]-phénol,
Z-[4-(4-chloro-benzylidène)-2-(3-méthoxy-phényl)-cyclohex-2-énylméthyl]-diméthyl-amine,
E-[4-(4-fluoro-benzylidène)-2-(3-méthoxy-phényl)-cyclohex-2-énylméthyl]-diméthyl-amine,
Z-[4-(4-fluoro-benzylidène)-2-(3-méthoxy-phényl)-cyclohex-2-énylméthyl]-diméthyl-amine,
E-3-[6-diméthylaminométhyl-3-(4-fluoro-benzylidène)-cyclohex-1-ényl]-phénol,
Z-3-[6-diméthylaminométhyl-3-(4-fluoro-benzylidène)-cyclohex-1-ényl]-phénol,
E-[4-(4-chloro-benzylidène)-2-(3-méthoxy-phényl)-cyclohex-2-énylméthyl]-diméthyl-amine,
E-3-[3-(4-chloro-benzylidène)-6-diméthylaminométhylcyclohex-1-ényl]-phénol,
ester méthylique d'acide Z-3-[4-diméthylaminométhyl-3-(3-hydroxy-phényl)-cyclohex-2-énylidèneméthyl]-benzoïque,
acide E-3-[4-diméthylaminométhyl-3-(3-hydroxyphényl)-cyclohex-2-énylidèneméthyl]-benzoïque,
ester méthylique d'acide rac-cis-E-3-[4-diméthylaminométhyl-3-(3-hydroxy-phényl)-cyclohexylidèneméthyl]-benzoïque,
ester méthylique d'acide *rac-cis*-Z-3-[4-diméthylaminométhyl-3-(3-hydroxy-phényl)-cyclohexylidèneméthyl]-benzoïque,
acide *rac-trans*-Z-3-[4-diméthylaminométhyl-3-(3-hydroxy-phényl)-cyclohexylidèneméthyl]-benzoïque,

acide *rac-trans*-E-3-[4-diméthylaminométhyl-3-(3-hydroxy-phényl)-cyclohexylidèneméthyl]-benzoïque,
acide rac-cis-Z-3-[4-diméthylaminométhyl-3-(3-hydroxy-phényl)-cyclohexylidèneméthyl]-benzoïque,
ester méthylique d'acide rac-cis-Z-3-[4-diméthylaminométhyl-3-hydroxy-3-(3-méthoxy-phényl)-cyclohexylidèneméthyl]-benzoïque,
ester méthylique d'acide rac-cis-E-3-[3-chloro-4-diméthylaminométhyl-3-(3-méthoxy-phényl)-cyclohexylidèneméthyl]-benzoïque,
ester méthylique d'acide rac-cis-E-3-[4-diméthylaminométhyl-3-hydroxy-3-(3-méthoxy-phényl)-cyclohexylidèneméthyl]-benzoïque,
ester méthylique d'acide rac-cis-Z-3-[4-diméthylaminométhyl-3-hydroxy-3-(3-méthoxy-phényl)-cyclohexylidèneméthyl]-benzoïque,
acide (+)-trans-E-3-[4-diméthylaminométhyl-3-(3-méthoxy-phényl)-cyclohexylidèneméthyl]-benzoïque;
acide (-)-trans-E-3-[4-diméthylaminométhyl-3-(3-méthoxy-phényl)-cyclohexylidèneméthyl]-benzoïque,
ester méthylique d'acide rac-trans-E-3-(4-diméthylaminométhyl-3-phényl-cyclohexylidèneméthyl)-benzoïque,
ester méthylique d'acide rac-trans-Z-3-(4-diméthylaminométhyl-3-phényl-cyclohexylidèneméthyl)-benzoïque,
acide rac-cis-E-3-(4-diméthylaminométhyl-3-phénylcyclohexylidèneméthyl)-benzoïque,
acide rac-cis-E-3-(4-diméthylaminométhyl-3-phénylcyclohexylidèneméthyl)-benzoïque,
acide rac-trans-Z-3-(4-diméthylaminométhyl-3-phénylcyclohexylidèneméthyl)-benzoïque,
acide rac-trans-E-3-(4-diméthylaminométhyl-3-phénylcyclohexylidèneméthyl)-benzoïque,
acide rac-trans-E-3-[4-diméthylaminométhyl-3-(3-trifluorométhyl-phényl)-cyclohexylidèneméthyl]-benzoïque,
acide rac-trans-Z-3-[4-diméthylaminométhyl-3-(3-trifluorométhyl-phényl)-cyclohexylidèneméthyl]-benzoïque,
ester méthylique d'acide rac-trans-E-3-[4-diméthylaminométhyl-3-(3-fluoro-phényl)-cyclohexylidèneméthyl]-benzoïque,
ester méthylique d'acide rac-trans-Z-3-[4-diméthylaminométhyl-3-(3-fluoro-phényl)-cyclohexylidèneméthyl]-benzoïque,
ester méthylique d'acide rac-cis-E-3-[4-diméthylaminométhyl-3-(3-fluoro-phényl)-cyclohexylidèneméthyl]-benzoïque,
acide rac-trans-E-3-[4-diméthylaminométhyl-3-(3-fluoro-phényl)-cyclohexylidèneméthyl]-benzoïque,
acide rac-trans-Z-3-[4-diméthylaminométhyl-3-(3-fluoro-phényl)-cyclohexylidèneméthyl]-benzoïque,
acide rac-cis-E-3-[4-diméthylaminométhyl-3-(3-fluorophényl)-cyclohexylidèneméthyl]-benzoïque,
E-[4-éthylidène-2-(3-méthoxy-phényl)-cyclohex-2-énylméthyl]-diméthyl-amine,
[4-isopropylidène-2-(3-méthoxy-phényl)-cyclohex-2-énylméthyl]-diméthyl-amine,
E-[2-(3-méthoxy-phényl)-4-propylidène-cyclohex-2-énylméthyl]-diméthyl-amine,
E-[4-butylidène-2-(3-méthoxy-phényl)-cyclohex-2-énylméthyl]-diméthyl-amine

sous la forme de leur base libre ou sous forme d'un sel formé avec un acide acceptable du point de vue physiologique, en particulier sous forme du chlorhydrate.

**15.** Utilisation d'au moins un dérivé substitué d'aminométhyl-phényl-cyclohexane de formule générale I ou Ia

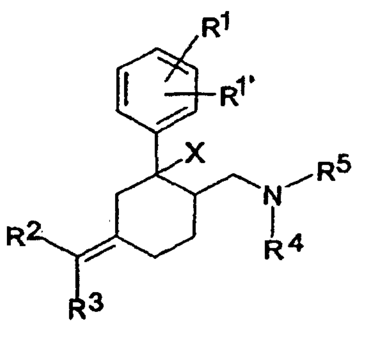

I

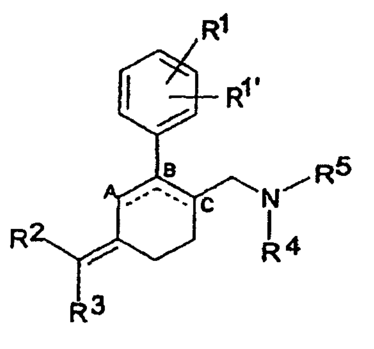

Ia ,

dans laquelle $R^1$, $R^{1'}$, $R^2$, $R^3$, $R^4$, $R^5$ et X ont l'une des définitions mentionnées dans la revendication 1, sous forme de ses diastéréoisomères ou énantiomères ainsi que de sa base libre ou d'un sel formé avec un acide acceptable du point de vue physiologique, en particulier sous forme du chlorhydrate, pour la préparation d'un médicament

destiné au traitement de la douleur.

**16.** Utilisation d'au moins un dérivé substitué d'aminométhyl-phényl-cyclohexane de formule générale I ou Ia

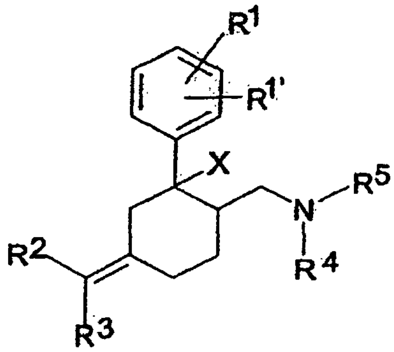

I

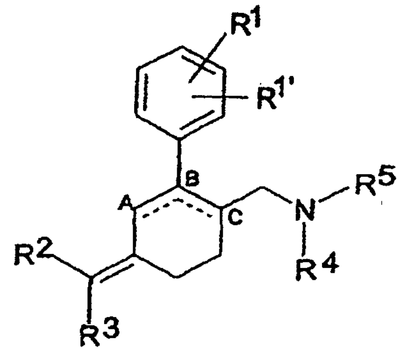

Ia

,

dans laquelle $R^1$, $R^{1'}$, $R^2$, $R^3$, $R^4$, $R^5$ et X ont l'une des définitions mentionnées dans la revendication 1, sous forme de ses diastéréoisomères ou énantiomères de même que sous forme de sa base libre ou d'un sel formé avec un acide acceptable du point de vue physiologique, en particulier sous forme du chlorhydrate, pour la préparation d'un médicament destiné au traitement de l'incontinence urinaire, du prurit et/ou de la diarrhée.

**17.** Utilisation d'au moins un dérivé substitué d'aminométhyl-phényl-cyclohexane de formule générale I ou Ia

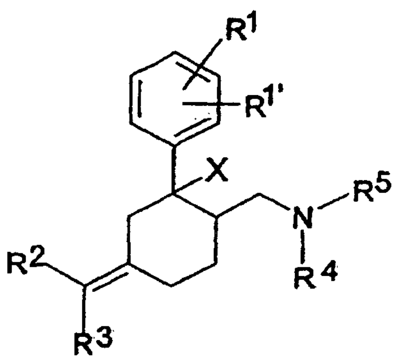

I

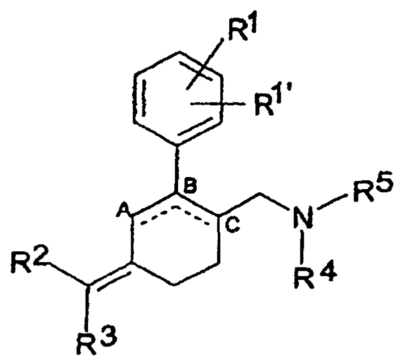

Ia

,

dans laquelle $R^1$, $R^{1'}$, $R^2$, $R^3$, $R^4$, $R^5$ et X ont l'une des définitions mentionnées dans la revendication 1, sous forme de ses diastéréoisomères ou énantiomères ainsi que de sa base libre ou d'un sel formé avec un acide acceptable du point de vue physiologique, en particulier sous forme du chlorhydrate, pour la préparation d'un médicament destiné au traitement de réactions inflammatoires et allergiques, de dépressions, d'abus de drogue et/ou d'alcool, de gastrite, d'affections cardiovasculaires, de maladies des voies respiratoires, de la toux, de maladies psychiques et/ou de l'épilepsie.